# EUROPEAN PATENT APPLICATION

(11) **EP 2 599 494 A1**
(43) Date of publication of application: **05.06.2013**
(21) Application number: 12008309.2
(22) Date of filing: 11.01.2011
(51) Int. Cl.: A61K 38/00, A61K 38/08, A61K 38/18

(54) **PKC inhibitors for the treatment of inflammatory disease or disorder**

(30) Priority: 11.01.2010 US 293794 P; 21.10.2010 US 405509 P
(62) Divisional of application: 11731747.9
(71) Applicant: Healor Ltd., 74140 Ness-Ziona (IL)
(72) Inventor: Tennenbaum, Tamar, IL96105 Jerusalem (IL); Braiman-Wiksman, Liora, IL75400 Rishon Le-Zion (IL); Sagiv, Yuval, IL70700 Gedera (IL); Gartsbein, Marina, IL49221 Petach Tikva (IL); Brenner, Ephraim, IL75241 Rishon Le-Zion (IL); Ben-Hamo, Moshe, IL51223 Bene Braq (IL); Hammer, Liat, IL71700 Modiin (IL)
(74) Representative: Helbig, Christian

(57) **Abstract**

The present disclosure provides a method, composition and kit for treatment of inflammatory disease and disorder using PKC isoform modulators.

## Description

The present application is a continuation-in-part application of 11/332,774 filed January 17, 2006, pending, which is a continuation-in-part application of PCT application No. PCT/IL2004/000640, filed Jul. 15, 2004, in which the US is designated, and also claims the benefit of U.S. Provisional Patent Application No. 60/486,906, filed Jul. 15, 2003, and furthermore is a continuation-in-part of U.S. patent application Ser. No. 10/644,775, filed Aug. 21, 2003, abaondoned, and which also claims the benefit of U.S. Provisional Application Serial No. 60/486,906 filed July 15, 2003 and which is a continuation-in-part application of U.S. patent application Ser. No. 10/169,801, filed Jul. 23, 2002, now U.S. Patent No. 7,402,571, which is a U.S. National Phase of PCT/IL01/00675 and is a continuation-in-part of U.S. patent application Ser. No. 09/629,970, filed Jul. 31, 2000, now abandoned. This application also claims the benefit of priority under 35 U.S.C. § 119(e) of U.S. Serial No. 61/405,509, filed October 21, 2010, and U.S. Serial No. 61/293,794, filed January 11, 2010. The entire contents of each application cited herein are incorporated herein by reference in their entirety for all purposes.

### BACKGROUND OF THE DISCLOSURE

### FIELD OF THE DISCLOSURE

The disclosure relates generally to methods of treating disease and more specifically to treatment of inflammatory disease and disorder.

### BACKGROUND INFORMATION

Initiation of inflammation begins with an inflammatory response and leads to the activation of neutrophils, granulocytes, monocytes, macrophages, as well as other immunomodulatory cells. This may result in a topical or systemic inflammatory cascade involving inflammatory cytokines and mediators, such as interleukins, TNFα, and prostaglandins. This complex inflammatory mediated cascade triggers a whole range of responses, such as cellular chemotaxis and endothelial injury and leads to the recruitment of additional cells from the innate and adaptive immune systems.

The skin serves as an important boundary between the internal body and the environment, preventing contact with potentially harmful pathogens. In the case of antigen/pathogen penetration, an inflammatory response is often induced to eliminate the antigen. This response leads to a dermal infiltrate that consists predominantly of T cells, polymorphonuclear cells, and macrophages.

The inflammatory response is not necessarily associated with external stimuli, or may be caused by a non-harmful environmental substances (in case of allergies). In both cases an over-expression of proinflammatory cytokines without proper controls leads to inflammation which is the hallmark of topical and systemic inflammation generally, as well as a variety of inflammatory diseases and disorders. Inflammation is associated with a variety of disorders such as eczema and dermatitis, including for example, atopic dermatitis, seborrheic dermatitis, dyshidrotic eczema, nummular dermatitis, stasis dermatitis, allergic dermatitis, psoriasis, pruritis, multiple sclerosis, cutaneous inflammation, cicatricial pemphigoid, scleroderma, hidradenitis suppurativa, toxic epidermal necrolysis, acne, osteitis, graft vs. host disease (GvHD), pyroderma gangrenosum, and Behcet's Syndrome.

Not surprisingly, over production of proinflammatory cytokines has been implicated in many inflammatory and autoimmune diseases. For example, the secretion of cytokines such as TNFα and Interleukin (IL)-23, which stimulates survival and proliferation of Th17 cells, are highly associated with psoriasis, where IL-6 is required for Th17 development in addition to its general role as proinflammatory cytokine. Other cytokines like IL-12 and IP-10 are initiators and involves in Th1 pathway which is typical to psoriasis and other autoimmune diseases. Interleukin 5 (IL-5), a cytokine that increases the production of eosinophils, is over-expressed in asthma resulting in accumulation of eosinophils in the asthmatic bronchial mucosa, a hallmark of allergic inflammation. Interleukin 4 (IL-4) and interleukin 13 (IL-13) are known mediators of the hypercontractility of smooth muscle found in inflammatory bowel disease and asthma. Additionally, as discussed further below, inflammatory cytokines have been shown to be implicated in, by way of example, psoriasis, multiple sclerosis, arthritis, ischemia, septic shock, and organ transplant rejection.

Similarly, granulocyte macrophage-colony stimulating factor (GM-CSF) is a regulator of maturation of granulocyte and macrophage lineage population and has been implicated as a key factor in many inflammatory and autoimmune diseases. For example, antibodies that inhibit GM-CSF secretion have been shown to ameliorate autoimmune disease.

Thus, development of therapeutics that reduce secretion of proinflammatory cytokines and/or regulate immunomodulators would be beneficial in alleviating topical and systemic inflammation generally, as well as a host of inflammatory and/or autoimmune diseases as discussed herein. Several lines of evidence point to modulators of PKC isoforms as useful in achieving these results.

Several *in vivo* studies have shown the involvement of T helper (Th) 17 cells as well as secretion of cytokines such as interleukins and TNFα, by skin associated cells such as keratinocytes, dendritic and T helper cells, as key players in the development of the inflammatory response involved in the pathogenesis of psoriasis and other autoimmune inflammatory diseases. As used herein, *in vivo* (Latin for "within the living") is experimentation using a whole, living organism as opposed to a partial or dead organism, or an *in vitro* ("within the glass", for instance, in a test tube or petri dish) controlled environment. The secretion of cytokines such as TNFα and Interleukin (IL)-23, which stimulates survival and proliferation of Th17 cells, also serves as a key master cytokine regulator for these diseases. (Fitch et al. (2007) Curr Rheumatol Rep. 9:461-7). Th17 cells within dermis in turn, induce secretion of IL-17A and IL-22. IL-22, in particular, derive keratinocyte hyperproliferation and augment the inflammatory response in psoriasis (Fitch et al. (2007) Curr Rheumatol Rep 9:461-7).

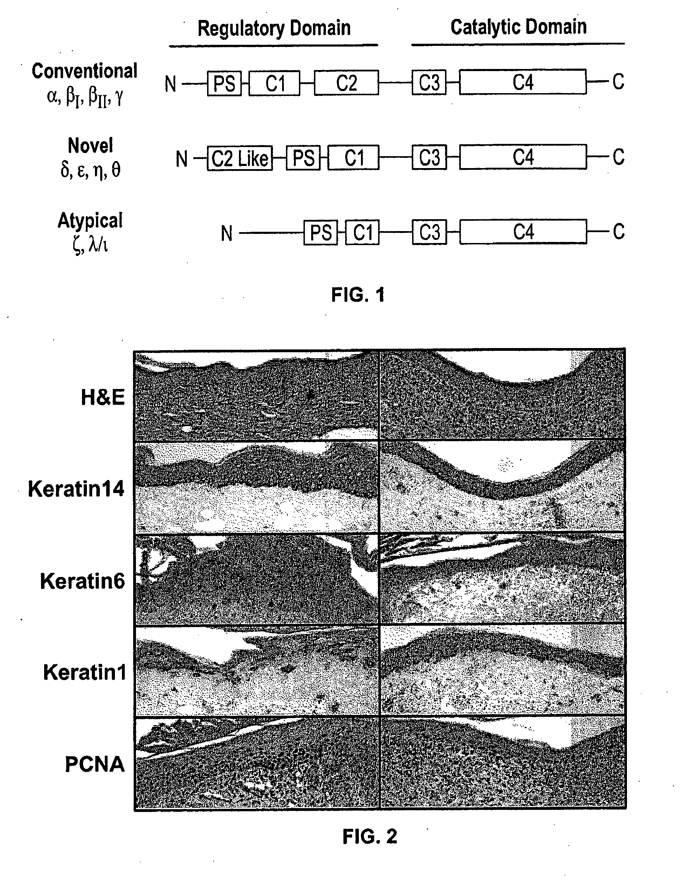

The protein kinase C (PKC) family represents a group of phospholipid dependent enzymes catalyzing the covalent transfer of phosphate from ATP to serine and threonine residues of proteins. The family is currently considered as composed of at least 12 individual isoforms which belong to 3 distinct categories based on their activation by calcium ion(s) and other factors. The PKC family consists of at least ten members, usually divided into three subgroups: classical, novel and atypical PKCs (Figure 1). The specific cofactor requirements, tissue distribution, and cellular compartmentalization suggest differential functions and the tuning of specific signaling cascades for each isoform. Thus, specific stimuli can lead to differential responses via isoform specific PKC signaling regulated by their factors, such as: expression, localization, and/or phosphorylation status in particular biological settings. PKC isoforms are activated by a variety of extracellular signals and, in turn, modify the activities of cellular proteins including receptors, enzymes, cytoskeletal proteins, and transcription factors. Accordingly, the PKC family plays a central role in cellular signal processing including regulation of cell proliferation, differentiation, survival and death.

PKCα, which is highly abundant in skin, is the major conventional, Ca²⁺ responsive, PKC isoform in epidermis and it was initially the only cPKC detected in the keratinocytes *in vitro* and *in vivo* (Dlugosz et al. (1992) Biomed Pharmacother 46:304; Wang et al. (1993) J Cancer Res Clin Oncol 119:279-287)*.* Therefore, PKCα had been proposed as a key player in Ca²⁺ induced differentiation (Denning et al. (1995) Cell Growth Differ 6:149-157; Dlugosz et al. (1992) Biomed Pharmacother 46:304). Being in epidermis and mainly restricted to suprabasal layers (Denning et al. (2004) Int J Biochem Cell Biol 36:1141-1146), PKCα is involved in cell cycle withdrawal and primarily associated with the keratin cytoskeleton and desmosomal cell-cell junctions (Jansen et al. (2001) Int J Cancer 93:635-643; Tibudan et al. (2002) J Invest Dermatol. 119:1282-1289). Since, upon exposure to the classical PKC activator TPA (12-O- tetradecanoylphorbol-13-acetate), spinous markers were suppressed, PKCα was thought to be largely responsible for the shift from spinous to granular differentiation as a result of TPA activation (Dlugosz and Yuspa (1993) J Cell Biol 120:217-225; Lee et al. (1998) J lnvest Dermatol 111:762-766; Matsui et al. (1992) J Invest Dermatol 99:565-571; Punnonen et al. (1993) J Invest Dermatol 101:719-726)*.* Indeed, blocking PKCα activity or its synthesis by antisense oligonucleotides appeared to abolished granular markers and revive spinous markers like K1 and K10. Likewise, implementation of dominant negative PKCα appeared to restore the (late) spinous marker involucrin (Deucher et al. (2002) J Biol Chem 277:17032-17040). Accordingly, defective differentiation in skin cancer (Tennenbaum et al. (1993) Cancer Res 3:4803-4810; Tomakidi et al. (2003) J Pathol 200:298-307) correlates with elevated PKCα activity, also observed in tumor cells *in vitro* (Dlugosz et al. (1992) Biomed Pharmacother 46:304; Yang et al. (2003) J Cell Physiol. 195:249-259). However, over-expression of PKCα in normal human keratinocytes did not appear to alter their differentiation pattern (Deucher et al. (2002) J Biol Chem 277:17032-17040). The influence of PKCα on the cellular traffic and membrane recruitment of β1-integrin during migration (Ng et al. (1999) EMBO J 18:3909-3923) may well promote both wound reepithelialization and tumor cell invasion.

Over-expression of PKCα in transgenic mice has appeared to induce a striking inflammatory response, increased epidermal thickening and edema correlated with neutrophil infiltration, multiple micro-abscesses, and a marked increase of inflammatory cytokines and chemokines, such as TNFα, MIP-2, COX-2 or macrophage inflammatory protein (MIP). These results implicate PKCα in the epidermal inflammatory response (Wang and Smart (1999) J Cell Sci 112:3497-3506). Treatment with TPA (a PKCα activator) apparently caused epidermal hyperplasia, intra-epidermal inflammation, and massive apoptosis (Cataisson et al. (2003) J Immunol 171:2703-2713; Jansen et al. (2001) Int J Cancer 93:635-643). In addition, recent *in vivo* studies in PKC isoenzyme-selective knockout and transgenic mice appear to have highlighted distinct functions of individual PKCs in the immune system. These genetic analyses, along with biochemical studies appear to indicate that PKC-regulated signaling pathways play a significant role in many aspects of the immune responses. For example, members of the PKC family appear crucial in T cell signaling pathways. Particularly, PKCα, isotype appears to determine the nature of lymphocyte-specific *in vivo* effector. PKCα is also discussed as being involved in macrophages activation and was apparently shown to be involved in mast cell signaling (Cataisson et al. (2005) J Immunol 174:1686-1692). Therefore, PKC isotypes are validated drug targets in adaptive immunity.

[0010] One example of an inflammatory disease is psoriasis. There are two main hypotheses about the basic pathology leading to psoriasis development. The first considers psoriasis as primarily a disorder of excessive growth and reproduction of skin cells. The second hypothesis considers psoriasis as an immune-mediated disorder in which the excessive reproduction of skin cells is secondary to factors produced by the immune system. Accordingly, most drugs for psoriasis target one component of the disease, either the hyperproliferative state of skin cells, or the skin inflammatory response as presented in psoriasis plaques.

[0011] Recent data support the notion that both pathways underlie the pathology of the diseases through a cross talk between skin cells and immunological milieu (encompassing environment, surroundings, location and/or setting). Classic genome wide linkage analysis has identified nine locations (loci) on different chromosomes associated with tendency to develop psoriasis named psoriasis susceptibility 1 through 9 (PSORS1 through PSORS9) loci. In these locations several genes were characterized and found to encode for proteins expressed in epidermal cells such as corneodesmosin, expressed in the granular and cornified layers of the epidermis and upregulated in psoriasis. On the other hand, other psoriasis linked genes encode for proteins involved in modulation of the immune system where characterized such as IL12B on chromosome 5q which expresses interleukin-12B (Frank et al. (2009) N Engl J Med 361:496-509).

[0012] Another example of inflammatory disease is Multiple Sclerosis (MS). MS is a chronic and unpredictable inflammatory disease of the CNS, which may affect the brain and spinal cord that commonly affects young adults (Hafler et al. (2005) Immunol Rev 204:208-31). It is currently alleged to be the most common neurological disease of young adults, and usually initiates between the ages of 20 and 40, with the tendency to occur in women at almost double the probability in comparison to men.

In MS, the myelin sheath, the material that surrounds and protects the nerve cells, and/or its ability for production is damaged, this is referred to as "demyelization". This damage has the effect of slowing down or blocking messages between the brain and the body, leading to the symptoms observed with MS. Demyelization and scarring or other lesions in areas disseminated in the brain and/or spinal cord are considered characteristic of the disease (Beeton et al. (2007) Journal of Visualized Experiments 594-604). These lesions appear to alter nerve conduction and induce the disabling neurological deficits that vary with the location of the demyelinated plaques in the CNS (Beeton et al. (2007) Journal of Visualized Experiments 594-604). Its clinical signs and symptoms are variable and depend on the parts of the CNS it affects, and may include motor, sensory, autonomic and cognitive disabilities (Noseworthy et al. (2000) N Engl J Med 343:938-52).

Some common symptoms of MS include: 1) overwhelming sense of tiredness; 2) balance - walking and co-ordination difficulties; 3) visual problems - double vision and loss of sight; 4) numbness and tingling in hands and feet; 5) pain - both mild and severe; loss of muscle strength; 6) stiffness and spasms in muscles; 7) mood swings - depression and anxiety; 8) memory and concentration problems; speech problems (The National MS Society Web Site).

Progressive disability is the fate of most patients with MS, especially when a 25-year perspective is included. Half of MS patients require a cane to walk within 15 years of disease onset. MS is a major cause of neurologic disability in young and middle-aged adults and, until the past decade, has had no known beneficial treatments. MS is difficult to diagnose because of the non-specific clinical findings, which led to the development of highly structured diagnostic criteria that include several technological advances, consisting of MRI scans, evoked potentials, and cerebrospinal fluid (CSF) studies. Diagnostic criteria generally rely upon the general principles of scattered lesions in the central white matter occurring at different times and not explained by other etiologies such as infection, vascular disorder, or autoimmune disorder.

MS is widely considered an autoimmune disease whereby an unknown agent or agents triggers a T - cell- mediated inflammatory attack, causing demyelization of CNS (central nervous system) tissue (Weiner et al. (2004) Arch Neurol 61:1613-1615). The evidence for an autoimmune reaction targeting myelin is strong but not definitive. There are, for example, descriptions of primary oligodendrocyte apoptosis with microglial activation in early multiple sclerosis lesions in the absence of lymphocytes or myelin phagocytosis (Manuel et al. (2006) Brain*).*

MS characteristically is reported as having four patterns of disease: relapsing-remitting MS (RRMS), primary progressive MS (PPMS), progressive relapsing MS (PRMS); and secondary progressive MS (SPMS). An estimated 50% of patients with RRMS will develop SPMS in 10 years, and up to 90% of RRMS patients will eventually develop SPMS. Each pattern of disease may present as mild, moderate or severe. Persons with RRMS present defined attacks of worsening neurologic function. These attacks are followed by partial or complete recovery periods (remissions), during which no disease progression occurs, (about 85% of people are initially diagnosed with RRMS). PPMS is characterized by slowly worsening neurologic function from the beginning, with no distinct relapses or remissions (about 10% of people are diagnosed with PPMS). In SPMS, following an initial period of RRMS, many people develop a Secondary-Progressive disease course in which the disease worsens more steadily, (about 50 % of people with RRMS develop this form of the disease within 10 years). In PRMS people experience steadily worsening disease symptoms from the beginning, but with clear attacks of worsening neurologic function along the way, while the disease appears to progress without remissions (5%) (The National MS Society web site).

There is currently no cure for MS although several treatments that attempt to reduce disease activity and disease progression are available. Six drugs in four classes are approved in the United States for the treatment of MS. FDA-Approved disease treatments include the following: interferon class, IFN-beta-1a (REBIF® and AVONEX®) and IFN-beta-1b (BETASERON®); glatiramer acetate (COPAXONE®), a polypeptide; natalizumab (TYSABRI®); and mitoxantrone (NOVANTRONE®), a cytotoxic agent. Other drugs have been used with varying degrees of success, including corticosteroids, methotrexate, cyclophosphamide, azathioprine, and intravenous (IV) immunoglobulin. The benefits of currently approved treatments are relatively modest for relapse rate and prevention of disability in MS.

REBIF® (interferon beta la) is a medication manufactured by a biotechnological process that produces the same interferon beta as found in the human body. REBIF® is reportedly given three times a week subcutaneously. (From the FDA approved prescription information for REBIF®).

AVONEX® (interferon beta la) is a medication manufactured by a biotechnological process that produces the same interferon beta as found in the human body. AVONEX® is reportedly given as a once a week intramuscular injection. (From the FDA approved prescription information for AVONEX®).

BETASERON® (interferon beta 1b) is a medication manufactured by a biotechnological process that made up the same interferon beta as found in the human body. BETASERON® is reportedly injected subcutaneously every other day. (From the FDA approved prescription information for BETASERON®).

COPAXONE® (glatiramer acetate) is a synthetic protein that simulates myelin basic protein. Through a mechanism that is not completely understood, this drug seems to block myelin damaging T cells by acting as a myelin decoy. COPAXONE® is reportedly injected subcutaneously once a day. (From the FDA approved prescription information for COPAXONE®).

TYSABRI® (natalizumab) is a laboratory produced monoclonal antibody. It is designed to hamper movement of potentially damaging immune cells from the bloodstream, across the "blood-brain barrier" into the brain and spinal cord. TYSABRI® is reportedly given once every four weeks by intravenous infusion. (From the FDA approved prescription information for TYSABRI®).

NOVANTRONE® (mitoxantrone) belongs to the general group of medicines called antineoplastics. It has been used to treat certain forms of cancer. It reportedly acts in MS treatment by suppressing the activity of T cells, B cells and macrophages that are presumed to lead the attack on the myelin sheath. (From the FDA approved prescription information for NOVANTRONE®).

Current therapies for combating inflammatory diseases generally fail to provide a multi-component approach targeting multiple components of pathogenesis. For example, many treatments for autoimmune diseases involve targeting a single component of a disease, either by blocking cellular proliferation, or by suppressing the immune response in order to block inflammation. Consequently, there is a strong need to provide effective therapeutics which target multiple components of inflammatory disease pathogenesis by targeting and modulating PCK isoform activity. Specifically targeted therapeutics that are capable of selective inhibition or activation of specific PKC isoforms are necessary and would provide for a therapeutic approach that targets multiple components of inflammatory disease pathogenesis, while retaining a low level of side effects, for example, when topically administered. Thus, development of therapeutics that reduce secretion of proinflammatory cytokines and/or regulate immunomodulators via PKC isoform modulation would be beneficial in alleviating topical and systemic inflammation generally, as well as a host of inflammatory and/or autoimmune diseases as discussed herein.

### SUMMARY OF THE DISCLOSURE

The present disclosure relates to treatment of inflammatory disease and disorder by administering to a subject a modulator of PKC, such as an inhibitor of PKCε, or PKCη, or activator of PKCδ.

Accordingly, in one aspect, the present disclosure provides a method of treating an inflammatory disease or disorder in a subject. The method includes administering to the subject an inhibitor of PKC, thereby treating the inflammatory disease or disorder in the subject. In exemplary embodiments, the inhibitor is a polypeptide which selectively inhibits PKCα, PKCε or PKCη, such as the polypeptides of SEQ ID NOs: 1-29.

In another aspect, the present disclosure provides a method of treating an inflammatory disease or disorder in a subject. The method includes administering to the subject an activator of PKCδ, thereby treating the inflammatory disease or disorder in the subject. In various embodiments, the activator is a polypeptide which selectively activates PKCδ, such as the polypeptides of SEQ ID NOs: 30-37.

In another aspect, the present disclosure provides a method of treating pruritus in a subject. The method includes administering to the subject an inhibitor of PKC, thereby treating pruritus in the subject. In various embodiments, the inhibitor is an inhibitor of PKCα, PKCε or PKC_{η}. In exemplary embodiments, the inhibitor is a polypeptide which selectively inhibits PKCα, PKCε or PKCη, such as the polypeptides of SEQ ID NOs: 1-29.

In another aspect, the present disclosure provides a method of treating pruritus in a subject. The method includes administering to the subject an activator of PKCδ, thereby treating pruritus in the subject. In various embodiments, the activator is a polypeptide which selectively activates PKCδ, such as the polypeptides of SEQ ID NOs: 30-37.

In another aspect, the present disclosure provides a method of treating multiple sclerosis in a subject. The method includes administering to the subject an inhibitor of PKCα PKCη, PKCε, or PKCε thereby treating multiple sclerosis in the subject. In exemplary embodiments, the inhibitor is a polypeptide which selectively inhibits PKCα or PKCη, such as the polypeptides of SEQ ID NOs: 1-13 and 26-29.

In various aspects, the present disclosure provides a kit for carrying out the method of the disclosure. In one embodiment, the kit includes an inhibitor of PKC, such as an inhibitor of PKCα, PKCε or PKC_{η}, or an activator of PKCδ, as well as instructions for administering the inhibitor or activator.

In another aspect, the present disclosure provides an isolated polypeptide of SEQ ID NO: 3 or a physiologically acceptable salt thereof, wherein the polypeptide is N-myristoylated. In an exemplary embodiment, the polypeptide is SEQ ID NO: 12.

The present disclosure further provides a pharmaceutical composition that includes the polypeptide of SEQ ID NO: 3 or a physiologically acceptable salt thereof, wherein the polypeptide is N-myristoylated; and a pharmaceutically acceptable vehicle.

In another aspect, the present disclosure provides an isolated polypeptide including the amino acid sequence of SEQ ID NO: 4 or a physiologically acceptable salt thereof. In exemplary embodiments, the isolated polypeptide is that of SEQ ID NO: 10 or SEQ ID NO: 13.

The present disclosure further provides a pharmaceutical composition that includes an isolated polypeptide including the amino acid sequence of SEQ ID NO: 4 or a physiologically acceptable salt thereof.

In another aspect, the present disclosure provides an isolated polypeptide selected from SEQ ID NOs: 30-33 or a physiologically acceptable salt thereof. In exemplary embodiments, the isolated polypeptide is that of SEQ ID NO: 34-37.

The present disclosure further provides a pharmaceutical composition that includes an isolated polypeptide including the amino acid sequence of SEQ ID NOs: 30-33 or a physiologically acceptable salt thereof.

### BRIEF DESCRIPTION OF THE DRAWINGS

Figure 1 is a pictorial representation depicting various members of the PKC family of isoforms.

Figure 2 is a series of pictorial representations depicting inhibition of PKCα which regulates keratinocytes structure integrity characteristic to psoriasis. Skin tissues were paraffin embedded and stained for hematoxiline and eosine (H&E) general histological staining or distinct markers for the various skin layers including Keratin 14 (K14) for basal layer, Keratin 1 (K1) for spinous layer, Keratin 6 (K6) for keratinocytes migration and PCNA for keratinocytes proliferation. The results demonstrate normalization of skin properties following PKCα inhibition (left column is WT, right column is PKCα knock out).

Figure 3 is a histogram comparing severity of scaling in different knock out mice as compared to control after treatment with IMQ.

Figure 4 is a series of pictorial representations showing scaling in knock out mice as compared with control after treatment with IMQ.

Figure 5 is a series of pictorial representations showing expression of Filaggrin (Fil), Loricrin (Lor) and Keratin 1 (K1).

Figures 6A-B are a series of pictorial and graphical representations assessing keratinocytes proliferation *in vitro* and *in vivo.* Figure 6A is a pictorial representation showing expression of PCNA. Figure 6B is a histogram comparing the percentage of PCNA positive cells treated with HO/02/10 and control.

Figure 7 is a series of pictorial representations showing expression of Filaggrin (Fil), Loricrin (Lor), Keratin 1 (K1), PCNA and Keratin 14 (K 14).

Figure 8 is graphical representation presenting a summary of protein expression data in keratinocytes for various peptide PKCα inhibitors.

Figure 9 is a histogram comparing the bursting pressure of skin samples treated with HO/02/10 and control.

Figure 10 is a histogram comparing the anti-inflammatory effect of HO/02/1 0 on skin wound in B57BL/6J mice after 4 and 9 days post wounds.

Figure 11 is a histogram comparing cytokine secretion in splenocytes treated with HO/02/10.

Figure 12 is a series of pictorial representations showing ICAM expression in basal keratinocytes and endothelial cells in blood vessels of the skin.

Figure 13 is a series of pictorial representations showing ICAM expression in basal keratinocytes and endothelial cells in blood vessels of the skin.

Figure 14 is a histogram comparing the percent of mice exhibiting positive ICAM-1 staining at wound edges.

Figure 15 is a histogram comparing the number of cells per field of Iba-1 positively stained cells.

Figures 16A-B are a series of pictorial and graphical representations showing MAC-2 expression in keratinocytes. Figure 16A is a series of stains showing MAC-2 expression. Figure 16B is a histogram comparing the number of cells per field of MAC-2 positively stained cells with control, 1, 10 and 100 micrograms per mL PKCα inhibitor (from left).

Figures 17A-D are a series of histograms comparing cytokine secretion in LPS activated keratinocytes treated with HO/02/10. Figure 17A compares secretion of IL-6, IL-1α, and GM-CSF. Figure 17B compares secretion of G-CSF. Figure 17C compares secretion of MIP-2. Figure 17D compares secretion of KC.

Figures 18A-C are a series of histograms comparing cytokine secretion in LPS activated macrophages treated with HO/02/10. Figure 18A compares secretion of G-CSF, KC and MIP-2. Figure 18B compares secretion of IL1α (left bars of histogram pairs) and TNFα (right bars of histogram pairs). Figure 18C compares secretion of IL1β (left bars of histogram pairs) and IL12 (right bars of histogram pairs).

Figure 19 is a histogram comparing cytokine secretion in LPS activated keratinocytes treated with peptide PKCα inhibitors.

Figure 20 is a histogram comparing cytokine secretion in LPS activated keratinocytes treated with peptide PKCα inhibitors.

Figures 21A-B are histograms comparing cytokine secretion in TNFα activated keratinocytes treated with peptide PKCα inhibitors. Figure 21A compares secretion of IL-1A. Figure 21B compares secretion of IL-6.

Figures 22A-B are histograms comparing cytokine secretion in TNFα activated keratinocytes treated with peptide PKCα inhibitors. Figure 22A compares secretion of G-CSF. Figure 22B compares secretion of GM-CSF.

Figures 23A-B are histograms comparing cytokine secretion in TNFα activated keratinocytes treated with peptide PKCα inhibitors. Figure 23A compares secretion of MIP-2. Figure 22B compares secretion of IP-10.

Figures 24A-B are histograms comparing cytokine secretion in IL-17A activated keratinocytes treated with peptide PKCα inhibitors. Figure 24A compares secretion of IL-1A. Figure 24B compares secretion of IL-6.

Figures 25A-B are histograms comparing cytokine secretion in IL-17A activated keratinocytes treated with peptide PKCα inhibitors.. Figure 25A compares secretion of TNFα. Figure 25B compares secretion of IP-10.

Figures 26A-B are histograms comparing cytokine secretion in IL-17A activated keratinocytes treated with peptide PKCα inhibitors. Figure 26A compares secretion of G-CSF. Figure 26B compares secretion of GM-CSF.

Figure 27A-B are histograms comparing cytokine secretion in IL-17A activated keratinocytes treated with peptide PKCα inhibitors. Figure 27A compares secretion of KC. Figure 27B compares secretion of MIP-2.

Figure 28 is a series of pictorial and graphical representations showing down regulation of T cell infiltration to the dermis and epidermis during the inflammatory stage after treatment with HO/02/10. Figure 28A is a series of stains using anti-CD3 antibodies. Figure 28B is a histogram comparing the number of cells per field of CD3 positively stained cells.

Figure 29 is a graphical representation presenting a summary of the effects of treatment using the peptide PKCα inhibitor MPDY-1 on different cell types.

Figure 30 is a graphical representation showing a schema of the overall effect of HO/02/10 on the psoriatic related pathway.

Figures 31A-B are a series of pictorial and graphical representations showing down regulation of neutrophil infiltration to the dermis and epidermis during the inflammatory stage after treatment with HO/02/10. Figure 31A is a stain using neutrophil specific antibodies. Figure 31B is a histogram comparing the number of cells per field of neutrophil specific positively stained cells.

Figure 32 is a pictorial representation of an SDS PAGE stained with Ser176/180 antibody.

Figure 33 is a graphical representation showing EAE score over time course of treatment.

Figure 34 is a graphical representation showing EAE score over time course of treatment.

Figure 35 is a graphical representation showing EAE score over time course of treatment.

Figure 36 is a graphical representation showing EAE score over time course of treatment.

Figure 37 is a graphical representation showing EAE score over time course of treatment.

Figure 38 is a pictorial representation of the mechanism of action of histamine that is used in a prick test model to assess MPDY-1 effect on pruritus.

Figure 39 is a pictorial representation showing subject's forearms injected with histamine and treated with or without MPDY-1.

Figure 40 is a pictorial representation showing subject's forearms injected with histamine and treated with or without MPDY-1.

Figure 41 is a pictorial representation showing subject's forearms injected with histamine and treated with or without MPDY-1.

Figure 42 is a pictorial representation showing subject's forearms injected with histamine and treated with or without MPDY-1.

Figure 43 is a table of data collected in *in vitro* immunological tests for PKCα inhibitor MPDY-1 and PKCδ activator DAP-1 (SEQ ID NO: 34) (all data not presented).

Figure 44 is a tabular summary of results for PKCδ activator DAP-1 (SEQ ID NO: 34) of cytokine secretion in keratinocytes treated with TNFα and inhibitor.

Figure 45 is a histogram showing comparing cytokine secretion in keratinocytes treated with LPS or TNFα and various PKCε inhibitors.

Figure 46 is a histogram showing comparing cytokine secretion in keratinocytes treated with LPS or TNFα and various PKCε inhibitors.

Figure 47 is a histogram showing comparing cytokine secretion in keratinocytes treated with LPS or TNFα and various PKCε inhibitors.

Figure 48 is a histogram showing comparing cytokine secretion in keratinocytes treated with LPS or TNFα and various PKCε inhibitors.

Figure 49 is a tabular summary of results for various PKCε inhibitors of cytokine secretion in keratinocytes treated with LPS or TNFα and inhibitor.

Figure 50 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

Figure 51 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

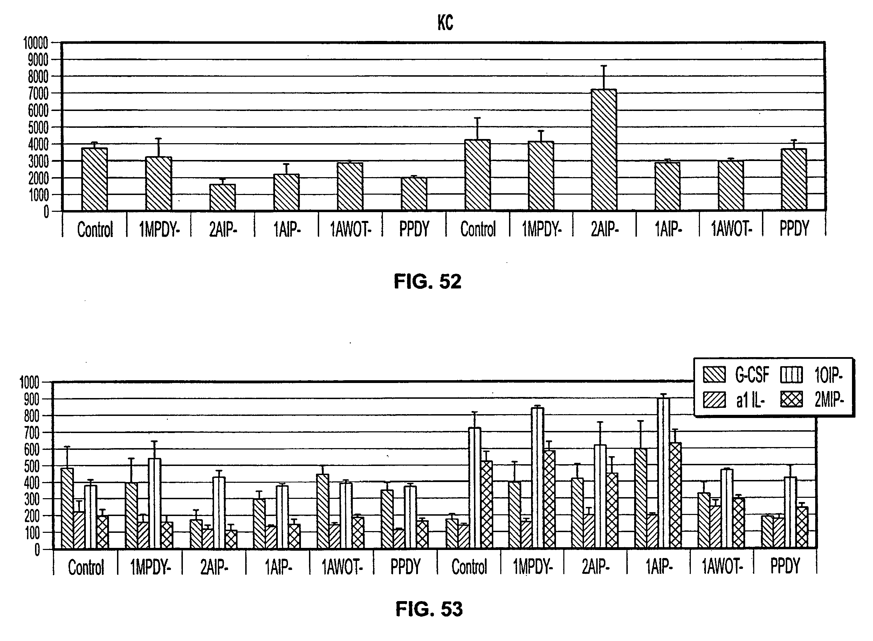

Figure 52 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

Figure 53 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

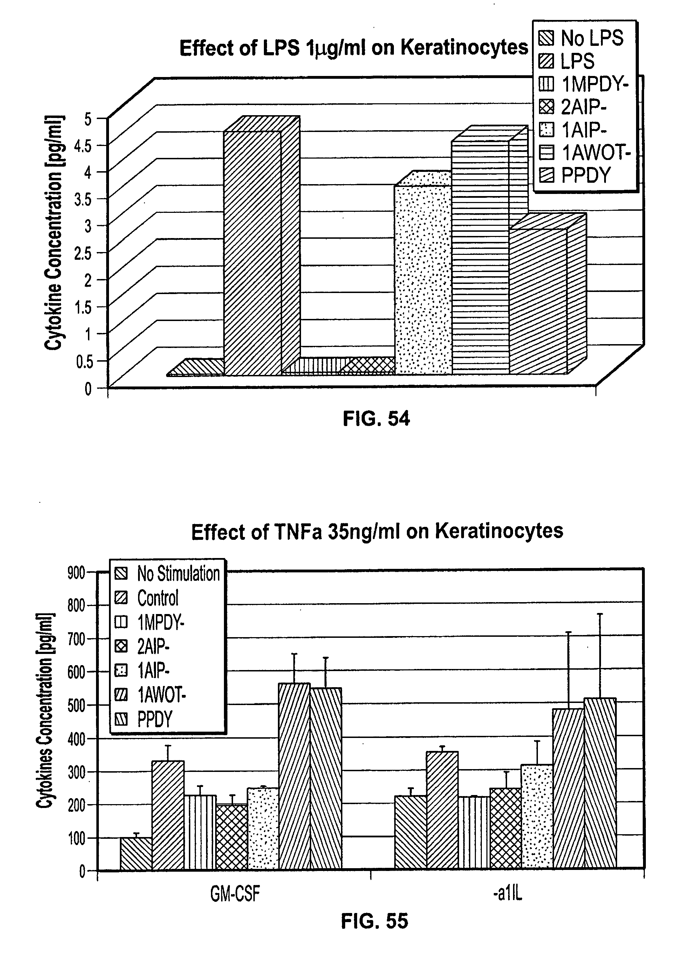

Figure 54 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

Figure 55 is a histogram comparing cytokine secretion in TNFα activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

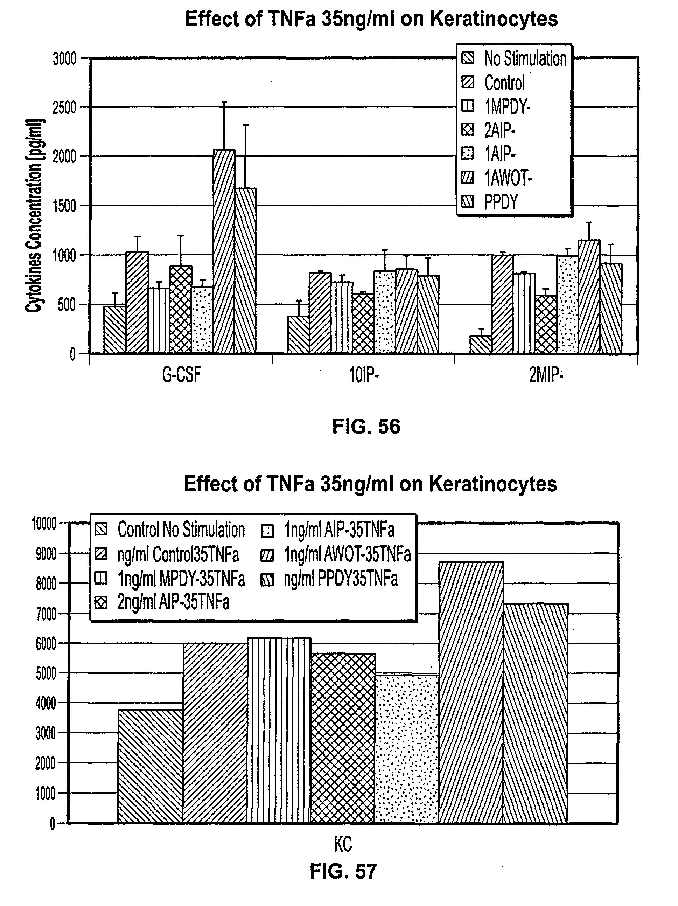

Figure 56 is a histogram comparing cytokine secretion in TNFα activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

Figure 57 is a histogram comparing cytokine secretion in TNFα activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

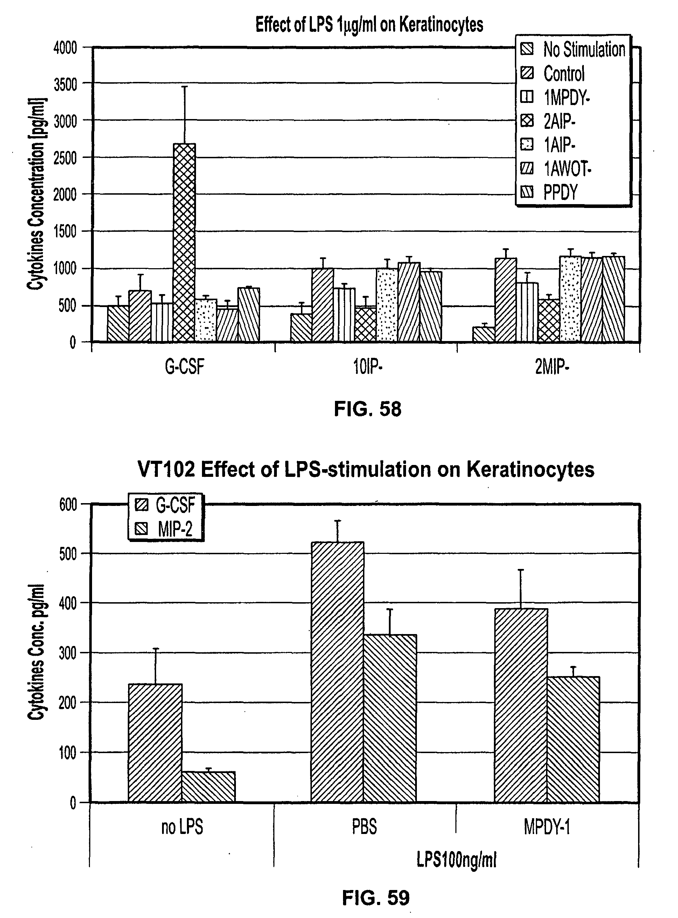

Figure 58 is a histogram comparing cytokine secretion in LPS activated keratinocytes treated with peptide PKCα inhibitors including MPDY-1 (SEQ ID NO: 6), AWOT-1 (SEQ ID NO. 7), AIP-2 (SEQ ID NO: 8), AIP-1 (SEQ ID NO: 9), and PPDY (SEQ ID NO: 10).

Figure 59 is a histogram comparing cytokine secretion in LPS activated keratinocytes treated with peptide PKCα inhibitor MPDY-1 (SEQ ID NO: 6).

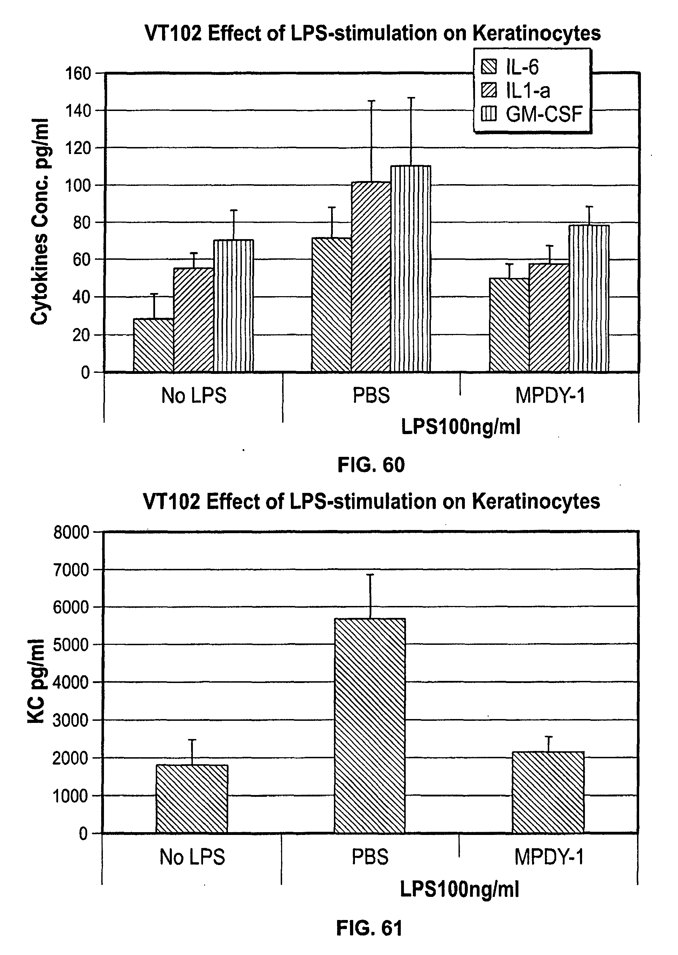

Figure 60 is a histogram comparing cytokine secretion in LPS activated keratinocytes treated with peptide PKCα inhibitor MPDY-1 (SEQ ID NO: 6).

Figure 61 is a histogram comparing cytokine secretion in LPS activated keratinocytes treated with peptide PKCα inhibitor MPDY-1 (SEQ ID NO: 6).

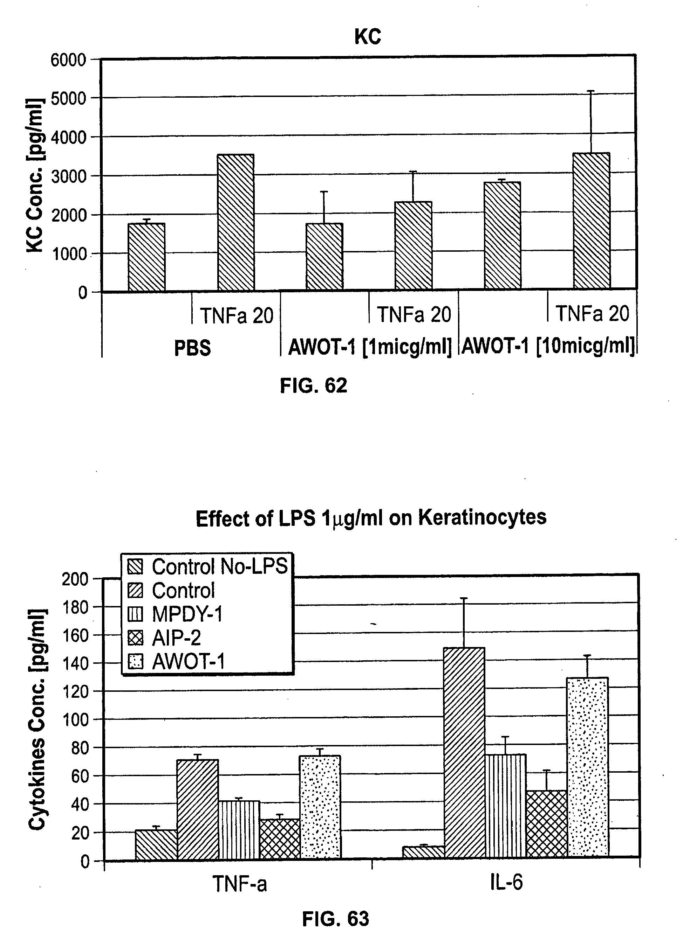

Figure 62 is a histogram comparing cytokine secretion in LPS activated keratinocytes treated with peptide PKCα inhibitor AWOT-1 (SEQ ID NO: 7).

Figure 63 is a histogram comparing cytokine secretion in LPS activated keratinocytes treated with peptide PKCα inhibitors MPDY-1 (SEQ ID NO: 6), AWOT-1 (SEQ ID NO: 7), and AIP-2 (SEQ ID NO: 8).

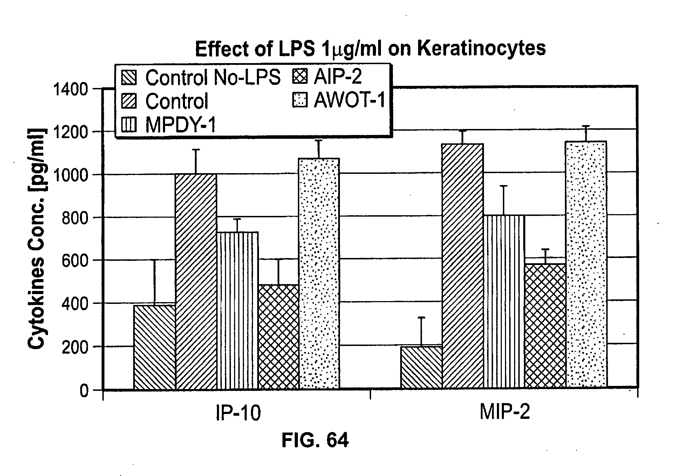

Figure 64 is a histogram comparing cytokine secretion in LPS activated keratinocytes treated with peptide PKCα inhibitors MPDY-1 (SEQ ID NO: 6), AWOT-1 (SEQ ID NO: 7), and AIP-2 (SEQ ID NO: 8).

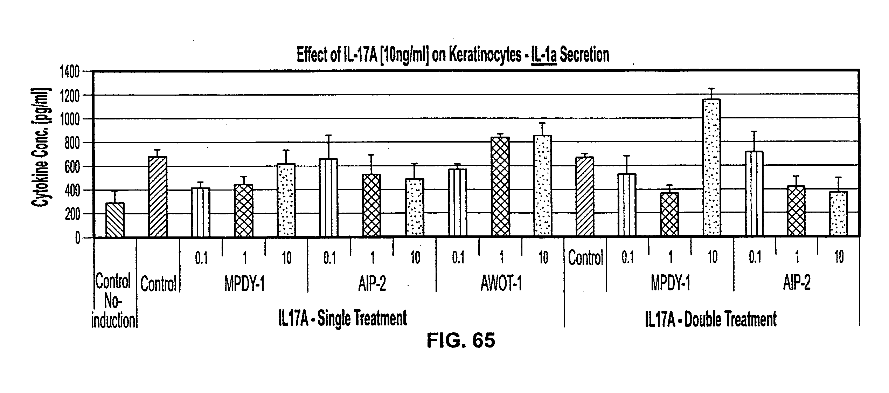

Figure 65 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

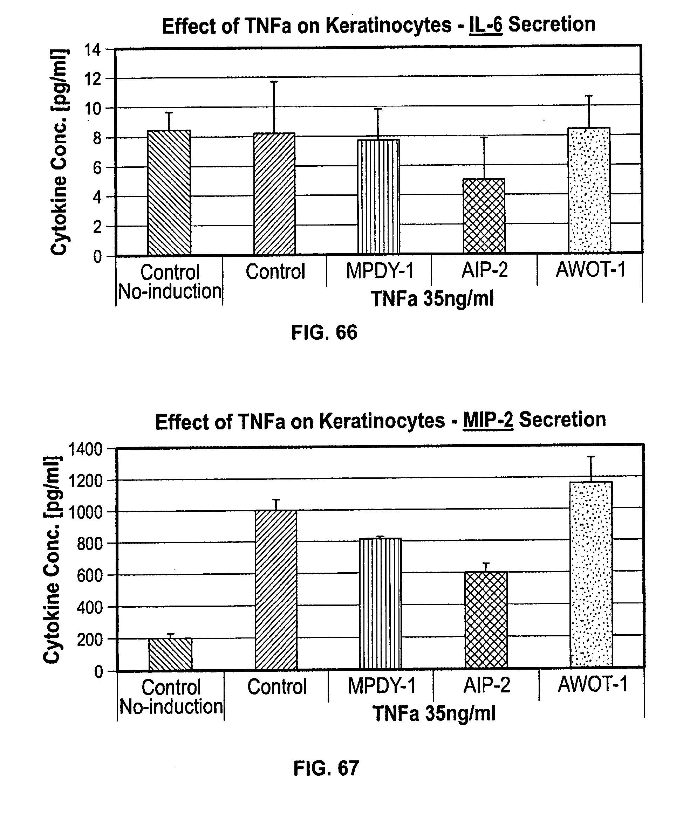

Figure 66 is a histogram comparing cytokine secretion in TNFα activated keratinocytes treated with peptide PKCα inhibitors MPDY-1 (SEQ ID NO: 6), AWOT-1 (SEQ ID NO: 7), and AIP-2 (SEQ ID NO: 8).

Figure 67 is a histogram comparing cytokine secretion in TNFα activated keratinocytes treated with peptide PKCα inhibitors MPDY-1 (SEQ ID NO: 6), AWOT-1 (SEQ ID NO: 7), and AIP-2 (SEQ ID NO: 8).

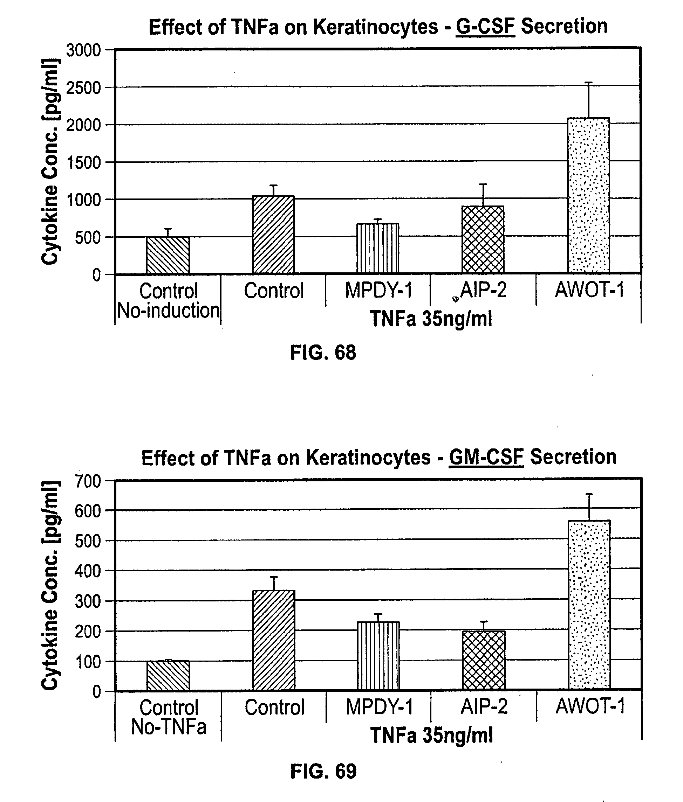

Figure 68 is a histogram comparing cytokine secretion in TNFα activated keratinocytes treated with peptide PKCα inhibitors MPDY-1 (SEQ ID NO: 6), AWOT-1 (SEQ ID NO: 7), and AIP-2 (SEQ ID NO: 8).

Figure 69 is a histogram comparing cytokine secretion in TNFα activated keratinocytes treated with peptide PKCα inhibitors MPDY-1 (SEQ ID NO: 6), AWOT-1 (SEQ ID NO: 7), and AIP-2 (SEQ ID NO: 8).

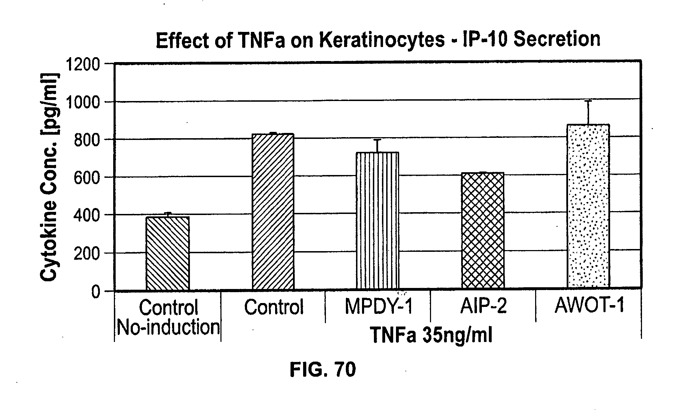

Figure 70 is a histogram comparing cytokine secretion in TNFα activated keratinocytes treated with peptide PKCα inhibitors MPDY-1 (SEQ ID NO: 6), AWOT-1 (SEQ ID NO: 7), and AIP-2 (SEQ ID NO: 8).

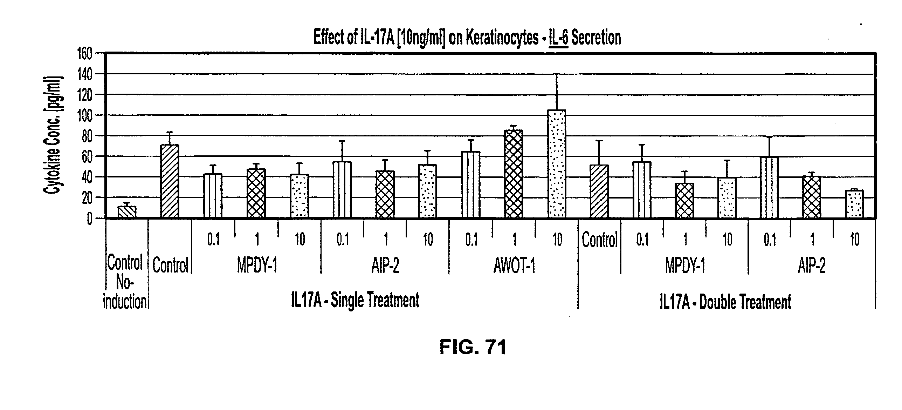

Figure 71 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

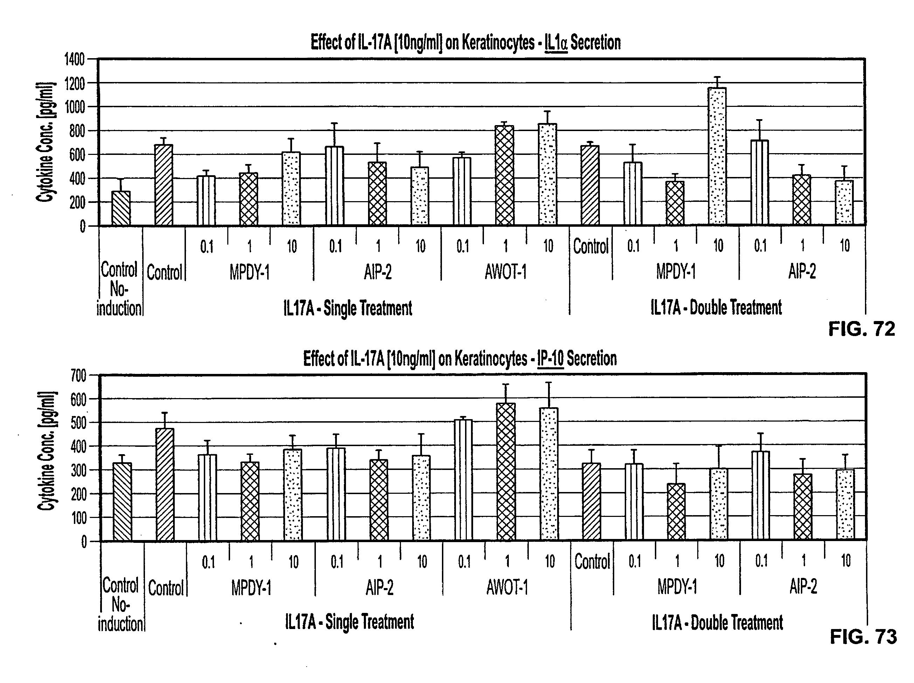

Figure 72 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

Figure 73 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

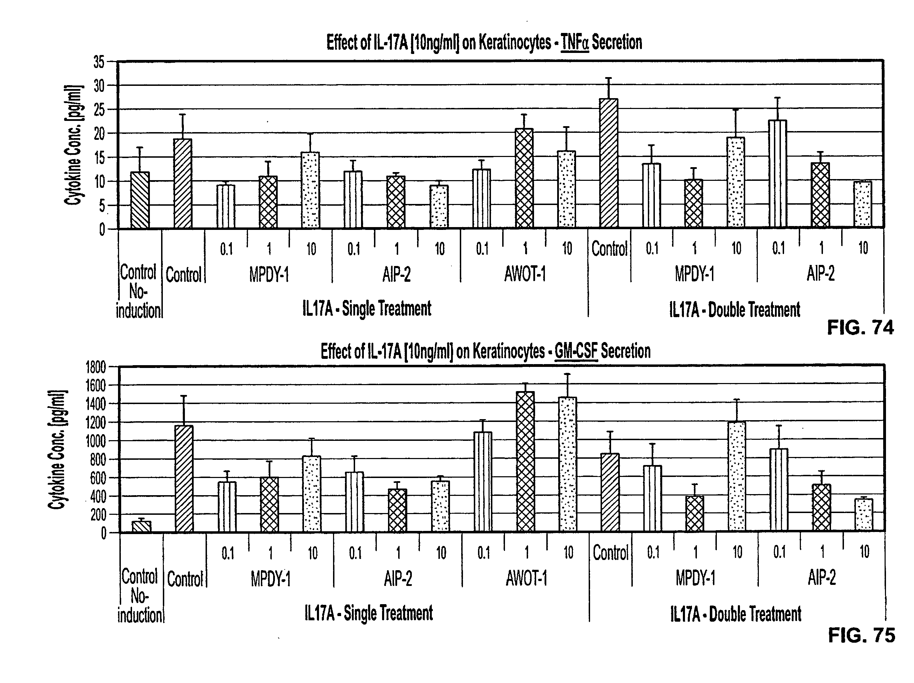

Figure 74 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

Figure 75 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

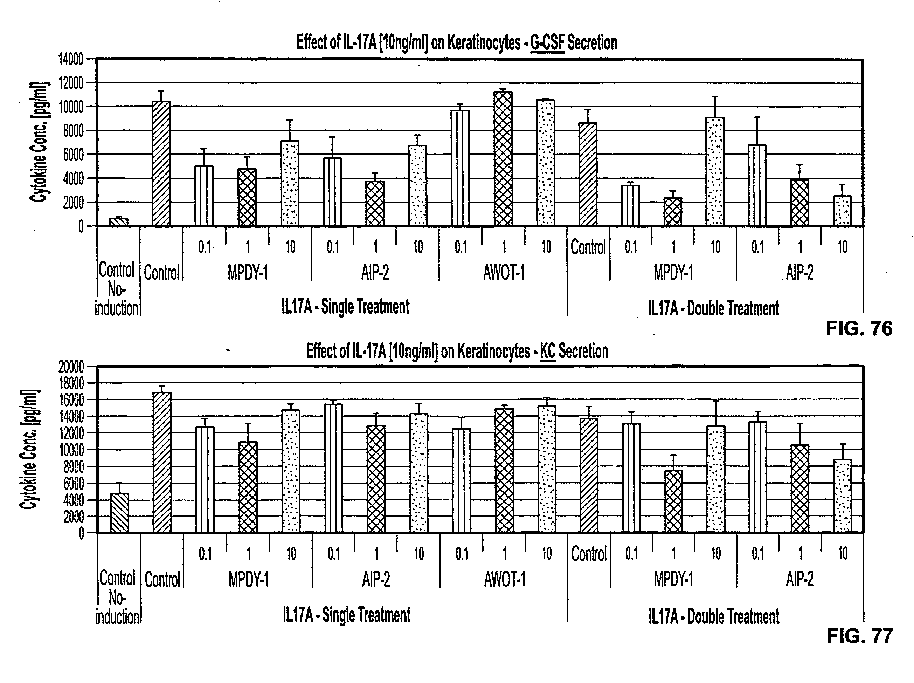

Figure 76 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

Figure 77 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

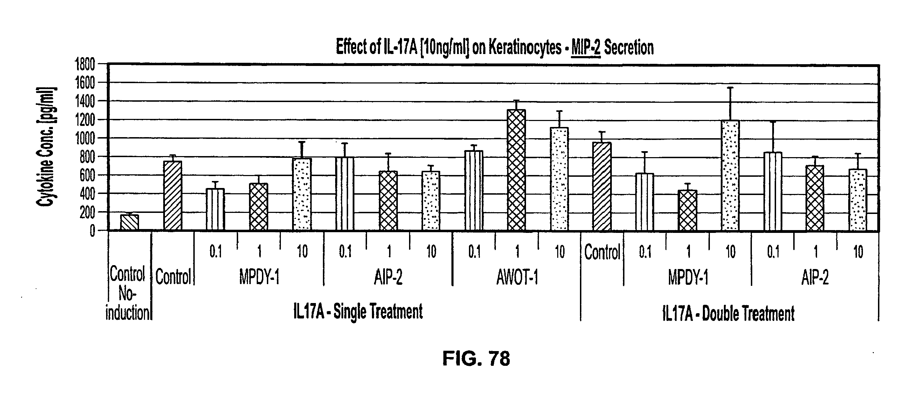

Figure 78 is a histogram comparing cytokine secretion in IL-17A activated keratinocytes treated with peptide PKCα inhibitors MPDY-1 (SEQ ID NO: 6), AWOT-1 (SEQ ID NO: 7), and AIP-2 (SEQ ID NO: 8).

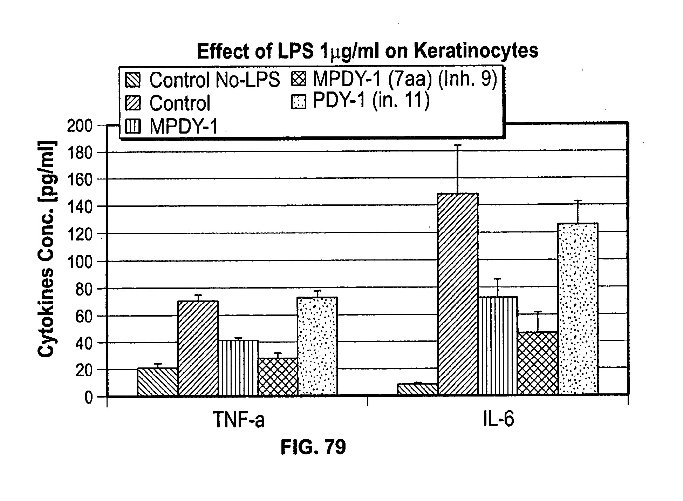

Figure 79 is a histogram comparing cytokine secretion in LPS activated keratinocytes treated with peptide PKCα inhibitors MPDY-1 (SEQ ID NO: 6) and PDY-1 (SEQ ID NO: 13).

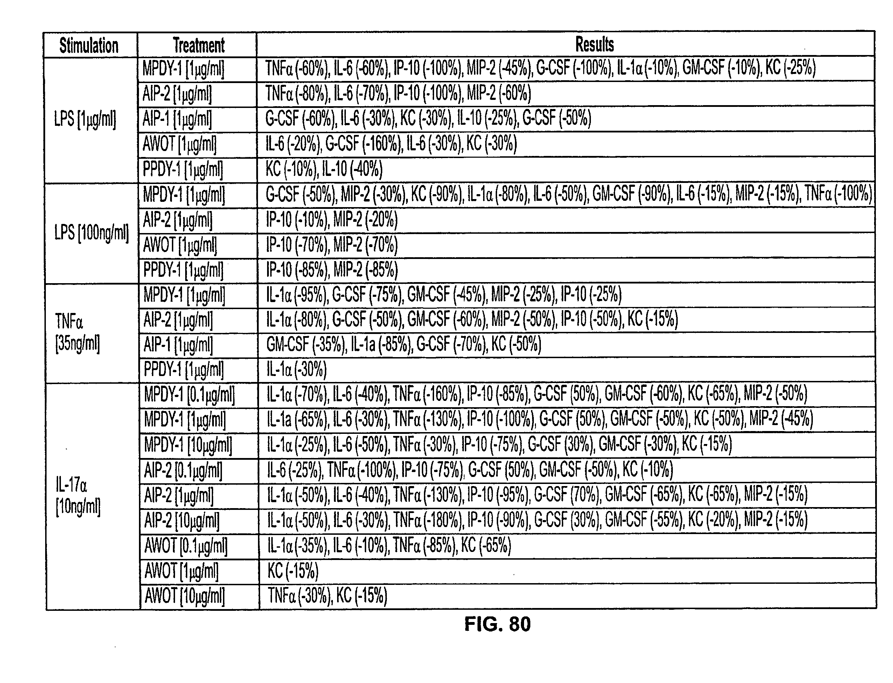

Figure 80 is a tabular summary of results for various PKCα inhibitors of cytokine secretion in keratinocytes treated with LPS, TNFα or IL-17A and inhibitor.

### DETAILED DESCRIPTION OF THE DISCLOSURE

The present disclosure is based on the seminal discovery that modulators of PKC isoforms may be administered as effective treatments for inflammatory disease and disorder. The involvement of PKC isoforms in major cellular processes of skin cells, as well as many components of the immune system, marks it as a potential target for the treatment of inflammatory pathologies. The data presented herein, demonstrate that PKC family isoforms regulate activation processes in skin and immune cells associated with inflammation and inflammatory diseases.

It is to be understood that this disclosure is not limited to particular compositions, methods, and experimental conditions described, as such methods and conditions may vary. It is also to be understood that the terminology used herein is for purposes of describing particular embodiments only, and is not intended to be limiting, as the scope of the present disclosure will be limited only in the appended claims.

The principles and operation of the methods according to the present disclosure may be better understood with reference to the figures and accompanying descriptions.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of skill in the art to which this disclosure belongs. Although any methods and materials similar or equivalent to those described herein can be used in the practice or testing of the disclosure, some preferred methods and materials are now described.

As used herein, the term "subject" refers to a mammalian subject. As such, treatment of any animal in the order mammalian is envisioned. Such animals include, but are not limited to horses, cats, dogs, rabbits, mice, goats, sheep, non-human primates and humans. Thus, the method of the present disclosure is contemplated for use in veterinary applications as well as human use.

"Treatment" of a subject herein refers to both therapeutic treatment and prophylactic or preventative measures. Those in need of treatment include those already with an inflammatory disease or disorder as well as those in which it is to be prevented. Hence, the subject may have been diagnosed as having an inflammatory disease or disorder or may be predisposed or susceptible to an inflammatory disease or disorder.

As used herein, an "inflammatory disease or disorder" is intended to include any disease and disorder having etiologies associated with PKC family isoform regulation. Such diseases include, and are not limited to, pruritus, skin inflammation, psoriasis, multiple sclerosis, rheumatoid arthritis, osteoarthritis, systemic lupus erythematosus, Hashimoto's thyroidis, myasthenia gravis, diabetes type I or II, asthma, inflammatory lung injury, inflammatory liver injury, inflammatory glomerular injury, atopic dermatitis, allergic contact dermatitis, irritant contact dermatitis, seborrhoeic dermatitis, Sjoegren's syndrome, keratoconjunctivitis, uveitis, inflammatory bowel disease, Crohn's disease, ulcerative colitis, an inflammatory disease of the joints, skin, or muscle, acute or chronic idiopathic inflammatory arthritis, myositis, a demyelinating disease, chronic obstructive pulmonary disease, interstitial lung disease, interstitial nephritis and chronic active hepatitis.

A "symptom" of an inflammatory disease or disorder is any morbid phenomenon or departure from the normal in structure, function, or sensation, experienced by the subject and indicative of an inflammatory disease or disorder.

The expression "effective amount" refers to an amount of an inhibitor or activator of a PKC isoform, such as the polypeptides of SEQ ID NOs: 1-37, that is effective for preventing, ameliorating or treating an inflammatory disease or disorder. Such an effective amount will generally result in an improvement in the signs, symptoms and/or other indicators of an inflammatory disease or disorder. For example, in skin inflammation, an effective amount results in the reduction of swelling and/or inflammation and/or clearance of redness. For pruritus, an effective amount may result in the clearance of redness and/or itchiness. For MS, an effective amount, may result in reducing relapse rate, preventing disability, reducing number and/or volume of brain MRI lesions, improving timed 25-foot walk, extending the time to disease progression, and the like.

As used herein, the term "PKC isoform" as used herein encompasses all PKC isoforms including PKCα, PKCβ, PKCδ, PKCε, PKCη, PKCξ, PKCγ, PKCθ, and PKCλ.

The phrase "modulating expression and/or activity of a PKC isoform" relates to an increased or reduced expression and/or activity of a PKC isoform. Increase of the expression leads to increased production of the PKC isoform.

The term "activator" is used herein to describe a molecule that enhances expression and/or activity of a PKC isoform. The term "inhibitor" is used herein to describe a molecule that inhibits expression and/or activity of a PKC isoform. Among others, the phosphoryl transfer region, the pseudosubstrate domain, the phorbolester binding sequences, and the phosphorylation sites may be targets for modulation of isoenzyme-specific PKC activity.

The "pseudosubstrate region" or autoinhibitory domain of a PKC isoform is herein defined as a consensus sequence of substrates for the kinase with essentially no phosphorylatable residue. The pseudosubstrate domain is based in the regulatory region, closely resembling the substrate recognition motif, which blocks the recognition site and prevents phosphorylation. Thus, inhibitory peptides of PKC isoforms, such as the polypeptides of the present disclosure, are obtained as by replacing a phosphorylatable residue of serine (S) or tyrosine (T) by alanine (A). PKCδ is the only PKC isoform known to have additional binding site enabling the isoform's activation on the C2 domain, the conserved domain 2 of PKCδ.

PKC is a major signaling pathway, which mediates keratinocyte proliferation, migration and differentiation. Many PKC isoforms are known to be expressed in skin tissue and their expression/activity appears to play a role in cell proliferation and/or cell migration and/or cell differentiation. However, their specific modulation of expression and activity to effectuate treatment of inflammatory diseases was previously unknown and is demonstrated in the present disclosure.

Overall, the results presented herein demonstrate that modulating expression and/or activity of distinct PKC isoforms is effective in treatment of inflammation and inflammatory disease.

Thus, in one aspect, the present disclosure provides a method of treating an inflammatory disease or disorder in a subject. The method includes administering to the subject an inhibitor of PKC, thereby treating the inflammatory disease or disorder in the subject. In exemplary embodiments, the inhibitor is a polypeptide which selectively inhibits PKCα, PKCε or PKCη, such as the polypeptides of SEQ ID NOs: 1-29.

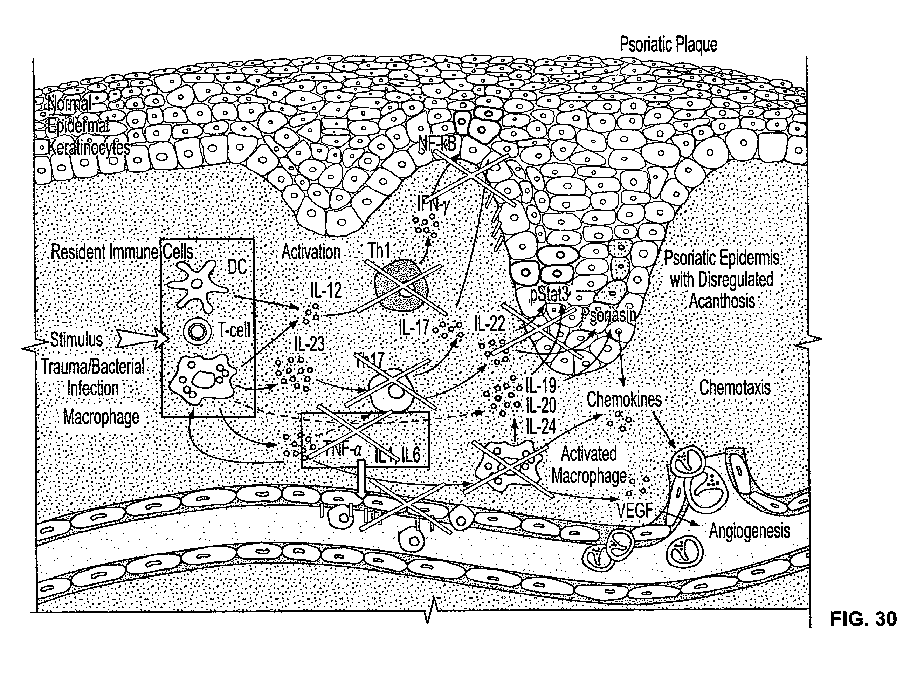

As disclosed in the Examples, administration of PKC isoform inhibitors has been shown to reduce secretion of pro-inflammatory cytokines, chemokines and Th1 cytokines in a variety of different skin cell types (not just skin cells i.e. macrophages are found and active in other tissues). In addition, administration of PKC isoform reduce the expression of activating factors such as ICAM-1 on keratinocytes and endothelial cells and mac-2 on macrophages. Additionally, PKCα inhibitors have been found effective in the treatment of skin inflammation and to attenuate the inflammatory symptoms in inflammatory skin models of psoriasis. As discussed further in the Examples, the mechanism of action of inhibitors of PCK isoforms has been elucidated implicating their use as an effective therapy for inflammatory diseases and disorders. For example, peptide inhibitors of PCK isoforms have been shown to: 1) normalize epidermal differentiation marker expression by reducing terminal differentiation; 2) attenuate abnormal hyper-proliferation; 3) regulate skin structure and augment skin strength; and/or 4) down-regulate inflammation by differentially affecting different cell type recruitment and activation in various steps of the inflammatory process as summarized, for example, in Figure 30.

Also, as disclosed in the Examples activators of PKCδ have also been shown to reduce secretion of pro-inflammatory cytokines in a variety of different skin cell types. Thus, in another aspect, the present disclosure provides a method of treating an inflammatory disease or disorder in a subject by administering to the subject an activator of PKCδ, thereby treating the inflammatory disease or disorder in the subject. In various embodiments, the activator is a polypeptide which selectively activates PKCδ, such as the polypeptides of SEQ ID NOs: 30-37.

Additionally, as disclosed in the Examples, administration of PKCα inhibitors and PKCη inhibitors has been found to attenuate the symptoms of MS. As such, in another aspect, the present disclosure provides a method of treating multiple sclerosis in a subject. The method includes administering to the subject an inhibitor of PKCα or PKCη, thereby treating multiple sclerosis in the subject.

Further, administration of PKC isoform inhibitors has been found effective in the treatment of pruritus. As such, in another aspect, the present disclosure provides a method of treating an pruritus in a subject. The method includes administering to the subject an inhibitor of PKC, thereby treating pruritus in the subject.

The Examples and Figures present data showing the ability of activators of PKCδ to inhibit the secretion of major pro-inflammatory cytokines, such as IL-1, IL-6 and TNFα. Similar data is shown for a variety of PKC isoform inhibitors, including PKCα, PKCε and PKCη. As shown in the Examples, formulations including the PKC inhibitors and activators of the present disclosure, have been shown to inhibit the secretion of major pro-inflammatory cytokines. As regards psoriasis, without being bound to a particular theory, it is believed that reducing the level of pro-inflammatory agents prevents the activation of endothelial cells in near-by blood vessels, and thus the recruitment of neutrophiles, macrophages and T cells to the psoriatic plaque. Moreover, TH1 and TH17 cells were shown to be implicated in the pathogenesis of psoriasis by the secretion of specific cytokines, which appear to enhance inflammation or drive keratinocyte hyperproliferation, respectively. The above mentioned pro-inflammatory cytokines appear essential for the development of these TH17 cells (Mangan et al. (2006) Nature 441:231-234; Bettelli et al. (2006) Nature 441:235-238) and for TH1 cell activity. The decrease of their secretion by PKC inhibitors and activators implicates their use in the effective treatment of inflammatory disorders and pruritus.

In various embodiments, the inhibitors of PKC isoforms are inhibitors of the pseudosubstrate region of PKC and are polypeptides, while the activators of PKC isoforms are also polypeptides. The terms "polypeptide", "peptide", or "protein" are used interchangeably herein to designate a linear series of amino acid residues connected one to the other by peptide bonds between the alpha-amino and carboxy groups of adjacent residues.

In various embodiments, Examples of peptide PKC activators and inhibitors that can be used include, without being limited to, peptides of SEQ ID NOs: 1-5, 14-19, 26, 27 and 30-33 as shown in Table 1 or physiologically acceptable salts thereof, as well as the peptides of SEQ ID NOs: 6-13, 20-25, 28, 29, 34-37 of Table 1 which are shown having particular modifications or terminal protecting groups.

**Table 1: PKC Isoform Inhibitor and Activator Peptides**

| **Amino Acid Sequence** | **SEQ ID NO** |
|---|---|
| **PKCα Inhibitors** | |
| Phe-Ala-Arg-Lys-Gly-Ala | **1** |
| Phe-Ala-Arg-Lys-Gly-Ala-Leu-Arg-Gln | **2** |
| Phe-Ala-Arg-Lys-Gly-Ala-Leu | **3** |
| Phe-Ala-Arg-Lys-Gly-Ala-Arg-Gln | **4** |
| Thr-Leu-Asn-Pro-Gln-Trp-Glu-Ser | **5** |
| Myristoyl-Phe-Ala-Arg-Lys-Gly-Ala-Leu-Arg-Gln-OH | **6** |
| H-Phe-Ala-Arg-Lys-Gly-Ala-Leu-Arg-Gln-OH | **7** |
| Myristoyl-Phe-Ala-Arg-Lys-Gly-Ala-Leu-OH-trifluoracetate salt | **8** |
| H-Thr-Leu-Asn-Pro-Gln-Trp-Glu-Ser-OH | **9** |
| Palmitoyl-Phe-Ala-Arg-Lys-Gly-Ala-Leu-Arg-Gln-OH-acetate salt | **10** |
| Palmitoyl-Phe-Ala-Arg-Lys-Gly-Ala-Arg-Gln-OH | **11** |
| Myristoyl-Phe-Ala-Arg-Lys-Gly-Ala-Leu-OH | **12** |
| H-Phe-Ala-Arg-Lys-Gly-Ala-Leu-Arg-Gln-OH-acetate salt | **13** |

| **PKCε Inhibitors** | |
|---|---|
| Glu-Ala-Val-Ser-Leu-Lys-Pro-Thr | **14** |
| Pro-Tyr-Ile-Ala-Leu-Asn-Val-Asp | **15** |
| Pro-Ala-Trp-His-Asp | **16** |
| Leu-Glu-Pro-Glu- Ala-Ala- Ala-Ala- Ala-Ala-Gly-Lys | **17** |
| His-Phe-Glu-Asp-Trp-Ile-Asp | **18** |
| Val-Tyr-Val-Ile-Ile-Asp-Leu | **19** |
| H-Glu-Ala-Val-Ser-Leu-Lys-Pro-Thr-OH | **20** |
| H-Pro-Tyr-Ile-Ala-Leu-Asn-Val-Asp-OH | **21** |
| H-Pro-Ala-Trp-His-Asp-OH | **22** |
| H-Leu-Glu-Pro-Glu- Ala-Ala-Ala-Ala- Ala-Ala-Gly-Lys-OH | **23** |
| H-His-Phe-Glu-Asp-Trp-Ile-Asp-OH | **24** |
| H-Val-Tyr-Val-Ile-Ile-Asp-Leu-OH | **25** |

| **PKCη Inhibitors** | |
|---|---|
| Thr-Arg-Lys-Arg-Gln-Arg-Ala-Met-Arg-Arg-Arg-Val-His-Gln-Ile-Asn-Gly | **26** |
| Lys-Arg-Thr-Leu-Arg | **27** |
| Myristoyl-Thru-Arg-Lys-Arg-Gln-Arg-Ala-Met-Arg-Arg-Arg-Val-His-Gln-Ile-Asn-Gly-OH | **28** |
| Myristoyl-Lys-Arg-Thr-Leu-Arg-OH | **29** |

| **PKCδ Activators** | |
|---|---|
| His-Phe-Glu-Asp-Trp-Ile-Asp | **30** |
| His-Phe-Glu-Asp-Trp-Ile-Asp-His-Phe-Glu-Asp-Trp-Ile-Asp | **31** |
| Met-Arg-Ala-Ala-Glu-Ala-Ala-Ala-Ala-Glu-Pro-Met | **32** |
| Val-Tyr-Val-Ile-Ile-Asp-Leu- His-Phe-Glu-Asp-Trp-Ile-Asp | **33** |
| H-His-Phe-Glu-Asp-Trp-Ile-Asp-His-Phe-Glu-Asp-Trp-Ile-Asp-OH | **34** |
| H-Met-Arg-Ala-Ala-Glu-Ala-Ala-Ala-Ala-Glu-Pro-Met-OH | **35** |
| H-His-Phe-Glu-Asp-Trp-Ile-Asp-OH | **36** |
| H-Val-Tyr-Val-Ile-Ile-Asp-Leu- His-Phe-Glu-Asp-Trp-Ile-Asp-OH | **37** |

| **PKCξ Inhibitor** | |
|---|---|
| Ser-Ile-Tyr-Arg-Arg-Gly-Ala-Arg-Arg-Trp-Arg-Lys-Leu | **38** |
| Myristoyl-Ser-Ile-Tyr-Arg-Arg-Gly-Ala-Arg-Arg-Trp-Arg-Lys-Leu-OH | **39** |

In various embodiments, the peptide PKC inhibitors or activators typically contain between 6 and 12 amino acids, but may be longer or shorter in length. In various embodiment, a peptide PKC inhibitor or activator may range in length from 6 to 45, 6 to 40, 6 to 35, 6 to 30, 6 to 25, 6 to 20, 6 to 15, or 6 to 10 amino acids. In one embodiment the peptide includes 6, 7, 8, 9, 10, 11, 12, 13, 14 or 15 amino acids.

In various embodiments, the peptide PKC inhibitors or activators may be N-acylated, preferably by an acyl group derived from a C12-C20 fatty acid, such as C14 acyl (myristoyl) or C16 acyl (palmitoyl).

In general, peptide PKCα inhibitors include the common motif sequence Phe-Ala-Arg-Lys-Gly-Ala (SEQ ID NO: 1). Alternatively, in another embodiment, PKCα inhibitors include the common motif sequence Thr-Leu-Asn-Pro-Gln-Trp-Glu-Ser (SEQ ID NO: 5).

While the peptide PKC inhibitors and activators may be defined by exact sequence or motif sequences, one skilled in the art would understand that peptides that have similar sequences may have similar functions. Therefore, peptides having substantially the same sequence or having a sequence that is substantially identical or similar to a PKC inhibitor or activator of Table 1 are intended to be encompassed. As used herein, the term "substantially the same sequence" includes a peptide including a sequence that has at least 60+% (meaning sixty percent or more), preferably 70+%, more preferably 80+%, and most preferably 90+%, 95+%, or 98+% sequence identity with the sequences defined by SEQ ID NOs: 1-37 and inhibit or activate PKC isoform activity.

A further indication that two polypeptides are substantially identical is that one polypeptide is immunologically cross reactive with that of the second. Thus, a polypeptide is typically substantially identical to a second polypeptide, for example, where the two peptides differ only by conservative substitutions.

The term "conservative substitution" is used in reference to proteins or peptides to reflect amino acid substitutions that do not substantially alter the activity (for example, antimicrobial activity) of the molecule. Typically conservative amino acid substitutions involve substitution of one amino acid for another amino acid with similar chemical properties (for example, charge or hydrophobicity). The following six groups each contain amino acids that are typical conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K) 5) Isoleucine (I), Leucine (L), Methionine (M), Valine (V); and 6) Phenylalanine (F), Tyrosine (Y), and Tryptophan (W).

The term "amino acid" is used in its broadest sense to include naturally occurring amino acids as well as non-naturally occurring amino acids including amino acid analogs. In view of this broad definition, one skilled in the art would know that reference herein to an amino acid includes, for example, naturally occurring proteogenic (L)-amino acids, (D)-amino acids, chemically modified amino acids such as amino acid analogs, naturally occurring non-proteogenic amino acids such as norleucine, and chemically synthesized compounds having properties known in the art to be characteristic of an amino acid. As used herein, the term "proteogenic" indicates that the amino acid can be incorporated into a protein in a cell through a metabolic pathway.

The terms "identical" or percent "identity" in the context of two polypeptide sequences, refer to two or more sequences or subsequences that are the same or have a specified percentage of amino acid residues that are the same, when compared and aligned for maximum correspondence, as measured using a sequence comparison algorithm or by visual inspection.

The phrase "substantially identical," in the context of two polypeptides, refers to two or more sequences or subsequences that have at least 60+%, preferably 80+%, most preferably 90-95+% amino acid residue identity, when compared and aligned for maximum correspondence, as measured using a sequence comparison algorithm or by visual inspection.

As is generally known in the art, optimal alignment of sequences for comparison can be conducted, for example, by the local homology algorithm of Smith & Waterman ((1981) Adv Appl Math 2:482), by the homology alignment algorithm of Needleman & Wunsch ((1970) J Mol Biol 48:443), by the search for similarity method of Pearson & Lipman ((1988) Proc Natl Acad Sci USA 85:2444), by computerized implementations of these algorithms by visual inspection, or other effective methods.

Peptide PKC inhibitors or activators may have modified amino acid sequences or non-naturally occurring termini modifications. Modifications to the peptide sequence can include, for example, additions, deletions or substitutions of amino acids, provided the peptide produced by such modifications retains PKCα inhibitory activity. Additionally, the peptides can be present in the formulation with free termini or with amino-protected (such as N-protected) and/or carboxy-protected (such as C-protected) termini. Protecting groups include: (a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Additional protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl.

In various embodiments, peptide PKC isoform inhibitors and activators may be administered by any suitable means, including topical, parenteral, subcutaneous, intraperitoneal, intrapulmonary, intranasal, intravenous, and/or intralesional administration in order to treat the subject. However, in exemplary embodiments, the peptides are formulated for topical application, such as in the form of a liquid, cream, gel, ointment, foam spray or the like.

Therapeutic formulations of the PKC isoform inhibitor or activator used in accordance with the present disclosure are prepared, for example, by mixing a PKC isoform inhibitor or activator having the desired degree of purity with optional pharmaceutically acceptable carriers, excipients and/or stabilizers (see, for example: Remington's Pharmaceutical Sciences, 16th edition, Osol, A. Ed. (1980)). Acceptable carriers, excipients, or stabilizers are nontoxic to recipients at the dosages and concentrations employed, and may include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (for example, Zn-protein complexes); and/or non-ionic surfactants such as TWEEN™, PLURONICS™ or polyethylene glycol (PEG).

In exemplary embodiments, the PKC isoform inhibitor or activator, are formulated in a cream. The inhibitors and activators of PKC isoforms are ideal for topical treatment of skin inflammation and other inflammatory disorders since the activity of PKC enzymes, may be specifically targeted. Inhibition or activation of specific PKC enzymes is achieved by the ability to selectively modulate a PKC isoform in lower concentrations, without affecting other PKC isoforms.

An exemplary formulation for topical administration is disclosed in Example 4, in which the peptide MPDY-1 (SEQ ID NO: 6) is formulated as a cream for topical administration. However, one skilled in the art would understand that alterations of the formulation may be made while retaining the essential characteristics of the cream, such as viscosity, stabilization, non-toxicity and the like. Also, one skilled in the art would recognize that the formulation may be used as a vehicle for any of the peptide PKC inhibitors or activators of the present disclosure.

In another embodiment, an article of manufacture, such as a kit containing materials useful for carrying out the treatment method of the disclosure is provided. In various embodiments, the kit includes a PKC isoform activator or inhibitor, namely a peptide PKC isoform inhibitor or activator as disclosed herein, and instructions for administering the activator or inhibitor to the subject.

The term "instructions" or "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products, and the like.

As disclosed herein, the inhibitor of PKCα may be formulated for a specific route of administration. As such, the kit may include a formulation including an inhibitor of PKCα that is contained in a suitable container, such as, for example, tubes, bottles, vials, syringes, and the like. The containers may be formed from a variety of materials such as glass or plastic. The container holds or contains a composition that is effective for treating the inflammatory disease and may have a sterile access port (for example the container may be an intravenous solution bag or a vial having a stopper pierceable by a hypodermic injection needle). At least one component in the formulation is an inhibitor or activator of a PKC isoform. The label or package insert indicates that the composition is used for treating inflammatory disease in a subject suffering therefrom with specific guidance regarding dosing amounts and intervals for providing the formulation including an inhibitor or activator of a PKC isoform. The article of manufacture may further include other materials desirable from a commercial and user standpoint, including other buffers, diluents, filters, needles, and syringes.

It will be understood, that the specific dose level and frequency of dosage for any particular subject in need of treatment may be varied and will depend upon a variety of factors including the activity of the PKC isoform inhibitor or activator employed, the metabolic stability and length of action of that compound, the age, body weight, general health, sex, diet, mode and time of administration, the severity of the particular condition, and the host undergoing therapy. Generally however, dosage will approximate that which is typical for known methods of administration of the specific PKC isoform inhibitor or activator. Persons of skill in the art can easily determine optimum dosages, dosing methodologies and repetition rates. The exact formulation and dosage can be chosen by the individual physician in view of the patient's condition (Fingl et al. "The Pharmacological Basis of Therapeutics", Ch. 1 p. 1 (1975)).

Thus, depending on the severity and responsiveness of the condition to be treated, dosing can be a single or repetitive administration, with course of treatment lasting from several days to several weeks or until cure is effected or diminution of the disorder is achieved.

In various embodiments where the PKC isoform inhibitor or activator is a peptide, the peptide is provided in the composition at a concentration of between 0.001 and 100 µg/ml. For example, the concentration may be between 0.001 and 100,0.01 and 50, 0.01 and 10, 0.01 and 1, and 0.01 and 0.5 µg/ml.

In one dosing protocol, the method comprises administering a peptide PKC isoform inhibitor or activator to the subject topically, for example as a cream. The peptide is topically applied at a concentration of from about 1 µg/ml to about 1000 µg/ml, 1 µg/ml to about 500 µg/ml, 1 µg/ml to about 100 µg/ml, 1 µg/ml to about 10 µg/ml, or 10 µg/ml to about 100 µg/ml. The peptide is administered at least once daily until the condition is treated.

In another dosing protocol, the method comprises administering a peptide PKC isoform inhibitor or activator to the subject parentally, subcutaneously or intravenously. The peptide is applied in a concentration of from about 1 µg/ml to about 1000 µg/ml, 1 µg/ml to about 500 µg/ml, 1 µg/ml to about 100 µg/ml, 1 µg/ml to about 10 µg/ml, or 10 µg/ml to about 100 µg/ml. The peptide is administered at least once daily, weekly, biweekly, or monthly until the condition is treated.

The following examples are provided to further illustrate the embodiments of the present disclosure, but are not intended to limit the scope. While they are typical of those that might be used, other procedures, methodologies, or techniques known to those skilled in the art may alternatively be used.

### EXAMPLE 1

### INHIBITION OF PKCα REGULATES KERATINOCYTE STRUCTURE INTEGRITY CHARACTERISTIC TO INFLAMMATORY SKIN DISORDER PSORIASIS

Inhibition of PKCα was shown to regulate keratinocyte structure integrity characteristic to psoriasis. Skin tissues were paraffin embedded and stained for H&E (hematoxiline and eosine) general histological staining or for distinct markers for the various skin layers including Keratin 14 (K14) for basal layer, Keratin 1 (K1) for spinous layer, Keratin 6 (K6) for keratinocytes migration and PCNA for keratinocytes proliferation. The results demonstrate normalization of skin properties following PKCα inhibition (Figure 2).

### EXAMPLE 2

### MODELS FOR ASSESSING IN VIVO AND EX VIVO TREATMENT OF INFLAMMATION VIA PSORIASIS MODELS

Numerous animal models have been previously used to study psoriasis, however, none of these models were sufficient to adequately mimic the human disease pathology characterized by excessive skin production, formation of new blood vessels, and severe immune dysfunction. In general, to be considered as a useful model of psoriasis, the model has to share some histopathology features with psoriasis, exhibit similar pathogenesis and/or disease mechanism, and respond similarly to therapeutic agents for the treatment of the disease Existing models exhibit several characteristics including acanthosis, altered epidermal differentiation, increase in vascularization, and Leukocytic/T cell infiltration. However, among the existing mice models, not many respond to existing drugs and therapies. As such, existing models were used to develop new *in-vitro, ex-vivo* and *in-vivo* models to assess psoriasis treatment which were utilized in the following Examples.

### In Vitro Models

Developed models included cell culture studies using cells lines and primary cultures of skin derived cells as well as immune cells, utilizing constructs and tools to over-express and inactivate STAT3 and PKCα mediated signaling pathways. A vast set of techniques for the study of skin cell proliferation, migration, differentiation, inflammation and signaling were utilized and proved useful in studying the mechanism of psoriasis development and to study the therapeutic effect of PKCα inhibition in psoriasis.

### In Vivo Models

A PKCα over-expressing and knockout mouse models were used. Over-expression of PKCα in keratinocytes using a K5-PKCα transgenic mice, was shown to exhibit severe intra-epidermal neutrophil infiltration and disruption of the epidermis that mimic conditions such as pustular psoriasis. Both PKCα and DN forms of transgenic mice were established which were studied *in vivo* by sub-dermal application. In addition, PKCα knockout mice are also used to study the effects of PKCα inactivation on skin structure and function.

A STAT3 over-expressing mouse model used. Among the leading mice models for psoriasis, in terms of similarity to human psoriasis, is a transgenic mouse in which Stat3, is over-expressed in epidermal keratinocytes. These mice, develop psoriasiform epidermal acanthosis and have a cutaneous lymphocytic infiltrate that is predominantly CD4+ in the dermis, and CD8+ in the epidermis, all are features that are similar to psoriasis in human.

Wound as a model for skin inflammation and hyperplasia. A screening methodology was developed to detect and quantitatively assess inflammation in skin lesions in a wound setting which allows to follow cutaneous inflammatory response in the different skin compartments and identify agents that affect this response.

### Ex Vivo Models

Psoriatic skin grafting on Chick Chorioalantoic Membrane (CAM). A technique of psoriatic skin grafting on Chick Chorioalantoic Membrane (CAM) was developed for the purpose of testing *ex-vivo* treatment applications. While this technique is commonly used for skin tumor studies and angiogenesis experiments, it was adopted and used for psoriasis studies. This original approach allows the application of new drugs directly on human psoriatic skin, thus creating a more clinically relevant study of new drugs for the treatment of psoriasis. Following grafting, psoriatic human skin is utilized to establish efficacy and timing of various treatments in various formulations, analyzed using morphological, histological and biochemical analysis.

### EXAMPLE 3

### ATTENUATION OF SCALING IN PKCα KNOCK OUT MICE

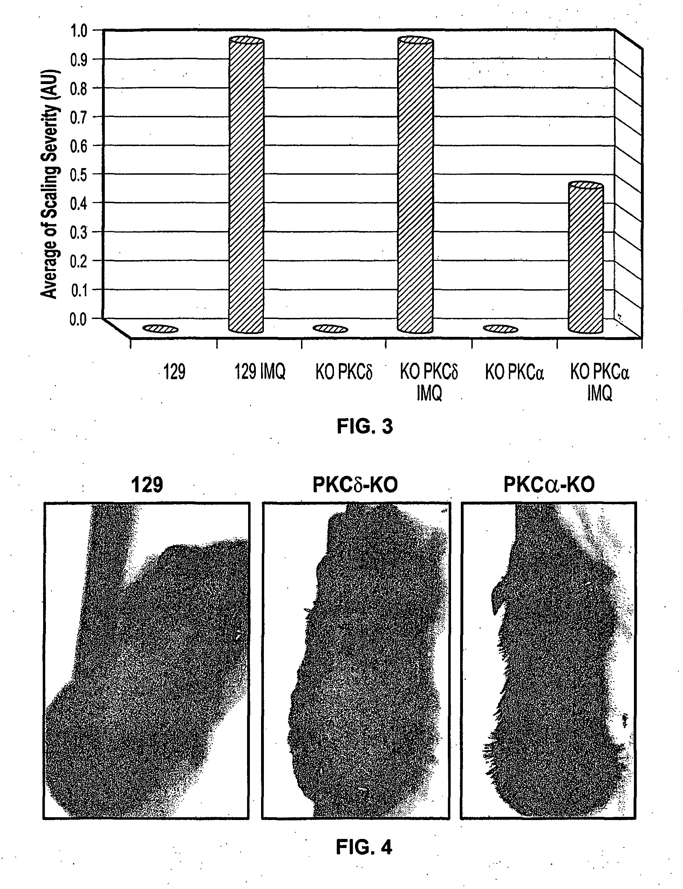

A PKCα knockout mouse model was developed and utilized to study the effects of PKCα inactivation on skin structure and function. As shown in Figures 3 and 4, attenuation of scaling was observed in PCKα knock out mice. Figure 3 is a histogram showing that the average scaling severity was reduced by over 50% in PCKα knock out mice as compared to control evidencing that inhibition of PKCα is a key requirement in treating psoriasis. This is also shown in Figure 4, which is a series of pictures comparing scaling in different mice.

### EXAMPLE 4

### TOPICAL PKCα INHIBITOR FORMULATION

A topical PKCα inhibitor formulation was developed and assessed for effectiveness in treatment of psoriasis. The peptide PKCα inhibitor MPDY-1 (SEQ ID NO: 6) was formulated in a cream (referred to herein as HO/02/10), the components of which are shown in Table 2.

**Table 2: MPDY-1 Cream Based Formulation**

| **INGREDIENTS** |
|---|
| Water |
| Glycerine |
| Propylene Glycol |
| Methylparaben |
| Phenoxyethanol |
| Glyceryl Stearate SE |
| Cetyl Alcohol |
| Cosbiol |
| PEG-40 Stearath |
| Sucrose Distearate |
| Isopropyl Myristate |
| Butylated Hydroxy Toluene |
| Paraffin Oil |
| Capric / Caprylic Triglyceride |
| Vaseline |
| Propylparaben |
| MPDY-1 |

### EXAMPLE 5

### EFFECT OF PKCα INHIBITORS ON IN VITRO EPIDERMAL DIFFERENTIATION

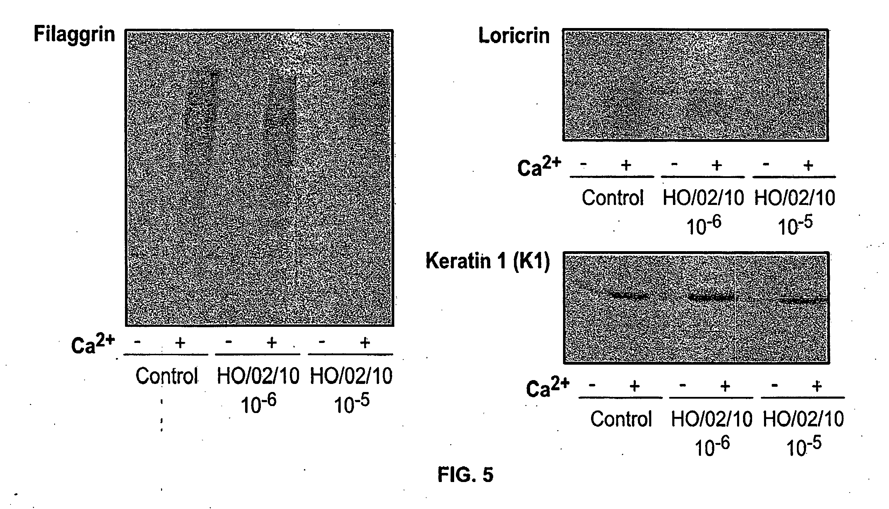

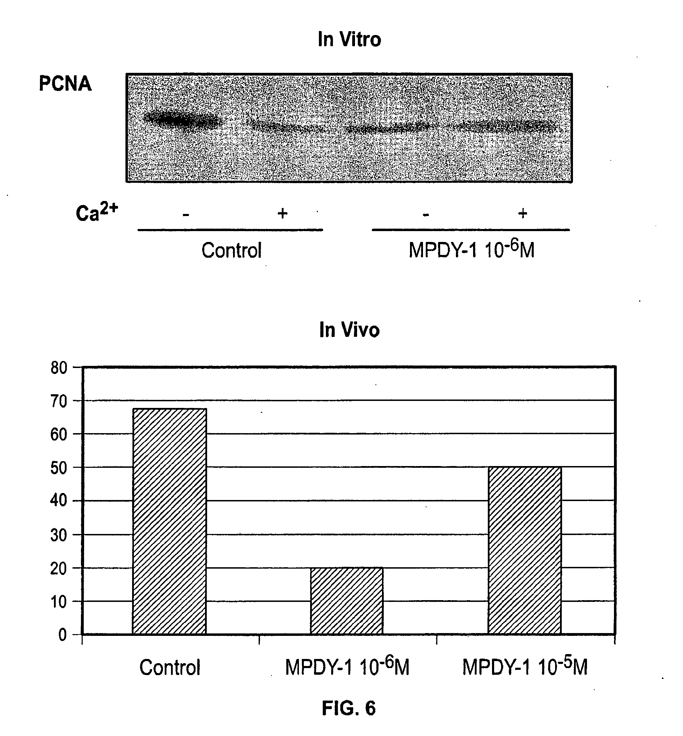

The formulation of Example 4 (HO/02/10) was determined to control epidermal differentiation *in vitro.* Basal keratinocytes differentiate to form the spinous layer, characterized by K1/K10 keratins, the granular layer that is characterized by Loricrin /Filaggrin and the stratum corneum. Defects in expression and incorporation of Loricrin and Filaggrin filaments are associated with various immunological skin diseases including psoriasis. Thus, the effects of HO/02/1 0, were assessed on skin differentiation and proliferation. As shown in Figures 5 and 6, HO/02/10 normalized skin proliferation (PCNA) (Figure 6) and regulated skin differentiation by reducing the expression of Loricrin and Filaggrin, while spinous layer remained unaffected (Figure 5). Since psoriatic skin keratinocytes differentiate rapidly to produce granular and mainly large amounts of corneal cells (scales), while the spinous layer thins, HO/02/10 served to normalize psoriatic skin by amending the skin characteristics toward a normal phenotype.

Figure 5 shows that HO/02/10 controls epidermal granular differentiation *in vitro.* Keratinocytes derived from C57BL/6J mice were incubated in medium containing Ca²⁺ to induce keratinocytes differentiation. Cells were then incubated in the presence of HO/02/10 (1 µg/ml). Cells were harvested, run on SDS PAGE gel and immunoblotted using anti-Filaggrin (Fil), anti Loricrin (Lor) and anti-Keratin 1 (K1) antibody.

Figure 6 shows that HO/02/10 reduced keratinocytes *proliferation in vitro* and *in vivo.* Primary murine keratinocytes from 2 day Balb/c mice were grown for 5 days to reach full confluence in 0.05mM Ca²+MEM medium. HO/02/10 treatment (10⁻⁶M and 10⁻⁵M) was applied 6h prior to induction of differentiation. Cells were harvested, run on SDS PAGE gel and immunoblotted using anti-PCNA antibodies. Results are shown in Figure 6A. C57Black mice, 8-10 weeks of age were subjected to full thickness wounding in the upper back area to induce epidermis remodeling and differentiation. Following the wounding, mice were treated daily with HO/02/10 (ranged 40-4000 mg/kg/day) for 7 days. At the termination point, mice were euthanized and upper back skin samples were fixed in 4% paraformaldehyde solution, following paraffin embedding and slide preparation. Skin samples were then subjected to immunohistochemical staining utilizing PCNA antibody. (n=18). The results are shown in Figure 6B.

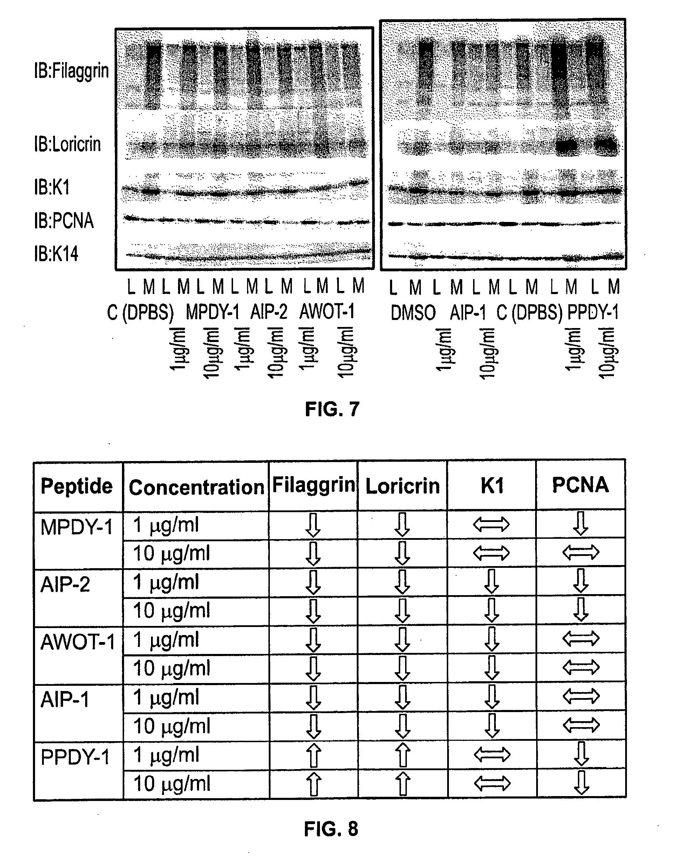

Figures 7 and 8 show additional expression data in keratinocytes utilizing MPDY-1 (SEQ ID NO: 6) as well as data for the peptide PKCα inhibitors AIP-1 (SEQ ID NO: 9), AIP-2 (SEQ ID NO: 8), AWOT-1 (SEQ ID NO: 7) and PPDY-1 (SEQ ID NO: 10). Figure 7 shows immunohistochemical staining utilizing anti-PCNA, anti-Filaggrin (Fil), anti-Loricrin (Lor), anti-Keratin 1 (K1) and anti-Keratin 14 (K14) antibody in keratinocytes treated with various peptide PKCα inhibitors. Figure 8 presents a summary of expression data in keratinocytes for various peptide PKCα inhibitors.

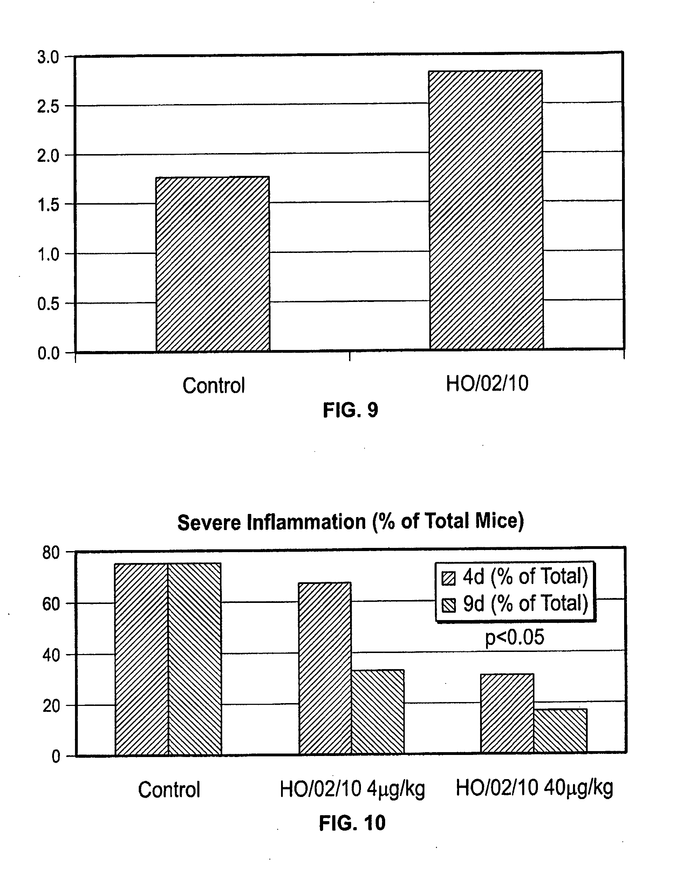

In order to test skin strength and elasticity, a bursting chamber was used to measure the pressure that required for skin samples to burst (a measurable indicator of skin elasticity and durability). The results in Figure 9, demonstrate that HO/02/10 treated skin exhibited enhanced skin strength. Thus, inhibition of PKCα may be beneficial to psoriatic skin as it was shown to enhance skin integrity and prevent bursting of psoriatic lesions.

Figure 9 shows that HO/02/10 dramatically enforced skin strength. Mice skin was treated for 14 days with HO/02/1 0 and subsequently was subjected to bursting pressure analysis. The bursting chamber device consisted of a fixed volume metal cylinder closed on one end and connected to a high-pressure CO² container via a control valve and a manometer. On the other end of the chamber, an adjustable frame was installed in order to mount and hold the tested skin tissue in place. Gas was gradually released into the chamber, and the pressure inside was continuously monitored until bursting of the tested tissue occurs.

### EXAMPLE 6

### EFFECT OF PKC ISOFORM INHIBITORS AND ACTIVATORS ON SKIN INFLAMMATION

A methodology was developed to detect and quantitatively assess inflammation in skin lesions in a wound setting which allows one to follow cutaneous inflammatory response in the different skin compartments and identify agents that affect this response (as a preliminary screening). Inflammatory response was considered severe when two of the following three conditions were evident: (1) abscess formation; (2) excessive leukocytosis (>1 00 cells in a fixed field x200); (3) high WB C/RBC ratio in blood vessels, where >20% of WBC content within the blood vessels is shown in a fixed field x200. Mechanistic characterization of the immunological response is studied utilizing markers to identify infiltration and activation of specific immunological cells. Examples for such markers are: ICAM-1(as a marker activated basal keratinocytes and endothelial cells), MAC-2 (as a marker for activated macrophages) and CD3 (T cell marker). Using this quantitative method, it was possible to demonstrate a strong anti-inflammatory effect of HO/02/10 and other peptide PCKα inhibitors in intact skin and in skin lesions in different cell types and processes in several animal models.

The representative results below demonstrate the anti-inflammatory effect of HO/02/10 on skin wound in B57BL/6J mice after 4 and 9 days post wounds (Figure 10). Figure 10 shows the dose response of HO/02/10 effects on inflammation in C57BL/6J mice. Skins of C57BL/6J mice were treated daily by application of HO/02/10 (4 µg/kg/day) or (40 µg/kg/day) (6 mice/group). Treatments were applied topically. Biopsies were collected 4 and 9 days post-wounding. Tissues were excised from euthanized animals for evaluation of inflammation by histology and immunohistochemistry.

HO/02/10 was also shown to decrease pro-inflammatory cytokine secretion from LPS-activated splenocytes. In order to assess general anti-inflammatory effects *in vitro,* mice-derived primary splenocytes were utilized as an immunological model. Splenocytes were derived from C57BL/6J mice, red blood cells were lysed and cells were incubated at 500,000 per well in a 96 well plate. LPS was added (1µg/ml for IL-1 and TNFα test, and 0.2 ng/ml for IL-6 test), and cells were treated with MPDY-1 (1 µg/ml) or PBS. No LPS was added in negative control samples. Medium was collected after 2 days and the amount of secreted cytokines was quantified using ELISA.

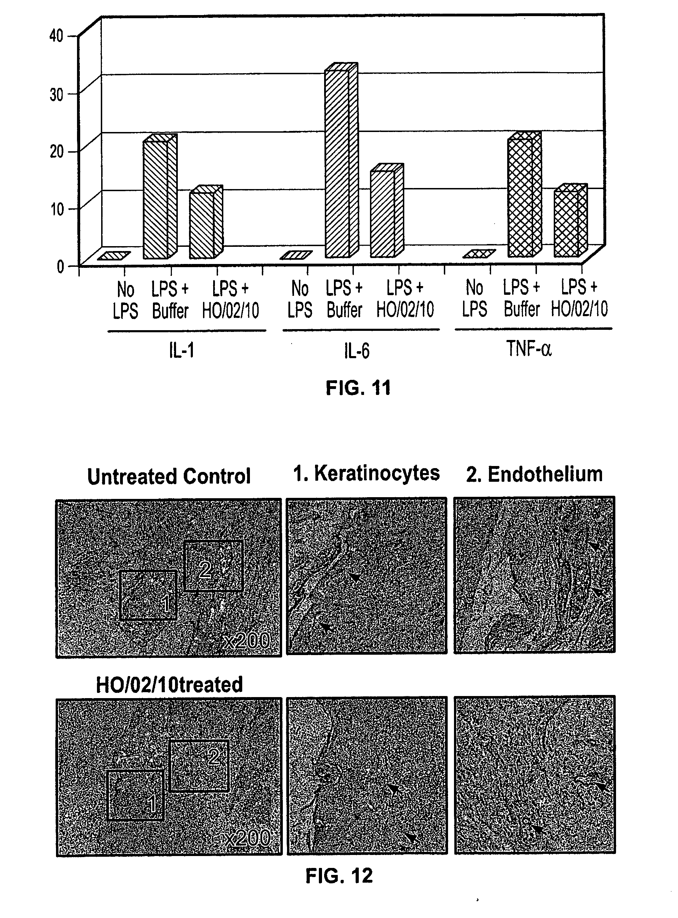

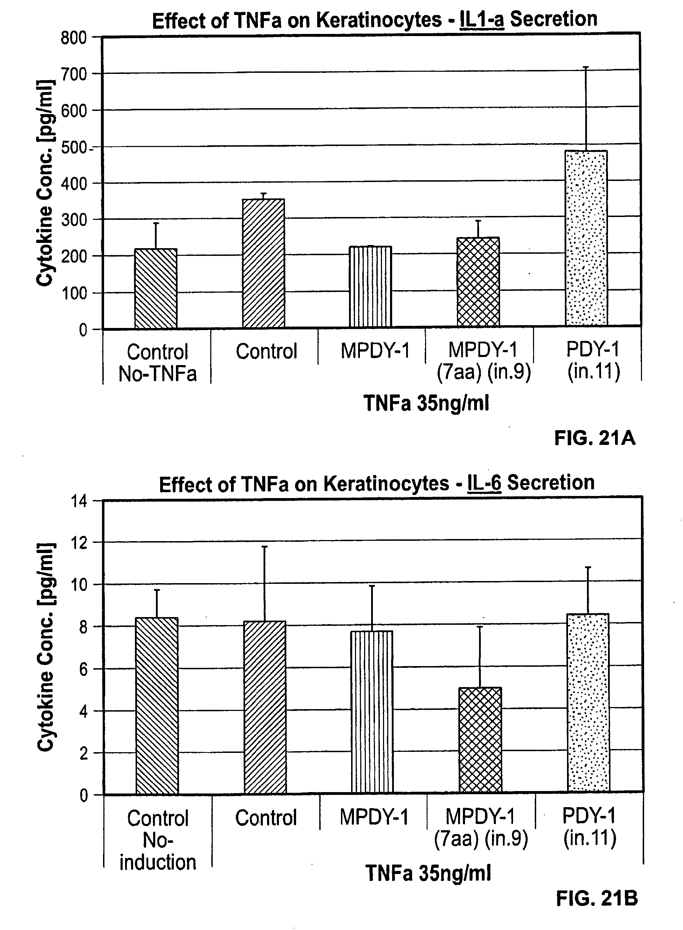

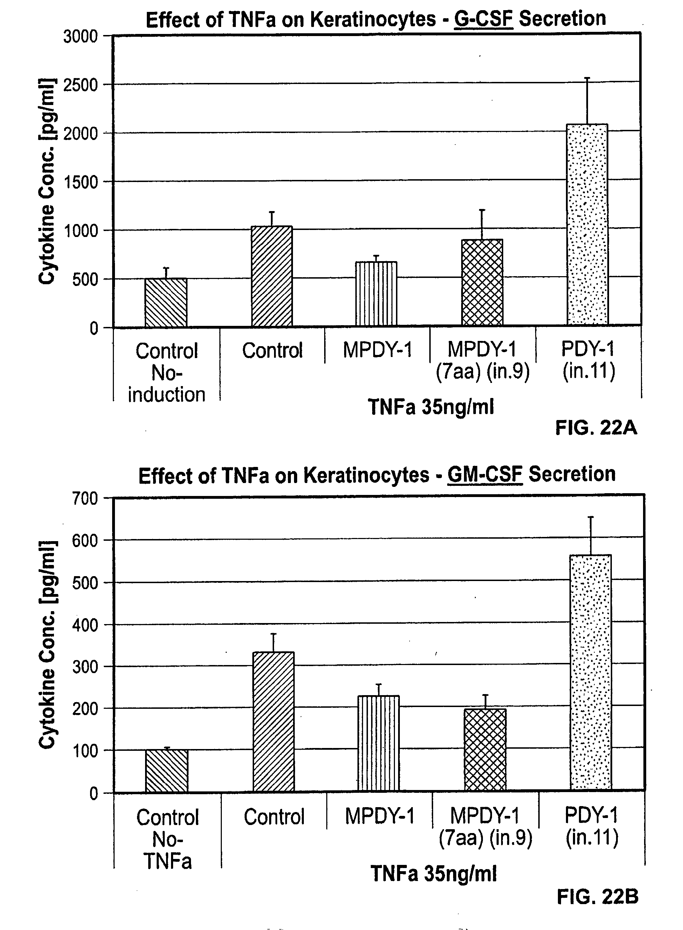

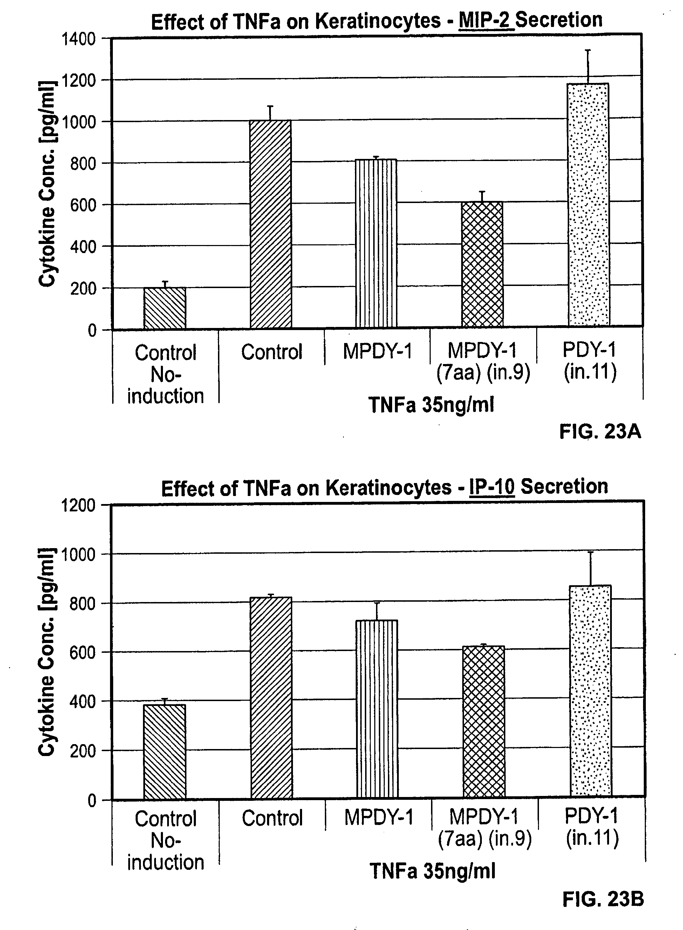

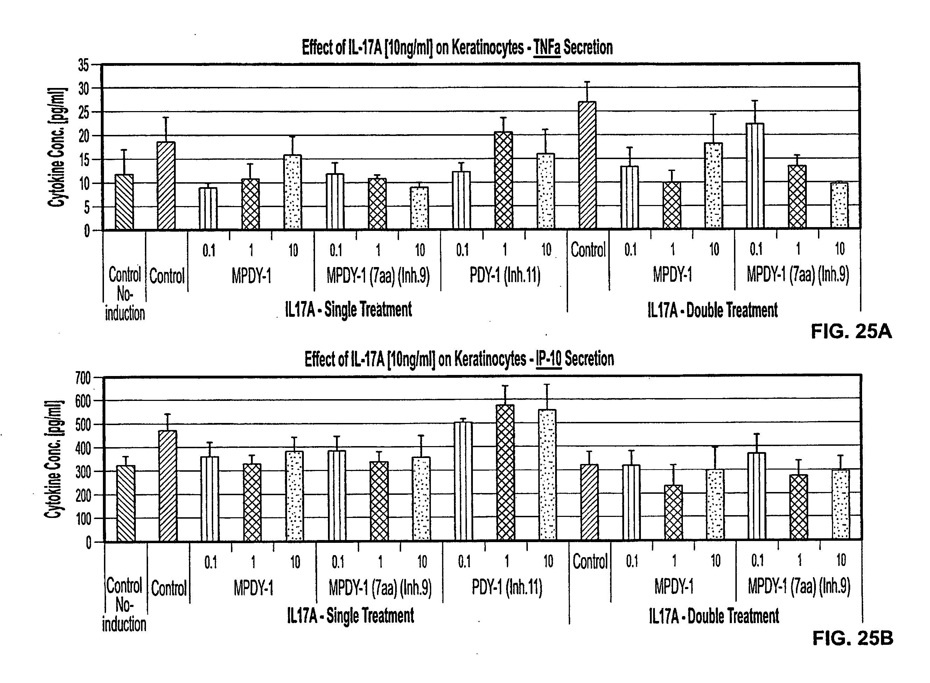

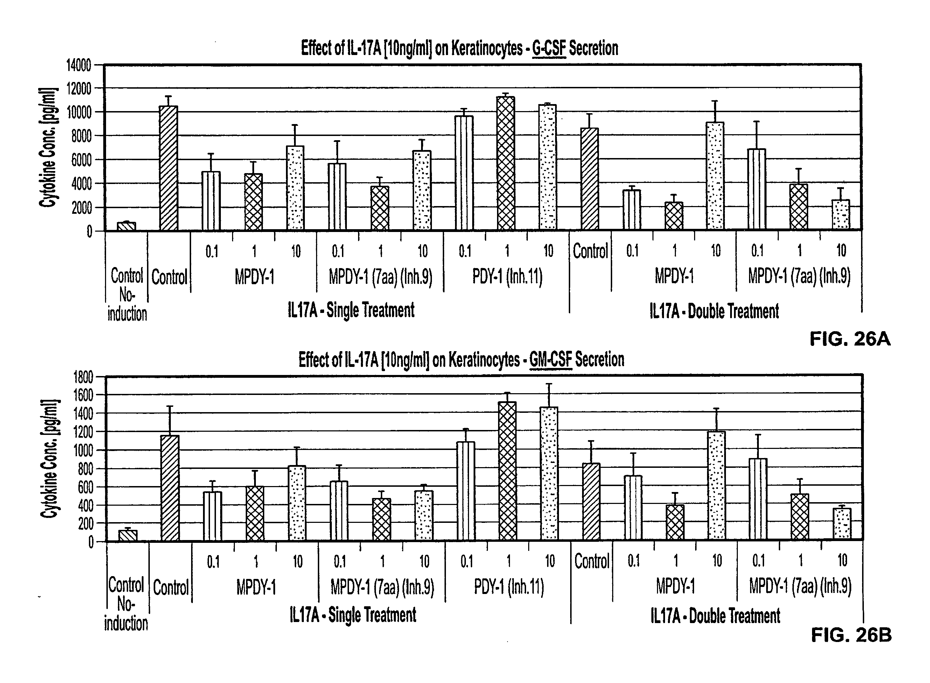

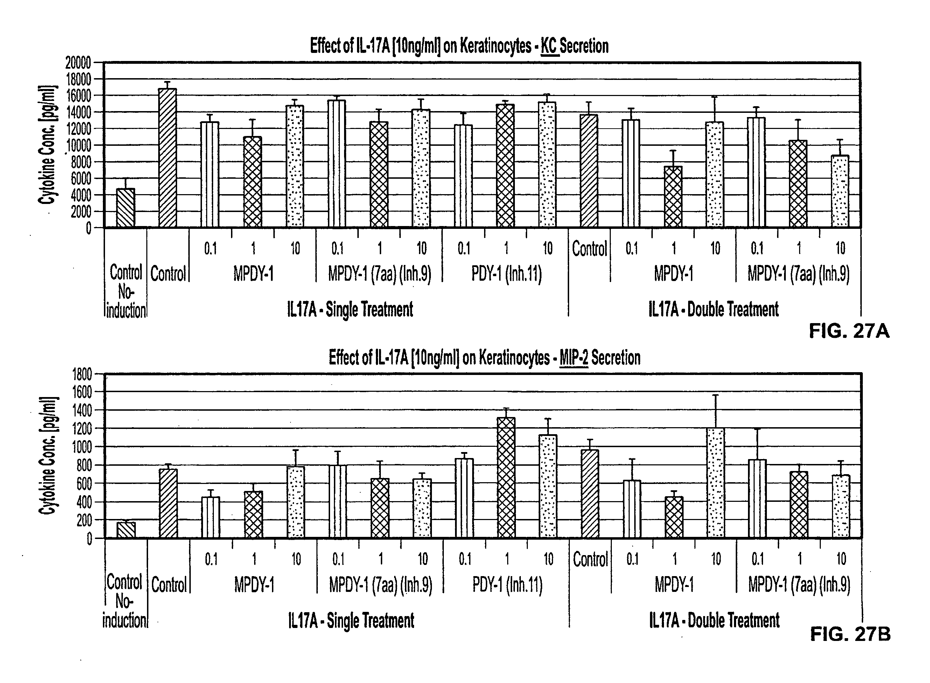

Figure 11, as well as Figures 17-27, 43 and 50 demonstrate the ability of HO/02/10 to decrease dramatically the secretion of major pro-inflammatory cytokines from activated keratinocytes, such as TNFα, IL-1 and IL-6. Specifically, IL-6 was shown to be essential for the development of TH17 cells that are involved in the pathogenesis of psoriasis, with enhancing effect demonstrated for IL-1 and TNFα. TNFα and IL-6 are known targets for psoriasis therapy. Figure 11 demonstrates the effect of 1µg/ml HO/02/10.

HO/02/10 was also shown to inhibit basal keratinocyte and endothelial cell immunological activation *in vivo.* ICAM is an adhesion molecule that allows leukocytes infiltration into inflammatory lesions. Specifically in skin, basal keratinocytes express ICAM-1 upon immunological activation which may enhance infiltration of neutrophils and CD8-T cells into the epidermis, one of the hallmarks of psoriasis. Thus, the effect of HO/02/1 0 on ICAM expression in skin was examined by immunohistochemistry in a wound inflammatory setting *in vivo.*

Down regulation of activated keratinocytes and endothelial cells (ICAM-1 staining) in skin inflammation was observed. A two-cm longitudinal incision was done on the upper back of a C57BL/6J mouse. Following wounding, a sterile pad was sutured to the mouse's skin. Animals were treated daily with HO/02/10 (n=12). Five days post-wounding, when inflammatory phase reaches its peak, the mice were sacrificed, skin tissues were embedded in paraffin and immunohistochemical staining was performed utilizing anti-ICAM-1 antibodies.

As shown in Figure 12, HO/02/10 dramatically reduces ICAM expression on basal keratinocytes and endothelial in blood vessels of the skin. This effect was shown to be dose dependent with maximal effect, demonstrated at 10µg/ml.

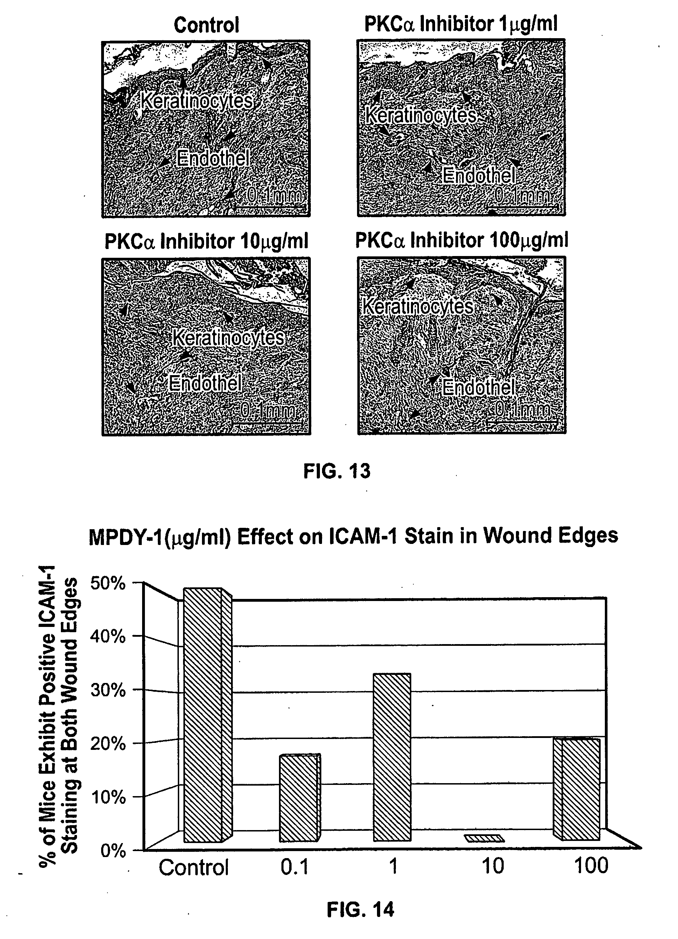

Figure 13 shows additional stains showing down regulation of activated keratinocytes and endothelial cells (ICAM-1 staining) in skin inflammation. As above, a two-cm longitudinal incision was done on the upper back of a C57BL/6J mouse. Following wounding, a sterile pad was sutured to the mouse's skin. Animals were treated daily with MPDY-1 (n=6). Five days post-wounding, when inflammatory phase reaches its peak, the mice were sacrificed, skin tissues were embedded in paraffin and immunohistochemical staining was performed utilizing anti-ICAM-1 antibodies.

Figure 14 is a histogram comparing the percent of mice exhibiting positive ICAM-1 staining at both wound edges.

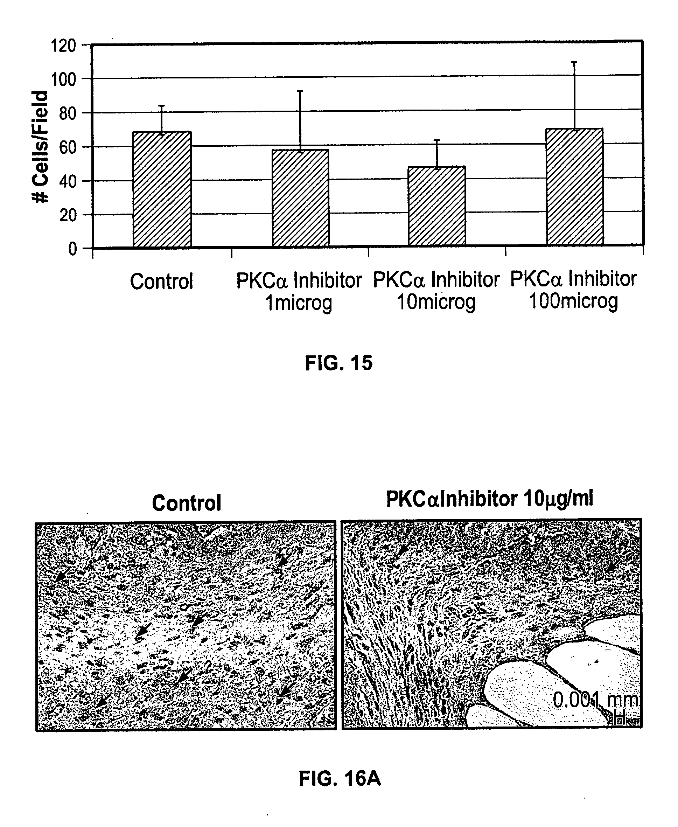

The effect of MPDY-1 on macrophage infiltration was also shown by Iba-1 staining. Iba-1 is a general marker for macrophages. Figure 15 is a histogram showing comparing the number of cells per field exhibiting positive Iba-1 staining. As above, a two-cm longitudinal incision was done on the upper back of a C57BL/6J mouse. Following wounding, a sterile pad was sutured to the mouse's skin. Animals were treated daily with MPDY-1 (n=6). Five days post-wounding, when inflammatory phase reaches its peak, the mice were sacrificed, skin tissues were embedded in paraffin and immunohistochemical staining was performed utilizing anti-Iba-1 antibodies. A dose dependent effect of MPDY-1 on macrophage infiltration was observed.

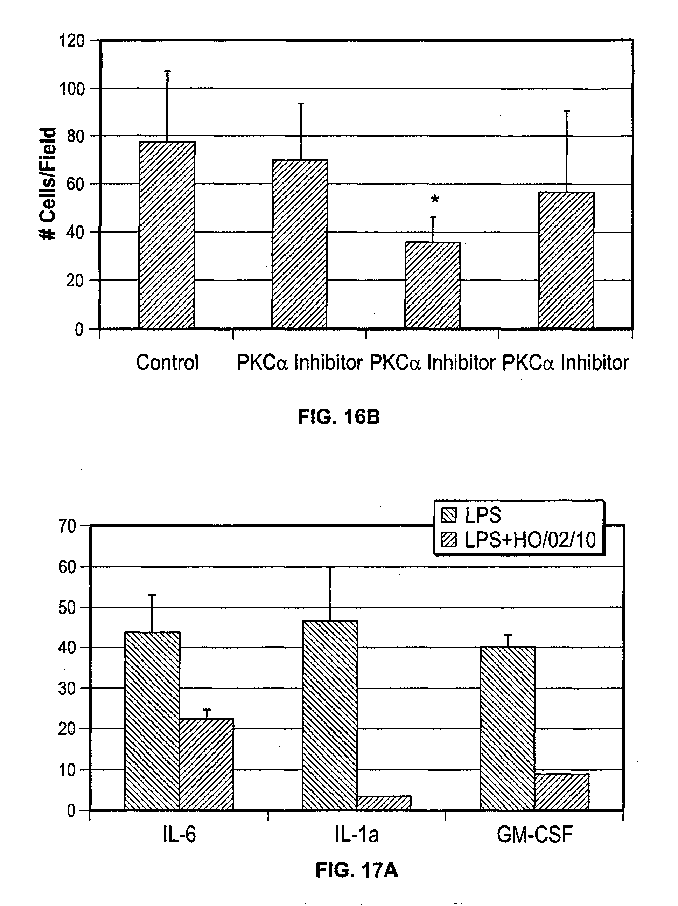

The effect of MPDY-1 on macrophage activation was also shown by MAC-2 staining. MAC-2 is a specific marker for activated macrophages. Figure 16 shows a series of MAC-2 stains and a histogram comparing the number of cells per field exhibiting positive MAC-2 staining. A two-cm longitudinal incision was done as described above. Animals were treated daily with DPBS^{-/-} (Control) or MPDY-1 in the specified concentrations (n=6). After 5 days immunohistochemical staining was performed utilizing anti-MAC-2 antibodies. Bar 1µm. (*p(control Vs. MPDY-1 10µg)=0.0028). Activation of macrophages was significantly inhibited following MPDY-1 treatment.

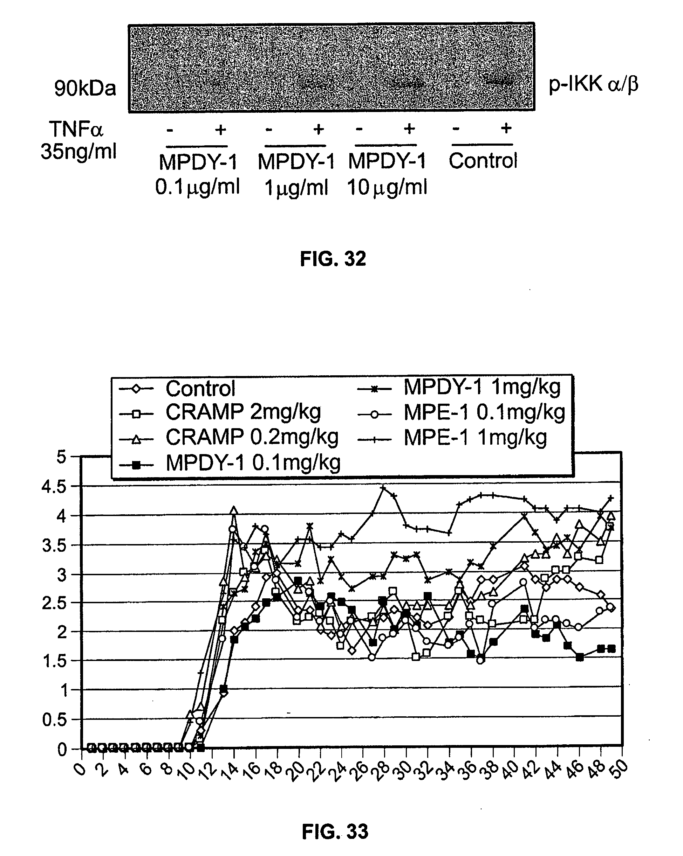

MPDY-1 was also shown to significantly reduce TNFα induced IKK activation in keratinocytes in dose dependant manner as shown in Figure 32. Murine primary keratinocytes were grown for 4 days to full confluence in Low Ca⁺² MEM. Cells were pretreated with designated concentration of MPDY-1 as described in the figure for 1 hour, prior to TNFα induction. Following MPDY-1 pretreatment, cells were incubated with TNFα 35 ng/ml for 15 minutes. Reaction was stopped by adding ice-cold dPBS-/- and keratinocytes were homogenized in RIPA buffer. Samples were subjected to SDS PAGE Western Blot analysis, utilizing phospho-IKKa/b (Ser176/180 antibody). Pretreatment with MPDY-1 significantly reduced TNFα induced IKK activation in keratinocytes in dose dependant manner, where lowest MPDY-1 concentration (0.1mg/ml) exhibited the strongest inhibition thus suppressing NFkB activation.

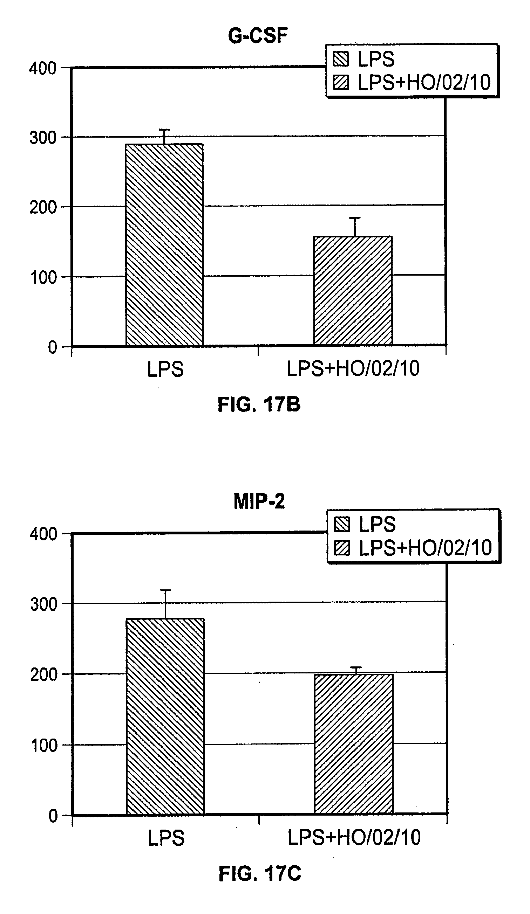

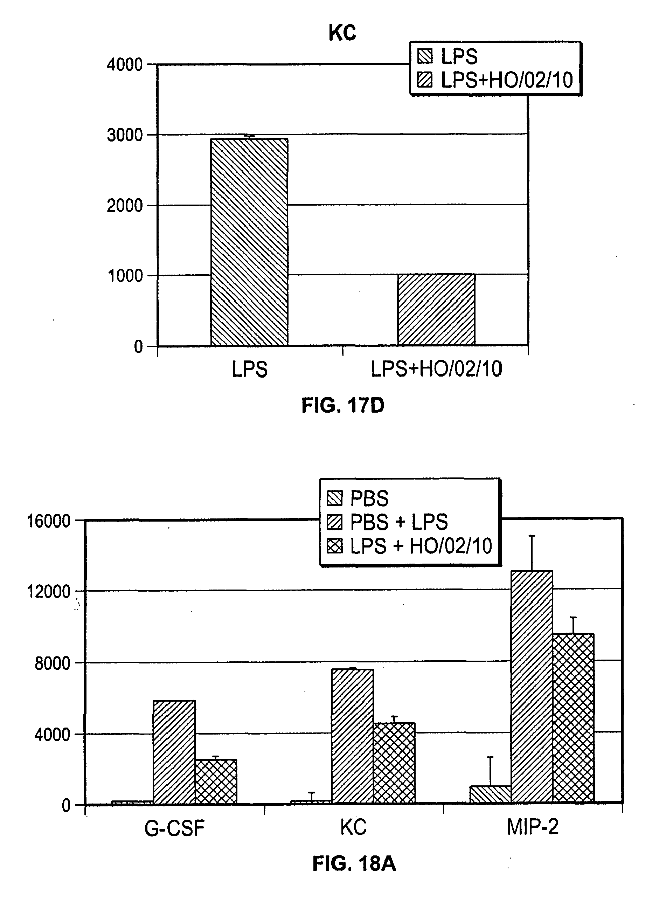

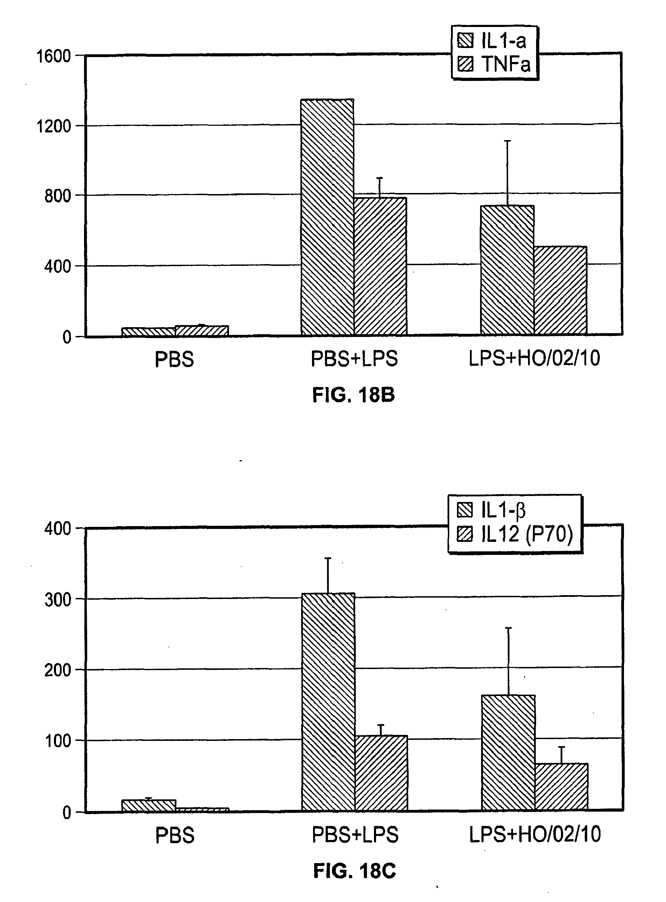

As discussed above, HO/02/10 was also shown to decrease cytokine secretion from activated keratinocytes and macrophages. In recent years it was found that both immune and skin components are equally contributing to the cycle underlying psoriatic pathogenesis. Resident skin cells and immunological cells (both resident and infiltrating cells) interact in the inflammatory psoriatic process by cell-cell interactions and cytokine secretion. Thus, HO/02/10 was examined for its direct effect on the secretion of pro-inflammatory, chemo-attractant and immunological pathway related cytokines form both keratinocytes and immune cells such as macrophages and dendritic cells. The results depicted in Figures 17 and 18 demonstrate that HO/02/1 0 down regulates secretion of immune related cytokines such as IL-6, IL-1α, GM-CSF, MIP-2 and KC from keratinocytes and macrophages.

The results of Figure 17 show the effect of HO/02/10 on cytokine secretion in keratinocytes. Keratinocytes were derived from newborn C57BL/6J mice skin. The cells were incubated for 5 days in 24 wells plates. Cells were then treated with DPBS-/- , LPS (100 ng/ml), or HO/02/10 (1 µg/ml) + LPS (100 ng/ml). Medium containing secreted cytokines was collected after 48 hr and analyzed using a Luminex system.

The results of Figure 18 show that HO/02/10 down regulates cytokine secretion in macrophages. Bone marrow cells were derived from B6 mice. Cells were incubated for 6 days in the presence of GM-CSF (20 ng/ml), and then were treated with DPBS-/-, LPS (100 ng/ml) or HO/02/10 + LPS (1 µg/ml and 100 ng/ml, respectively).

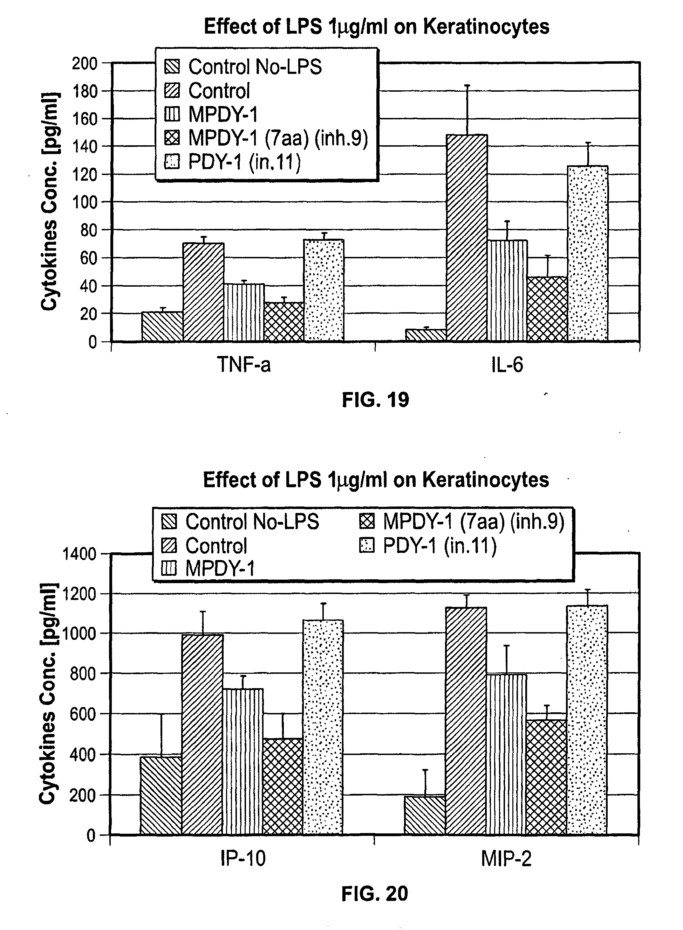

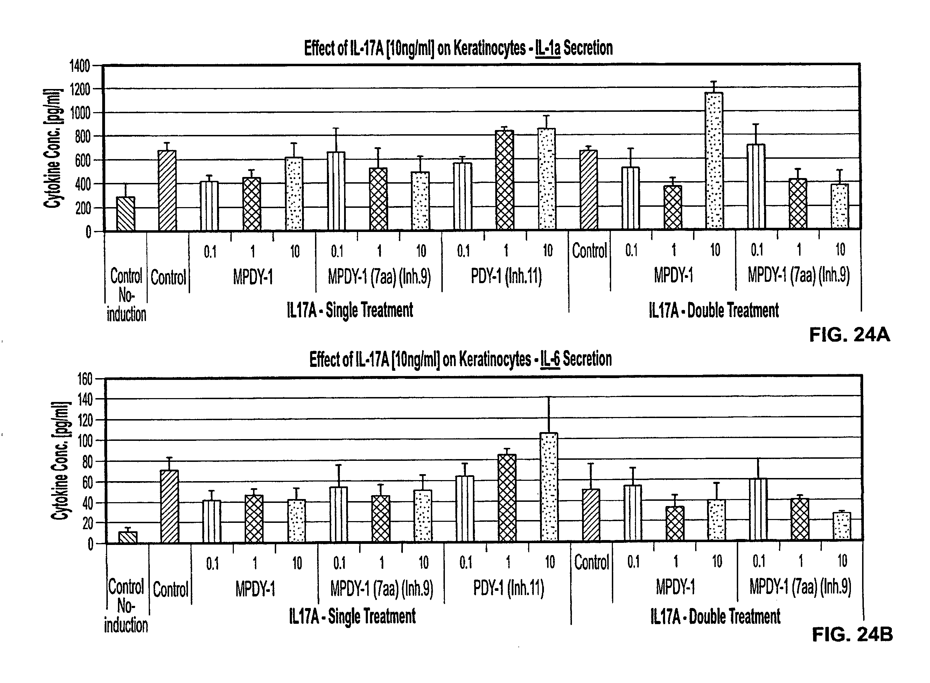

Other peptide PKCα inhibitors were also shown to decrease cytokine secretion from activated keratinocytes and macrophages. Figures 19 to 23 show that the peptide inhibitors MPDY-1 (SEQ ID NO: 6), MPDY-1 sh (SEQ ID NO: 12) and PDY-1 (SEQ ID NO: 13) decrease cytokine secretion from LPS and TNFα activated keratinocytes. Figures 24 to 27 show that the peptide inhibitors MPDY-1 (SEQ ID NO: 6), MPDY-1 sh (SEQ ID NO: 12) and PDY-1 (SEQ ID NO: 13) decrease cytokine secretion from IL-17A activated keratinocytes.

Table 3 summarizes the results according to cytokine roles and origin for HO/02/10.

**Table 3: HO/02/10 Effect On Stimulated Mice Derived-Cells**

| | Proinflammatory (% inhibition) | Chemoattractants (% inhibition) | Systemic (% inhibition) | Th1 (% inhibition) | Th17 (% inhibition) |
|---|---|---|---|---|---|
| Keratinocytes | IL-1 (80%) | KC (65%) | GM-CSF (50%) | | IL-6 (40%) |
| | IL-6 (40%) | MIP-2 (30%) | G-CSF (30%) | | |
| Spleen | IL-1 (50%) | | | | |
| | IL-6 (40%) | | | | |
| | TNFa (50%) | | | | |
| Bone marrow macrophages | IL-1 50% | KC (40%) | G-CSF (40%) | IL-12 (40%) | |
| | TNFa (50%) | MIP-2 (30%) | | TNFα (50%) | |
| Bone marrow DCs | IL-6 (30%) | | | IP-10 (20%) | |

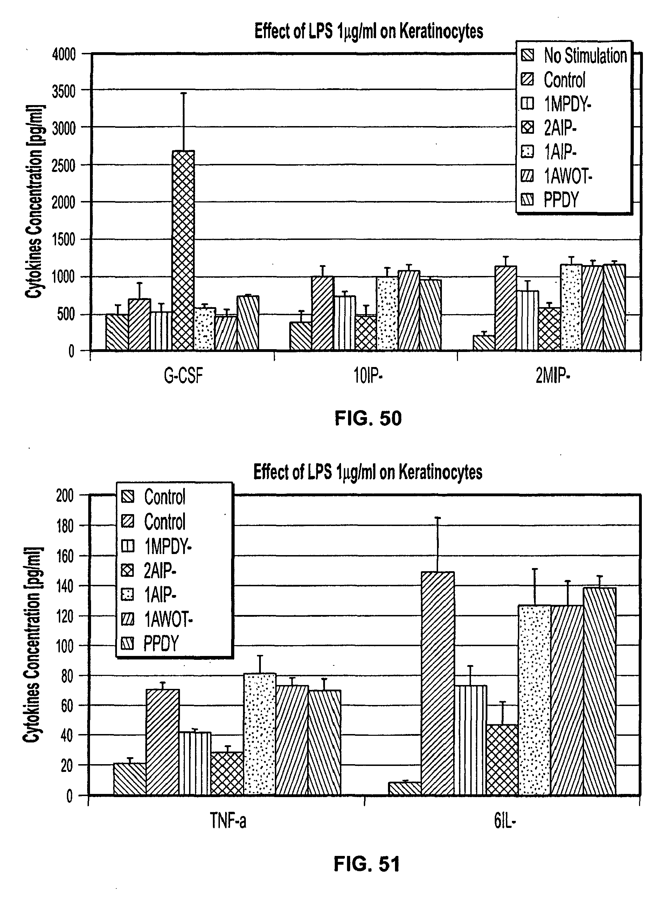

Various other PKCα inhibitors were also shown to decrease cytokine secretion in activated keratinocytes. To determine their effects, keratinocytes were derived from newborn BALB/C mice skin. The cells were incubated for 5 days in 24 wells plate. Cells were then incubated with PBS -/- as control or stimulated by LPS, TNFα, or IL-17. PKCα inhibitors were added as indicated. Medium containing secreted cytokines was collected after 48 hr and analyzed using ELISA. Figure 50 is a tabular summary of cytokine secretion. The PKCα inhibitors MPDY-1 (SEQ ID NO: 6), AIP-2 (SEQ ID NO: 8), AIP-1 (SEQ ID NO: 9), AWOT (SEQ ID NO: 7) and PPDY-1 (SEQ ID NO: 10) were all shown to be effective in decreasing cytokine secretion in keratinocytes.

HO/02/10 was also shown to attenuate T cell infiltration to the skin. The effect of HO/02/10 on T cell infiltration was studied *in vivo* using anti-CD3 specific staining.

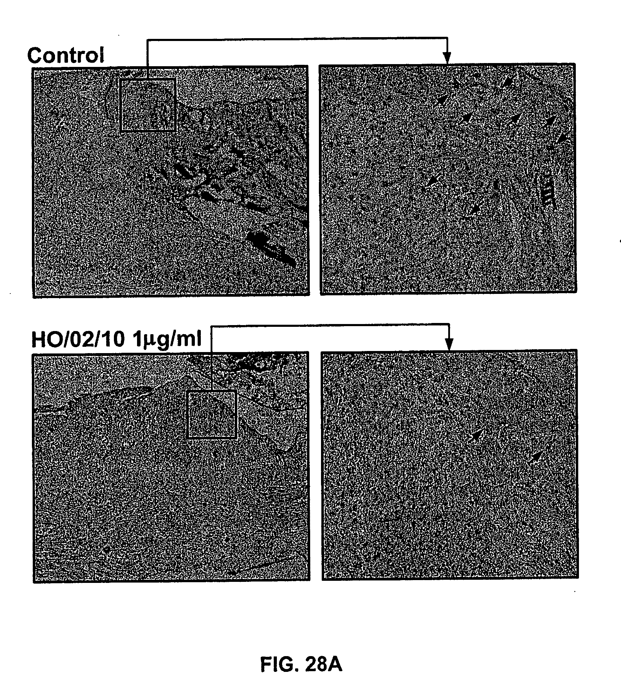

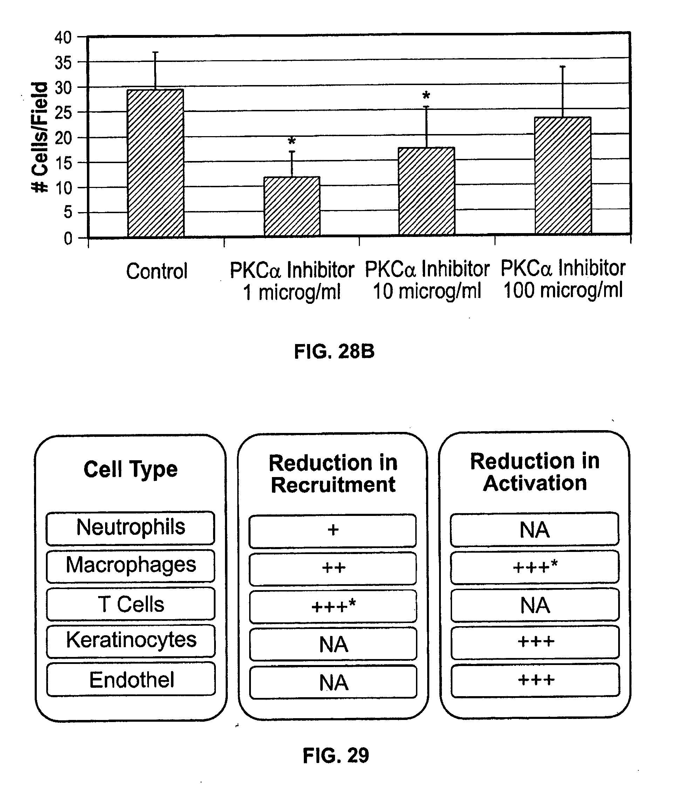

As can be seen in Figure 28, HO/02/10 down regulated T cell infiltration to the dermis and epidermis during the inflammatory stage. Specifically HO/02/10 inhibited T cell infiltration into the epidermis which indicates additional anti-inflammatory properties also characteristic of psoriasis plaques. A two-cm longitudinal incision was done as described above. Animals were treated daily with HO/02/10 (n=12). After nine days immunohistochemical staining was performed utilizing anti-CD3 antibodies. Figure 28B is a histogram comparing the number of cells per field positively stained for CD3. The effect was statistically significant at concentrations of 1 µg/ml and 10 µg/ml, where 1 µg/ml treatment demonstrates stronger effects than 10 µg/ml.

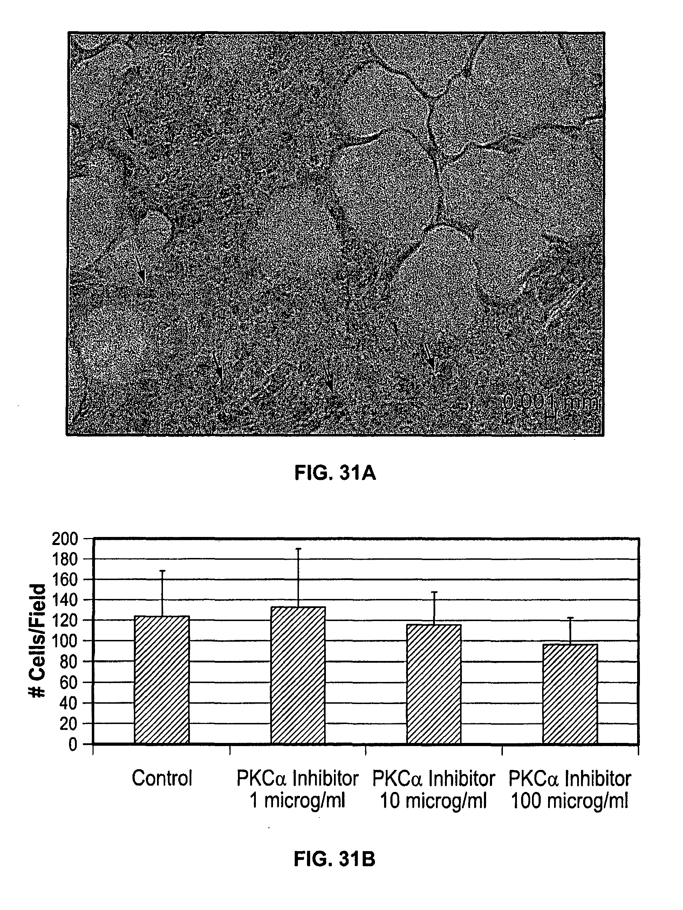

HO/02/10 was also shown to attenuate neutrophil infiltration to the skin (Figure 31). The effect of HO/02/1 0 on neutrophil infiltration was studied *in vivo* using neutrophil specific staining. A two-cm longitudinal incision was done as described above. Animals were treated daily with DPBS-/- (Control) or PKCα inhibitor in the specified concentrations (n=6). After five days the mice were sacrificed, skin tissues were embedded in paraffin and immunohistochemical staining for neutrophils was performed. Although a dose dependent trend was observed, results were not statistically significant.

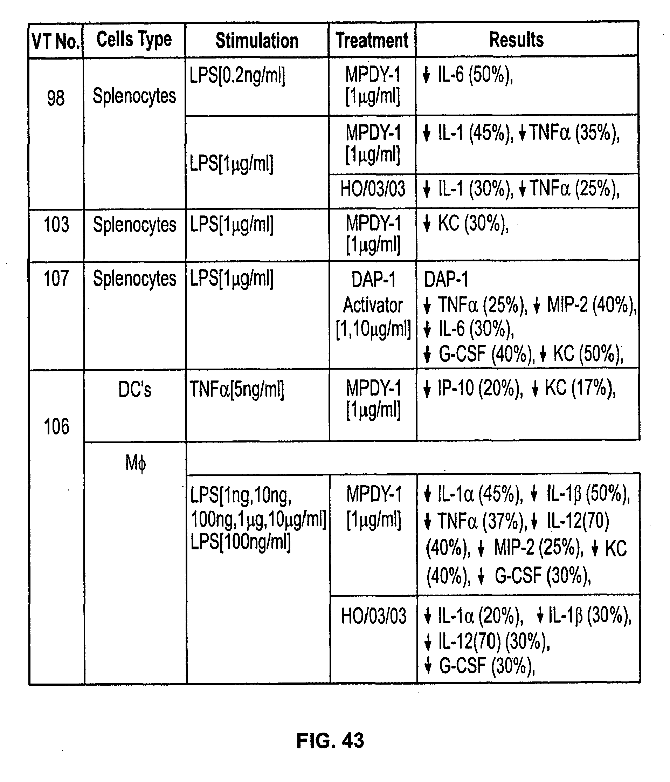

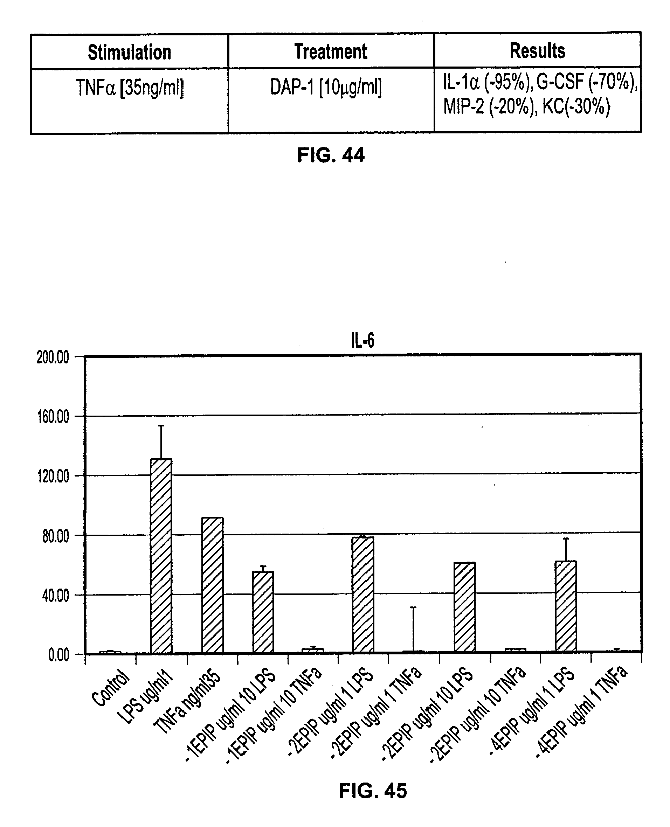

PKCδ activators were also shown to have an anti-inflammatory effect on keratinocytes and splenocytes. Keratinocytes were derived from newborn BALB/C mice skin. The cells were incubated for 5 days in 24 wells plate. Cells were then incubated with PBS -/- as control or stimulated by LPS or TNFα. The PKCδ inhibitor DAP-1 (SEQ ID NO: 34) was added. Medium containing secreted cytokines was collected after 48 hr and analyzed using ELISA. Figure 43 is a tabular summary showing cytokine secretion in splenocytes stimulated with LPS. Figure 44 is a tabular summary of cytokine secretion in keratinocytes stimulated by TNFα. DAP-1 was shown to significantly decease inflammatory cytokine secretion in both keratinocytes and splenocytes.

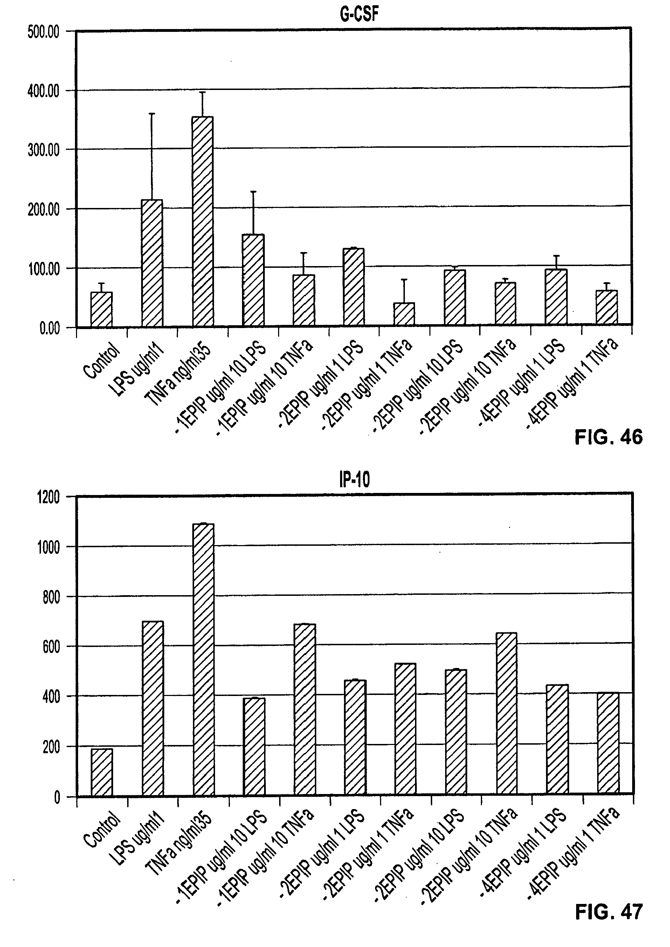

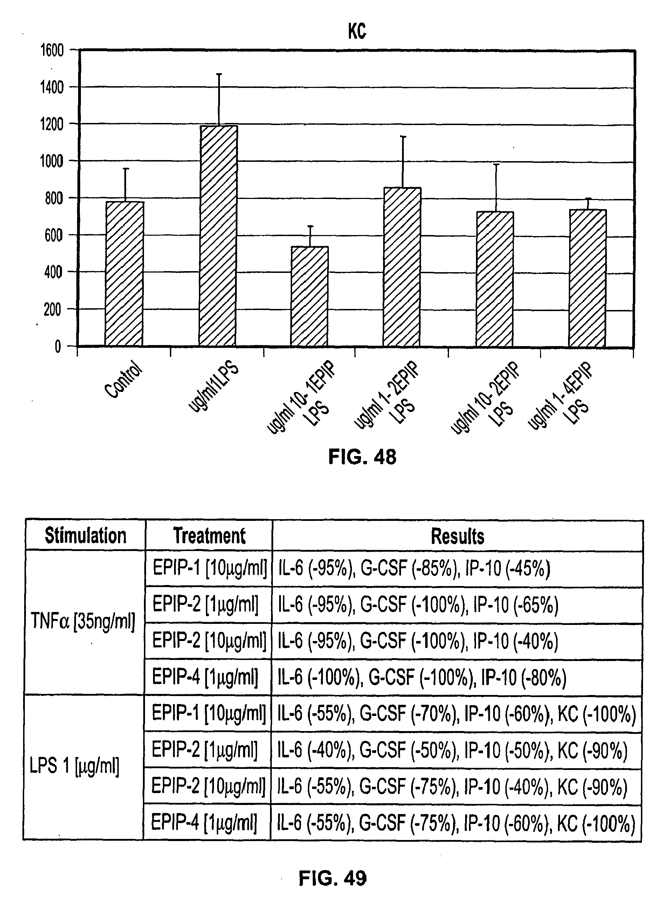

PKCε inhibitors were also shown to have an anti-inflammatory effect on keratinocytes. Keratinocytes were derived from newborn BALB/C mice skin. The cells were incubated for 5 days in 24 wells plate. Cells were then incubated with PBS -/- as control or stimulated by LPS or TNFα. The PKCε inhibitors EPIP-1 (SEQ ID NO: 20), EPIP-2 (SEQ ID NO: 21), or EPIP-4 (SEQ ID NO: 23) were added. Medium containing secreted cytokines was collected after 48 hr and analyzed using ELISA. Figures 45-48 show the results for secretion of specific cytokines while Figure 49 is a tabular summary of cytokine secretion for the various PKCε inhibitors. Several of the PKCε inhibitors were shown to significantly decease inflammatory cytokine secretion in keratinocytes.

In summary, the mechanism of action of PKC isoform inhibitors and activators was determined implicating their use as an effective therapy for inflammation and inflammatory disease. Such peptides were shown to 1) normalize epidermal differentiation markers expression by reducing terminal differentiation; 2) attenuate abnormal hyper-proliferation; 3) regulate skin structure and augment skin strength; and 4) down-regulate inflammation by differentially affecting different cell type recruitment and activation in various steps of the inflammatory process.

Figure 30 shows a schema depicting the overall effect of the PKC isoform inhibitors and activators of the present disclosure on the skin inflammatory and psoriatic related pathway. The scheme summarizes the inhibitory effect of the inhibitors and activators on various cell types and inflammatory stages in the skin. PKC isoform inhibitors and activators inhibit secretion of pro-inflammatory cytokines (such as, IL-1, IL-6 and TNFα) by resident skin immune cells. Accordingly, a decrease in endothelial cells and keratinocytes activation is achieved, resulting a significant reduction in ICAM-1 expression, chemokines secretion and reduce in leukocytes infiltration to the site of inflammation, including neutrophils, macrophages, and T-cells. Cytokines involved in the development and progression of the The and Th17 pathways, both main pathways in psoriasis, were also down regulated.

### EXAMPLE 7

### TREATMENT OF MULTIPLE SCLEROSIS WITH PKCα AND PKCη INHIBITORS

Animal Models of Multiple Sclerosis: As CNS tissue is not easily sampled, to gain information regarding disease mechanisms, a number of models have been developed and are commonly used in the literature. These models reportedly include myelin mutants, chemically induced lesions, viral and autoimmune models that mimic the clinical status and pathology of MS (Baker et al. (2007) ACNR 6(6):10-12). Among the models, experimental allergic encephalomyelitis (EAE) is reportedly the most commonly used model for MS (Baker et al. (2007) ACNR 6(6):10-12).

Autoimmune Model of multiple sclerosis: Experimental allergic encephalomyelitis (EAE) has apparently received the most attention as a model of the MS disease and progression, and is routinely used in testing therapeutic strategies for MS (Baker et al. (2007) ACNR 6(6):10-12).

This disease model exhibits many clinical and histological features of MS and is reportedly caused by the induction of autoimmunity to antigens that are either naturally or artificially expressed in the CNS (Lavi et al. (2005) ISBN 0-387-25517-6; and Owens et al. (2006) Adv Neurol 98:77-89)*.* Following sensitization to myelin antigen, animals appear to develop a disease, typified by limb paralysis. This is associated with blood-brain barrier dysfunction, mononuclear cell infiltration into the CNS and conduction block resulting in impaired neurotransmission.

EAE is polygenic where susceptibility and the clinical course appears to vary depending on the immunizing antigen and the strain/species of animal being investigated. MOG, a minor myelin protein, appears to induce chronic paralytic EAE in C57BL/6 mice. EAE is not a single model, but a number of models that present with varying degrees of pathology in similarity to MS (Lavi et al. (2005) ISBN 0-387-25517-6).

To test the effects of several PKC inhibitors on development and clinical conditions of EAE mice various studies were performed utilizing the following experimental protocols. Specifically, the peptide PKCα inhibitor MPDY-1 (SEQ ID NO: 6) and the peptide PKCη inhibitor MPE-1 (SEQ ID NO: 28) were assessed.

Female 8-10 week C57BL/6J mice were used for the study. The total number of Groups was 7 (n=7 per group); total number of animals was 49. Following anesthesia mice were immunized with MOG35-55/CFA. Mice were immunized subcutaneously (s.c.) in the flank with 200µg MOG35-55/CFA supplemented with 300µg tuberculosis (Mt) H37RA (Difco). Pertussis Toxin (PTX) was injected intravenous (i.v.) at the time of immunization and 48h (hours) later. The need for a boost immunization was determined based on a preliminary calibration experiment (20 mice) with the synthesized MOG35-55 peptide (done as a calibration procedure prior to the treatment experiment).

Treatment was administered as follows. Starting on the day of immunization, mice were treated by i.p (intraperitoneal) injection three times a week (200µl/injection).

Clinical observation and scoring was performed 6 days/week for an observation period of 49 days. Body weight was determined prior to immunization and twice weekly thereafter. Active EAE was scored on a scale of 0-6 depending on quantifiable clinical presentation of mice as shown in Table 4 below.

**Table 4: EAE Scoring**

| Score | Impairment |
|---|---|
| 0 | no impairment |
| 1 | limp tail |
| 2 | limp tail and hind limb paresis |
| 3 | ≥1 hind limb paralysis |
| 4 | full hind limb and hind body paralysis |
| 5 | hind body paralysis and front limb paresis |
| 6 | death |

Groups of mice were treated as shown in Table 5.

**Table 5: Treatment Regime**

| Group | Treatment |
|---|---|
| 1 | DPBS^{-/-} (control) |
| 2 | CRAMP 2 mg/kg (CRAMP) |
| 3 | CRAMP 0.2 mg/kg (CRAMP) |
| 4 | MPDY-1 0.1 mg/kg |
| 5 | MPDY-1 1 mg/kg |
| 6 | MPE-1 0.1 mg/kg |
| 7 | MPE-1 mg/kg |

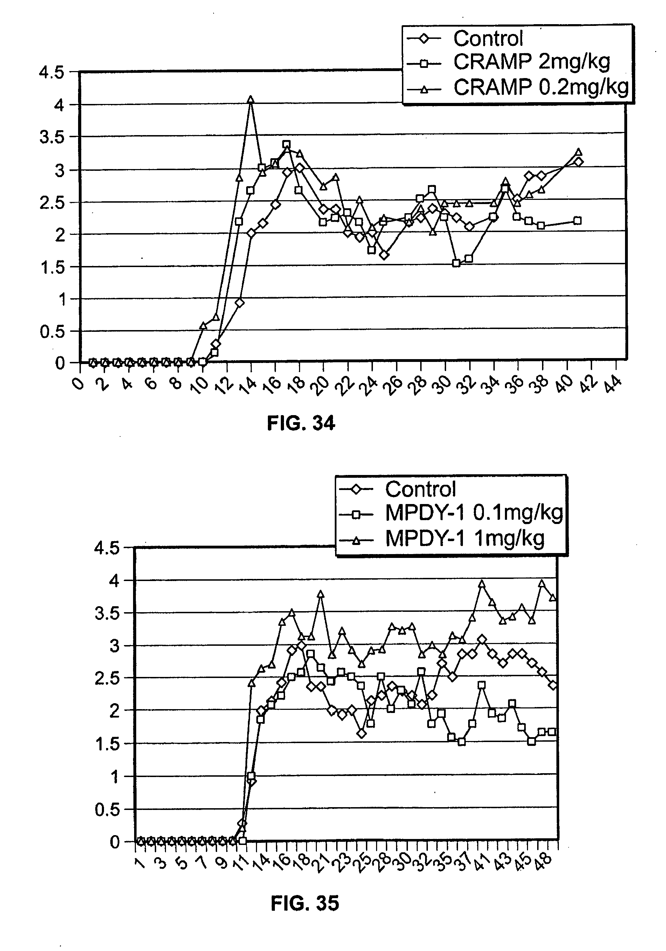

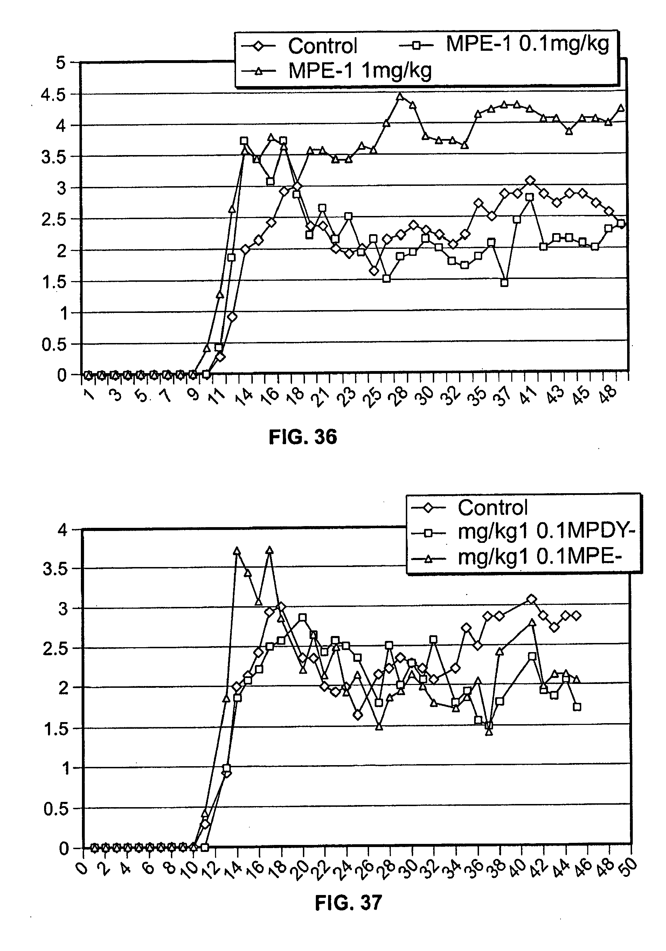

Results are presented and summarized in Figures 33-37.

As can be seen from Figure 33, mice that were treated with MPDY-1 0.1mg/kg started to show illness signs on the 13^{th} day of the experiment, two days later than the control group. The mice in the MPDY-1 0.1 mg/kg group showed a lower score during most of the experiment days compared to the mice in the control group. Moreover, no mouse in the MPDY-1 0.1 mg/kg group and in the MPE-1 0.1 mg/kg group died during the experiment (score 6), while, in all the other groups mice died over the course of time (control group - the first mice died on the 34^{th} day of the experiment and another one on the 35^{th} day) A summary of all mice deaths is provided in Table 6 below.

**Table 6: Summary of Mice Deaths During Treatment**

| Group | Number of dead mice during 49 days | Details (on day) |
|---|---|---|
| 1 | 2 | 34,35 |
| 2 | 2 | 23,44 |
| 3 | 2 | 27,38 |
| 4 | 0 | |
| 5 | 2 | 16,38 |
| 6 | 0 | |
| 7 | 4 | 20,33,37,35 |

A summary of scores derived from Figure 33-37 at specific time points is shown in Table 7.

**Table 7: Summary of Scores at Specific Time Points**

| Group | The initial day | Score on day 18 | Score on day 29 | Score on day 37 |
|---|---|---|---|---|
| Control | 11 (0.285) | 3.000 | 2.357 | 2.857 |
| CRAMP 0.2mg/kg | 10 (0.571) | 3.214 | 2.000 | 2.571 |
| CRAMP 2mg/kg | 11 (0.143) | 2.643 | 2.643 | 2.143 |
| MPDY-10.1mg/kg | 13 (1.000) | 2.571 | 2.000 | 1.500 |
| MPDY-1 1mg/kg | 11 (0.214) | 3.143 | 3.286 | 3.071 |
| MPE-1 0.1 mg/kg | 11 (0.429) | 2.857 | 1.929 | 1.429 |
| MPE-1 1mg/kg | 10 (0.429) | 3.071 | 4.286 | 4.286 |

Groups 4 and 6 of the treatments show resistance to development of the clinical symptoms of the disease, including non-mortality until the 49th day of the experiment. MPDY-1 and MPE treatment at a concentration of 0.1µg/ml (group 4) reduced EAE severity and protected mice from fatal EAE observed at a late stage of the disease in control animals, thus resulting in reducing of the average group score (MPDY-1 of 1.93; MPE-1 of 2.00) to below the average of the control group score (2.86) at the end of the experiment (day 42). MPDY-1 treatment at the concentration of 0.1 µg/ml induced two days delay of the clinical conditions of development of the EAE comparison as compared to control group. Therefore, MPDY-1 and MPE appeared to be shown as effective agents for the treatment of MS.

### EXAMPLE 8

### IN VIVO ASSESSMENT OF PRURITUS TREATMENT

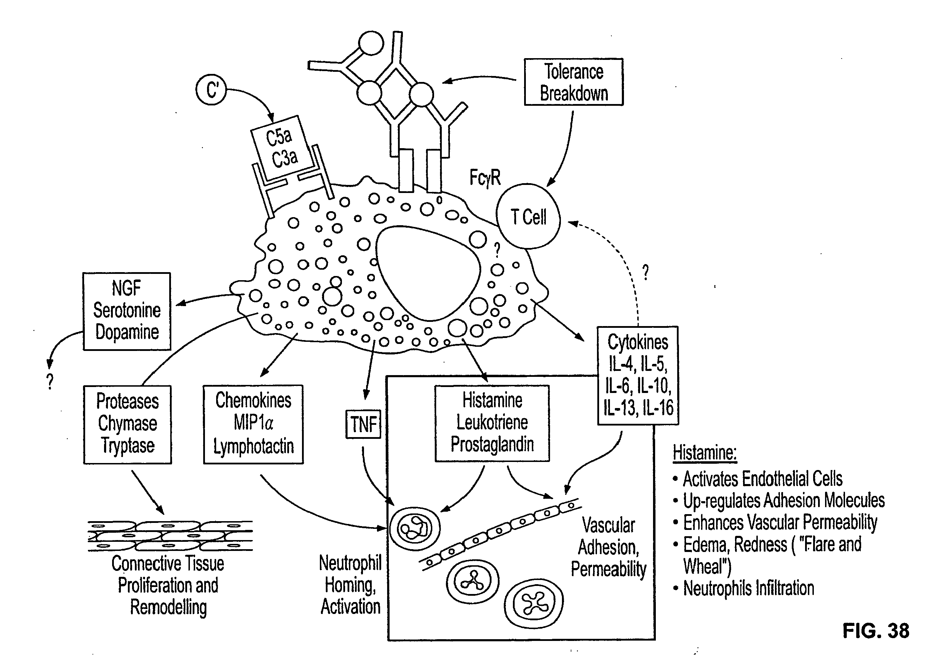

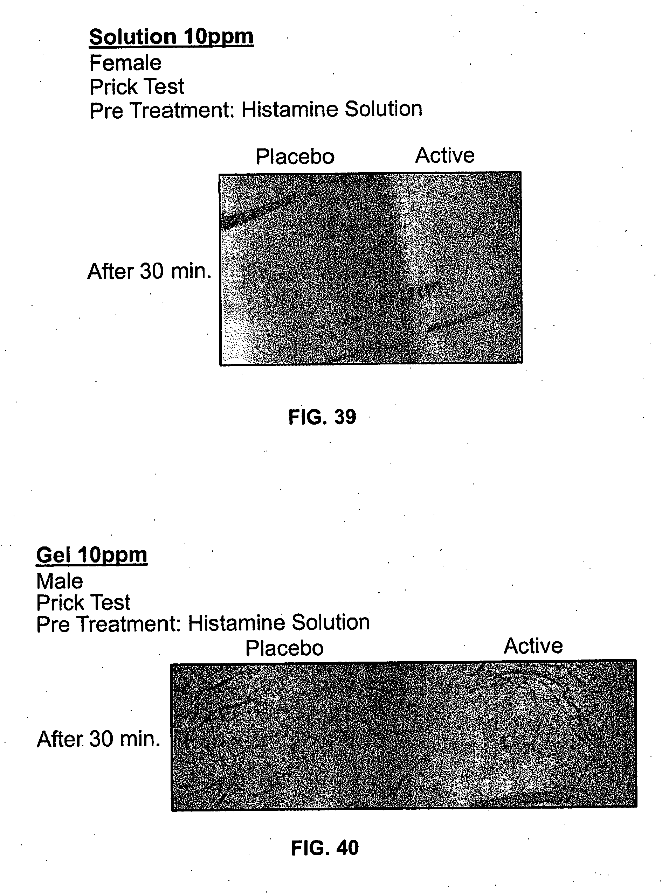

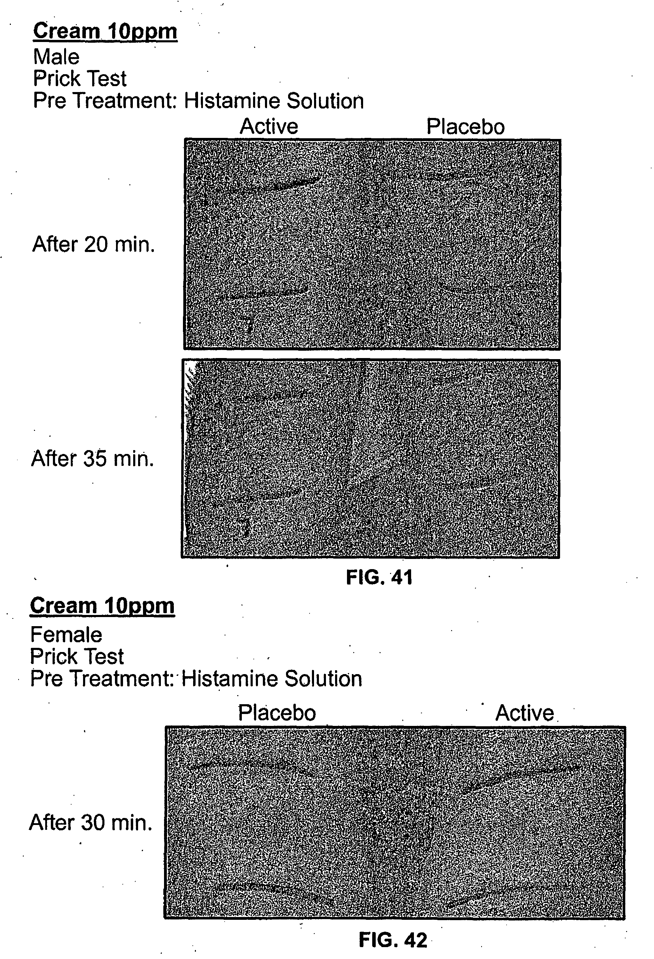

A prick test model utilizing histamine to assess pruritus was developed as shown in Figure 38. Forearms of individual subjects were injected with histamine solution and placebo. The formulation of Example 4 was topically applied with MPDY-1 at various concentrations and pruritus was assessed over a time course as shown in Figures 39-42.

This test was performed by placing a drop of a solution containing a possible allergen on the skin, and a series of scratches or needle pricks allow the solution to enter the skin. The extract enters into the outer layer of the skin (epidermis) using a fine needle (such as a. 26G disposable needle). This testing is not painful, and generally there is no bleeding involved since the needle only scratches the surface of the skin. If the skin develops a red, raised itchy area (called a wheal), it is as a result of allergic reaction to that allergen. This is called a positive reaction. A drop of extract is introduced through a fine needle (such as a No. 26 disposable needle). The test causes no discomfort and minimal trauma so that controls and negative test show only the site of the prick; if anything.

26G needles were utilized to introduce histamine stock (Histatrol Positive Control Histamine, 1mg/ml, code #HIST14999V, Trupharm). The test was conducted on healthy volunteers in a double-blind, randomized test. The formulations were applied on the forearms. Three treatment areas were chosen and marked (the surface of the forearm from the elbow to the wrist was divided transversely into proximal, middle and distal thirds); the areas were pricked prior the below treatments.

An area was treated with the active formulation in a double-blind manner, for 10 minutes- marked as A.

An area was treated with the Placebo in a double-blind manner, for 10 minutes-marked as C.

A color photograph was taken at time zero (T₀), after 10, 20, 30 minutes.

Pruritus questionnaire was answered by the subjects 5 and 15 minutes after treatment.

In one study, three subjects were tested with treatment as shown in Tables 8 and 9.

**Table 8: Prick Test Sample Group**

| | **Left forearm** | **Right forearm** |
|---|---|---|
| Subject 1 | A5 | C2 |
| Subject 1 | A4 | C1 |
| Subject 2 | A2 | C3 |
| Subject 2 | A5 | C2 |
| Subj ect 3 | A4 | C1 |
| Subject 3 | A1 | C3 |

**Table 9: Prick Test Treatments**

| **Treatment** | **Group** |
|---|---|
| MPDY-1 1µg/ml | A1 |
| MPDY-1 10µg/ml | A2 |
| Cream 10ppm | A4 |
| Gel 10ppm | A5 |
| Cream W/O active material | C1 |
| Gel W/O Active material | C2 |
| DPBS | C3 |

Subjects were provided with pruritus sensation forms and asked to indicate the level of pruritus sensed from 0 (no response) to 4 (uncontrollable pruritus) at different time intervals. Results are shown in Table 10 below.

**Table 10: Results**

| **5 minutes after histamine** | |
|---|---|
| Pruritus score | Subject & Site |
| **2** | 1 - A5 |
| **1** | 1 - A4 |
| **2** | 2 - A2 |
| **1** | 2 - A5 |
| **1** | 3 - A4 |
| **3** | 3 - A1 |
| **3** | 1 - C2 |
| **4** | 1 - C1 |
| **4** | 2 - C3 |
| **3** | 2 - C2 |
| **3** | 3 - C1 |
| **4** | 3 - C3 |

| **15 minutes after histamine** | |
|---|---|
| **0** | 1 - A5 |
| **0** | 1 - A4 |
| **0** | 2 - A2 |
| **0** | 2 - A5 |
| **0** | 3 - A4 |
| **1** | 3 - A1 |
| **4** | 1 - C2 |
| **4** | 1 - C1 |
| **4** | 2 - C3 |
| **4** | 2 - C2 |
| **3** | 3 - C1 |
| **4** | 3 - C3 |

Additionally, as is evident in Figures 39-42, application of MPDY-1 significantly attenuated redness, inflammation and pruritus as compared with control over the time course.

Although the objects of the disclosure have been described with reference to the above example, it will be understood that modifications and variations are encompassed within the spirit and scope of the disclosure. Accordingly, the disclosure is limited only by the following claims.

**EXAMPLE 9**

Combining insulin and PKCα inhibitor circumvent adverse side effects caused by insulin only treatment

Wounds were performed on the back of 8-10 week old C57BL mice by incision and were treated daily for 7 days with either vehicle (PBS) control or with 1 µM insulin (Humulin, Eli, Lilly, USA) or a mixture of 1 µM insulin combined with 1 µM of the PKCα inhibitor of SEQ ID NO: 6. Seven days after wounding all mice were sacrificed and treated wounds were histologically analyzed for proliferative capacity of the epidermis (proliferating cell nuclear antigen- PCNA), angiogenesis, inflammation, epidermal cells and the remodeling processes at the wound gap.

As shown in **Table 11,** the insulin-only treatment caused a substantial increase in the incidence of abnormal angiogenesis in the wound area, as compared with the PBS control (60% and 25%, respectively). Since the wound healing process involves rapidly proliferating epidermal cells, such increased angiogenesis may also increase the risk of impaired wound closure by delaying formation of normal granulation tissue. On the other hand, when insulin was combined with the PKCα inhibitor of SEQ ID NO: 6 no abnormal angiogenesis was observed in the treated wound area.

**Table 11: The effect of insulin only and insulin combined with PKCα inhibitor on the severity of angiogenesis at the wound area**

| **Treatment** | **Proliferative capacity of the epidermis (layers of basal cells, PCNA)** | **Angiogenesis** |
|---|---|---|
| PBS (control) N=4 | 5 | high abnormal |
| | 7 | normal |
| | 8 | normal |
| | 6 | normal |
| | average 6.5 | |
| Insulin only N=5 | 8 | high abnormal |
| | 8 | normal |
| | 6 | normal |
| | 5 | high abnormal |
| | 5 | high abnormal |
| | average 6.4 | |
| Insulin+PKCα inhibitor N=5 | 6 | normal |
| | 6 | normal |
| | 4 | normal |
| | 2 | normal |
| | 3 | normal |
| | average 4.2 | |

In addition, the insulin-only treatment resulted in increased inflammation, hyperplasia of epidermal cells, delayed differentiation of the spinous layer of epidermal cells and increased scarring. None of the adverse side effects, which resulted from the insulin-only treatment, were observed when the PKCα inhibitor was combined with insulin.

**Example 10** PKCα inhibitor reduces wounds inflammation

Late and severe inflammatory response in wounds may suppress the process of healing, thus preventing such inflammation from development may promote the wound healing process. Accordingly, the effect of PKCα inhibitor HO/02 and insulin on wound inflammation was tested in the following experiment.

Wounds were performed on the back of C57BL mice by incision and were treated daily for 7 days with: (i) PBS, control; (ii) 1 µM of the PKCα inhibitor of SEQ ID NO: 6; (iii) 1 µM insulin (Humulin, Eli Lilly, USA); or (iv) a mixture of 1 µM PKCα inhibitor and 1 µM insulin. Seven days after wounding all mice were sacrificed and the treated wounds were observed for inflammation under a microscope. The resulting incidences of severe inflammation observed in the wound area are summarized in **Table 12.**

As shown in **Table 12,** administering the PKCα inhibitor to wounds caused a substantial (33.3%) decrease of severe wound inflammation incidence, as compared to control. Insulin alone had no anti-inflammatory effect under the experimental conditions.

**Table 12**

| **Treatment** | **Incidence of severe inflammation in wound (%)** |
|---|---|
| PBS control | **60** |
| PKCα inhibitor | **40** |
| Insulin | **56** |
| PKCα inhibitor + insulin | **50** |

These results indicate that a PKCα inhibitor can be used in therapy to control severe inflammation of wounds. The demonstrated capacity of the PKCα inhibitor of SEQ ID NO:6 to reduce inflammation, coupled with its capacity to promote epidermal closure, dermal closure and spatial differentiation of epidermal cells, makes it a potentially most effective therapeutic agent for wound healing.

**Example 11.** Efficacy of insulin in combination with the PKCa inhibitor HO/02 in wound healing in STZ-induced diabetic mice

Full thickness skin incisions (20 mm) were performed on the upper back of anesthetized streptozocin (STZ)-injected (175 mg/kg body weight) diabetic, citrate buffer injected (6 mice/group) non-diabetic, and non-injected (6 mice/group), C57BL/6J mice. Periodical blood glucose monitoring was performed by measuring glucose levels from tail vein after STZ injection. Only mice exhibiting blood glucose levels higher than 450 mg/dl were taken into the study. Following incision, STZ-induced diabetic wounds were treated daily by application of PBS (7/6 mice/group), insulin (0.1 units/ml) (6 mice/group), the PKCa inhibitor of SEQ ID NO:6 (1 µg/ml) (7/5 mice/group) or a formulation (herein designated HO/03/03) containing insulin and the N-myristoylated PKCα inhibitor of SEQ ID NO: 6 (7/6 mice/group) directly to the surgical dressing. Biopsies were collected 9 days post-wounding. Wounds were excised from euthanized animals for evaluation of wound-healing parameters by histology and immunohistochemistry.

Wounds were assessed for epidermal closure by Keratin 14 staining. Wounds were considered closed when complete epidermal staining was observed across the wound gap. Dermal contraction was considered when both dermal edges were visible in a fixed field x100 magnification utilizing H&E staining. Epidermal differentiation was assessed by Keratin 1 staining. Wounds that displayed positive staining across the entire wound gap were considered as differentiated (Kl positive).

As shown in Table 13, STZ-induced diabetic animals were wound-healing impaired. Comparison of important wound healing parameters in diabetic treated, untreated and non-diabetic mice revealed that the group treated with the formulation HO/03/03 exhibited a synergistic healing effect. The synergy was evident in all critical healing stages including epidermal closure (71% vs. 17% in the diabetic control p<0.05), epidermal differentiation (28% vs. 0%) and dermal contraction (33% vs. 0%).

The organization of hypodermis was assessed by the presence (or absence) of hypodermis at both wound edges. Granulation tissue formation was assessed by the presence of fibroblasts and collagen fibers in the wound bed. The wound was considered positive for granulation tissue formation when a continuous layer of granulation tissue was present in the wound gap. In addition, we have defined 3 specific histological parameters characterizing severe inflammation of diabetic wounds: (i) abscess formation at the wounded area, (ii) excessive leukocytosis (>100 cells in a fixed field (x200)) and (iii) high white blood cells (WBC)/red blood cells (RBC) ratio within the blood vessels shown in a fixed field (x200). When at least 2 of these parameters are present at the wound gap, the wound is considered severely inflamed.

**Table 13: Histological analysis of wound healing parameters 9-days post wounding**

| | | | |
|---|---|---|---|
| | | ***Dermal closure (H&E)*** | ***Epidermal differentiation (Kl)*** |
| | ***Epidermal closure (K14)*** | | |
| ***Treatment group*** | | | |
| | | | |
| ***Diabetic+PBS*** | **17%** | ***0%*** | ***0%*** |
| | | | |
| ***Diabetic+Insulin 0.1 units*** | **17%** | ***0%*** | ***0%*** |
| | | | |
| ***Diabetic+HO*/*02 1 ug*/*ml*** | **17%** | **17%** | **17%** |
| | | | |
| ***Diabetic*+*HO*/*03*/*03*** | **71%** | **28%** | **33%** |
| | | | |
| ***Non-diabetic*** | | | |
| | | | |
| ***Buffer Citrate Injected+PBS*** | ***100%*** | **17%** | ***100%*** |

* Quantitative analyses of healing parameters were made as described above.

Results are presented as percent of wounds in each group.

**Table 14: Histological analysis of wound healing parameters 9-days post wounding**

| | | | |
|---|---|---|---|
| | | ***Severe inflammation*** | ***Granulation tissue*** |
| ***Treatment group*** | ***Hypodermis*** | | |
| | | | |
| ***Diabetic+PBS*** | **25%** | **67%** | ***42%*** |
| | | | |
| ***Diabetic+Insulin 0.1 units*** | ***10%*** | ***100%*** | ***40%*** |
| | | | |
| ***Diabetic+SEQ** ID* ***NO:6 1 ug*/*ml*** | ***10%*** | ***30%*** | ***50%*** |
| | | | |
| ***Diabetic+HO*/*03*/*03*** | ***43%*** | ***28%*** | ***86%*** |
| | | | |
| ***Non-diabetic*** | | | |
| | | | |
| ***Buffer Citrate Injected+PBS*** | ***100%*** | **17%** | **92%** |

* Quantitative analyses of healing parameters were made as described above.

Results are presented as percent of wounds in each group.

As shown in **Table 14,** diabetic animals exhibited impairment in other wound healing parameters such as the organization of hypodermis at wound edges, inflammation and granulation tissue formation. Diabetic associated impaired healing parameters were corrected by treatment with the formulation HO/03/03 as demonstrated by organization of the hypodermis at the wound gap edges (43% vs. 25%) and granulation tissue formation (86% vs. 42%). Treatment with HO/03/03 reduced inflammatory response in the wound gap (28% vs. 67%). Healing efficacy of the formulation agents alone (insulin or peptide of SEQ ID NO:6 exhibited only partial healing effects.

The results summarized above demonstrate that the formulation HO/03/03 exhibits a synergistic effect in overcoming diabetes related healing impairments in multiple healing parameters.

**Example 12.** Efficacy of insulin in combination with the PKCa inhibitor HO/02 in wound healing in vivo in the pig skin model

Several wound healing studies were conducted in the pig model system for further understanding of the wound healing process as well as the healing effects of the formulation HO/03/03.

Full thickness 35-40 mm skin incisions were performed on the back of an anesthetized female pig (5 months old, 60-70 kg). Ten symmetrical incisions were performed on both sides of the back area at the same distance from the back bone (20 wounds total). The wounds were treated daily, twice a day, by application of 1 ml PBS (10 per group) or the formulation (herein designated HO/03/03) containing insulin 0.1 units and the N-myristoylated PKCα inhibitor 1 µg/ml of SEQ ID NO:6 (10 per group) directly to the wound area. Wounds were excised from sacrificed animals on 7 and 22 days post-wounding and morphological, histological and immunohistochemical assessments were performed.

As shown in **Table 15,** treatment with the formulation HO/03/03 accelerates wound healing by affecting epidermal migration and granulation tissue formation at the early stages of healing. Moreover, these wounds are large and prone to infections from environmental pathogens (animals are not kept in sterile conditions), yet HO/03/03 exhibits attenuation of the inflammatory response at the wound gap thus promoting healing progression.

**Table 15: Histological analysis of efficacy of the formulation HO/03/03 in pig 7-days post wounding**

| | | | |
|---|---|---|---|
| | ***Epidermal migration*** | ***Severe inflammation*** | ***Granulation tissue formation*** |
| ***Treatment group*** | | | |
| | | | |
| ***PBS*** | ***30%*** | ***60%*** | ***50%*** |
| | | | |
| ***HO*/*03*/*03*** | ***80%*** | ***30%*** | ***85%*** |

* Results are presented as percent of wounds in each group

**Table 16: Histological analysis of efficacy of the formulation HO/03/03 in pig 22-days post wounding**

| | | | |
|---|---|---|---|
| | | | |

| | ***Epidermal closure*** | ***Epidermal Differentiation*** | ***Dermal Contraction*** |
|---|---|---|---|
| ***Treatment group*** | | | |
| | | | |
| ***PBS*** | ***88%*** | **67%** | ***25%*** |
| | | | |
| ***HO*/*03*/*03*** | ***100%*** | ***90%*** | ***70%*** |

* Results are presented as percent of wounds in each group

When testing advanced healing stages, HO/03/03-treated wounds further exhibit accelerated healing. As shown in Table 16, no difference was noted in the epidermal closure, though dermal contraction and epidermis differentiation was significantly higher in treated wounds. It is important to emphasize that these wounds were performed on the same animal thus contributing to the marked healing effects of the treatment.

From the results summarized above it is readily understood that the formulation HO/03/03 promotes accelerated healing in the pig skin model affecting healing parameters in early as well as late wound healing stages.

## Claims

1. An inhibitor of PKC for use in treating an inflammatory disease or disorder in a subject, thereby treating the inflammatory disease or disorder in the subject, preferably wherein the inhibitor is a polypeptide, preferably wherein the polypeptide comprises an N-terminal modification, C-terminal modification, or combination thereof, more preferably wherein the polypeptide is N-acylated, wherein the polypeptide is N-myristoylated or N-palmitoylated.

2. The use of claim 1, wherein the polypeptide is use parentally, subcutaneously or intravenously, wherein the polypeptide is administered topically, orally, mucosally, rectally, pulmonarily, nasally, or otically.

3. The use of claim 2, wherein the polypeptide is use at a dose of about 0.1 to about 10000 micrograms per kilogram, preferably wherein the polypeptide is use at a dose of about 0.1 to about 1000 micrograms per kilogram, more preferably wherein the polypeptide is use at a dose of about 1.0 to about 50 micrograms per kilogram.

4. The use of claim 2, wherein the polypeptide is used daily, weekly, biweekly or monthly.

5. The use of claim 1, wherein the inflammatory disease or disorder is selected from the group consisting of psoriasis, multiple sclerosis, rheumatoid arthritis, osteoarthritis, systemic lupus erythematosus, Hashimoto's thyroidis, myasthenia gravis, diabetes type I or II, asthma, inflammatory lung injury, inflammatory liver injury, inflammatory glomerular injury, atopic dermatitis, allergic contact dermatitis, irritant contact dermatitis, seborrhoeic dermatitis, Sjoegren's syndrome, keratoconjunctivitis, uveitis, inflammatory bowel disease, Crohn's disease, ulcerative colitis, an inflammatory disease of the joints, skin, or muscle, acute or chronic idiopathic inflammatory arthritis, myositis, a demyelinating disease, chronic obstructive pulmonary disease, interstitial lung disease, interstitial nephritis and chronic active hepatitis.

6. A kit for treating an inflammatory disease or disorder in a subject comprising:
a) an inhibitor of PKC; and
b) instructions for administering the PKC inhibitor to the subject, preferably wherein the inhibitor is a polypeptide, preferably
wherein the polypeptide comprises an N-terminal modification, C-terminal modification, or combination thereof, or
wherein the polypeptide is N-acylated, or
wherein the polypeptide is N-myristoylated or N-palmitoylated.

7. The kit of claim 6, wherein the instructions specify the polypeptide is administered parentally, subcutaneously, intravenously, topically, orally, mucosally, rectally, pulmonarily, nasally, or otically.

8. The kit of claim 6, wherein the instructions specify that the polypeptide is administered at a dose of about 0.1 to about 10000 micrograms per kilogram.

9. The kit of claim 6, wherein the instructions specify that the polypeptide is administered daily, weekly, biweekly or monthly.


SEQUENCE LISTING

<110>  Healor Ltd.

<120>  METHOD FOR TREATMENT OF INFLAMMATION

<130>  HO20164PCTEPD1

<150>  61/405509
<151>  2010-10-21

<150>  61/293794
<151>  2010-01-11

<160>  39

<170>  PatentIn version 3.5

<210>  1
<211>  6
<212>  PRT
<213>  Artificial Sequence

<220>
<223>  Synthetic peptide

<400>  1

Phe Ala Arg Lys Gly Ala
1               5

<210>  2
<211>  9
<212>  PRT
<213>  Artificial Sequence

<220>
<223>  Synthetic peptide

<400>  2

Phe Ala Arg Lys Gly Ala Leu Arg Gln
1               5

<210>  3
<211>  7
<212>  PRT
<213>  Artificial Sequence

<220>
<223>  Synthetic peptide

<400>  3

Phe Ala Arg Lys Gly Ala Leu
1               5


<210>  4
<211>  8
<212>  PRT
<213>  Artificial Sequence

<220>
<223>  Synthetic peptide

<400>  4

Phe Ala Arg Lys Gly Ala Arg Gln
1               5


<210>  5
<211>  8
<212>  PRT
<213>  Artificial Sequence

<220>
<223>  Synthetic peptide

<400>  5

Thr Leu Asn Pro Gln Trp Glu Ser
1               5


<210>  6
<211>  9
<212>  PRT
<213>  Artificial Sequence

<220>
<223>  Synthetic peptide


<220>
<221>  MISC_FEATURE
<222>  (1)..(1)

<223>  Modified with myristoyl group

<220>
<221>  MISC_FEATURE
<222>  (9)..(9)
<223>  C-terminal alcohol group

<400>  6

Phe Ala Arg Lys Gly Ala Leu Arg Gln
1               5


<210>  7
<211>  9
<212>  PRT
<213>  Artificial Sequence

<220>
<223>  Synthetic peptide


<220>
<221>  MISC_FEATURE
<222>  (9)..(9)
<223>  C-terminal alcohol group

<400>  7

Phe Ala Arg Lys Gly Ala Leu Arg Gln
1               5


<210>  8
<211>  7
<212>  PRT
<213>  Artificial Sequence

<220>
<223>  Synthetic peptide


<220>
<221>  MISC_FEATURE
<222>  (1)..(1)
<223>  Modified with myristoylgroup

<220>
<221>  MISC_FEATURE
<222>  (7)..(7)

<223>  C-terminal alcohol group

<400>  8

Phe Ala Arg Lys Gly Ala Leu
1               5

<210>  9
<211>  8
<212>  PRT
<213>  Artificial Sequence

<220>
<223>  Synthetic peptide

<220>
<221>  MISC_FEATURE
<222>  (8)..(8)
<223>  C-terminal alcohol group

<400>  9

Thr Leu Asn Pro Gln Trp Glu Ser
1               5

<210>  10
<211>  9
<212>  PRT
<213>  Artificial Sequence

<220>
<223>  Synthetic peptide

<220>
<221>  MISC_FEATURE
<222>  (1)..(1)
<223>  Modified with palmitoyl group

<220>
<221>  MISC_FEATURE
<222>  (9)..(9)
<223>  C-terminal alcohol

<400>  10

Phe Ala Arg Lys Gly Ala Leu Arg Gln

1               5


<210>  11
<211>  8
<212>  PRT
<213>  Artificial Sequence

<220>
<223>  Synthetic peptide



<220>
<221>  MISC_FEATURE
<222>  (1)..(1)
<223>  Modified with palmitoyl group

<220>
<221>  MISC_FEATURE
<222>  (8)..(8)
<223>  C-terminal alcohol group

<400>  11

Phe Ala Arg Lys Gly Ala Arg Gln
1               5


<210>  12
<211>  7
<212>  PRT
<213>  Artificial Sequence

<220>
<223>  Synthetic peptide



<220>
<221>  MISC_FEATURE
<222>  (1)..(1)
<223>  Modified with myristoyl group

<220>
<221>  MISC_FEATURE
<222>  (7)..(7)
<223>  C-terminal alcohol group

<400>  12

Phe Ala Arg Lys Gly Ala Leu

1               5


<210>  13
<211>  9
<212>  PRT
<213>  Artificial Sequence

<220>
<223>  Synthetic peptide


<220>
<221>  MISC_FEATURE
<222>  (9)..(9)
<223>  C-terminal alcohol group

<400>  13

Phe Ala Arg Lys Gly Ala Leu Arg Gln
1               5


<210>  14
<211>  8
<212>  PRT
<213>  Artificial Sequence

<220>
<223>  Synthetic peptide

<400>  14

Glu Ala Val Ser Leu Lys Pro Thr
1               5


<210>  15
<211>  8
<212>  PRT
<213>  Artificial Sequence

<220>
<223>  Synthetic peptide

<400>  15

Pro Tyr Ile Ala Leu Asn Val Asp
1               5

<210>  16
<211>  5
<212>  PRT
<213>  Artificial Sequence

<220>
<223>  Synthetic peptide

<400>  16

Pro Ala Trp His Asp
1               5


<210>  17
<211>  12
<212>  PRT
<213>  Artificial Sequence

<220>
<223>  Synthetic peptide

<400>  17

Leu Glu Pro Glu Ala Ala Ala Ala Ala Ala Gly Lys
1               5                   10


<210>  18
<211>  7
<212>  PRT
<213>  Artificial Sequence

<220>
<223>  Synthetic peptide

<400>  18

His Phe Glu Asp Trp Ile Asp
1               5


<210>  19
<211>  7
<212>  PRT
<213>  Artificial Sequence

<220>
<223>  Synthetic peptide

<400>  19

Val Tyr Val Ile Ile Asp Leu
1               5

<210>  20
<211>  8
<212>  PRT
<213>  Artificial Sequence

<220>
<223>  Synthetic peptide

<220>
<221>  MISC_FEATURE
<222>  (8)..(8)
<223>  C-terminal alcohol group

<400>  20

Glu Ala Val Ser Leu Lys Pro Thr
1               5

<210>  21
<211>  8
<212>  PRT
<213>  Artificial Sequence

<220>
<223>  Synthetic peptide

<220>
<221>  MISC_FEATURE
<222>  (8)..(8)
<223>  C-terminal alcohol group

<400>  21

Pro Tyr Ile Ala Leu Asn Val Asp
1               5

<210>  22
<211>  5
<212>  PRT

<213>  Artificial Sequence

<220>
<223>  Synthetic peptide


<220>
<221>  MISC_FEATURE
<222>  (5)..(5)
<223>  C-terminal alcohol group

<400>  22

Pro Ala Trp His Asp
1               5


<210>  23
<211>  12
<212>  PRT
<213>  Artificial Sequence

<220>
<223>  Synthetic peptide


<220>
<221>  MISC_FEATURE
<222>  (12)..(12)
<223>  C-terminal alcohol group

<400>  23

Leu Glu Pro Glu Ala Ala Ala Ala Ala Ala Gly Lys
1               5                   10


<210>  24
<211>  7
<212>  PRT
<213>  Artificial Sequence

<220>
<223>  Synthetic peptide


<220>
<221>  MISC_FEATURE
<222>  (7)..(7)
<223>  C-terminal alcohol group

<400>  24

His Phe Glu Asp Trp Ile Asp
1               5

<210>  25
<211>  7
<212>  PRT
<213>  Artificial Sequence

<220>
<223>  Peptide

<220>
<221>  MISC_FEATURE
<222>  (7)..(7)
<223>  C-terminal alcohol group

<400>  25

Val Tyr Val Ile Ile Asp Leu
1               5

<210>  26
<211>  17
<212>  PRT
<213>  Artificial Sequence

<220>
<223>  Peptide

<400>  26

Thr Arg Lys Arg Gln Arg Ala Met Arg Arg Arg Val His Gln Ile Asn
1               5                   10                  15

Gly

<210>  27
<211>  5
<212>  PRT
<213>  Artificial Sequence

<220>
<223>  Synthetic peptide

<400>  27

Lys Arg Thr Leu Arg
1               5


<210>  28
<211>  17
<212>  PRT
<213>  Artificial Sequence

<220>
<223>  Synthetic peptide


<220>
<221>  MISC_FEATURE
<222>  (1)..(1)
<223>  Modified with myristoyl group

<220>
<221>  MISC_FEATURE
<222>  (17)..(17)
<223>  C-terminal alcohol group

<400>  28

Thr Arg Lys Arg Gln Arg Ala Met Arg Arg Arg Val His Gln Ile Asn
1               5                   10                  15


Gly


<210>  29
<211>  5
<212>  PRT
<213>  Artificial Sequence

<220>
<223>  Synthetic peptide


<220>
<221>  MISC_FEATURE
<222>  (1)..(1)

<223>  Modified with myristoyl group

<220>
<221>  MISC_FEATURE
<222>  (5)..(5)
<223>  C-terminal alcohol group

<400>  29

Lys Arg Thr Leu Arg
1               5



<210>  30
<211>  7
<212>  PRT
<213>  Artificial Sequence

<220>
<223>  Synthetic peptide

<400>  30

His Phe Glu Asp Trp Ile Asp
1               5



<210>  31
<211>  14
<212>  PRT
<213>  Artificial Sequence

<220>
<223>  Synthetic peptide

<400>  31

His Phe Glu Asp Trp Ile Asp His Phe Glu Asp Trp Ile Asp
1               5                   10



<210>  32
<211>  12
<212>  PRT
<213>  Artificial Sequence

<220>
<223>  Synthetic peptide

<400>  32

Met Arg Ala Ala Glu Ala Ala Ala Ala Glu Pro Met
1               5                   10

<210>  33
<211>  14
<212>  PRT
<213>  Artificial Sequence

<220>
<223>  Synthetic peptide

<400>  33

Val Tyr Val Ile Ile Asp Leu His Phe Glu Asp Trp Ile Asp
1               5                   10

<210>  34
<211>  14
<212>  PRT
<213>  Artificial Sequence

<220>
<223>  Synthetic peptide

<220>
<221>  MISC_FEATURE
<222>  (14)..(14)
<223>  C-terminal alcohol group

<400>  34

His Phe Glu Asp Trp Ile Asp His Phe Glu Asp Trp Ile Asp
1               5                   10

<210>  35
<211>  12
<212>  PRT
<213>  Artificial Sequence

<220>
<223>  Synthetic peptide

<220>
<221>  MISC_FEATURE

<222>  (12)..(12)
<223>  C-terminal alcohol group

<400>  35

Met Arg Ala Ala Glu Ala Ala Ala Ala Glu Pro Met
1               5                   10


<210>  36
<211>  7
<212>  PRT
<213>  Artificial Sequence

<220>
<223>  Synthetic peptide


<220>
<221>  MISC_FEATURE
<222>  (7)..(7)
<223>  C-terminal alcohol group

<400>  36

His Phe Glu Asp Trp Ile Asp
1               5


<210>  37
<211>  14
<212>  PRT
<213>  Artificial Sequence

<220>
<223>  Synthetic peptide


<220>
<221>  MISC_FEATURE
<222>  (14)..(14)
<223>  C-terminal alcohol group

<400>  37

Val Tyr Val Ile Ile Asp Leu His Phe Glu Asp Trp Ile Asp
1               5                   10


<210>  38

<211>  13
<212>  PRT
<213>  Artificial Sequence

<220>
<223>  Synthetic peptide

<400>  38

Ser Ile Tyr Arg Arg Gly Ala Arg Arg Trp Arg Lys Leu
1               5                   10


<210>  39
<211>  13
<212>  PRT
<213>  Artificial Sequence

<220>
<223>  Synthetic peptide


<220>
<221>  MISC_FEATURE
<222>  (1)..(1)
<223>  Modified with myristoyl group

<220>
<221>  MISC_FEATURE
<222>  (13)..(13)
<223>  C-terminal alcohol group

<400>  39

Ser Ile Tyr Arg Arg Gly Ala Arg Arg Trp Arg Lys Leu
1               5                   10